(19) Europäisches Patentamt / European Patent Office / Office européen des brevets

(11) **EP 3 971 827 B1**

(12) **EUROPÄISCHE PATENTSCHRIFT**

(45) Veröffentlichungstag und Bekanntmachung des Hinweises auf die Patenterteilung:
**27.12.2023 Patentblatt 2023/52**

(21) Anmeldenummer: **20196746.0**

(22) Anmeldetag: **17.09.2020**

(51) Internationale Patentklassifikation (IPC):
**G16H 30/40** (2018.01)    **G06N 3/045** (2023.01)
**G16H 50/20** (2018.01)    **G06N 3/08** (2023.01)
**G06V 20/69** (2022.01)

(52) Gemeinsame Patentklassifikation (CPC):
**G16H 30/40; G06N 3/045; G06N 3/08; G06V 20/69; G16H 50/20**

(54) **VERFAHREN ZUM DETEKTIEREN EINER BINDUNG VON ANTIKÖRPERN EINER PATIENTENPROBE AN DOPPELSTRÄNGIGE DNS UNTER VERWENDUNG VON CRITHIDIA LUCILIAE ZELLEN UND FLUORESZENZMIKROSKOPIE**

METHOD FOR DETECTING A BINDING OF ANTIBODIES OF A PATIENT SAMPLE TO DOUBLE-STRANDED DNA USING CRITHIDIA LUCILIAE CELLS AND FLUORESCENCE MICROSCOPY

PROCÉDÉ DE DÉTECTION D'UNE LIAISON D'ANTICORPS D'UN ÉCHANTILLON DE PATIENT À L'ADN DOUBLE BRIN À L'AIDE DES CELLULES DE CRITHIDIA LUCILIAE ET MICROSCOPIE PAR FLUORESCENCE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(43) Veröffentlichungstag der Anmeldung:
**23.03.2022 Patentblatt 2022/12**

(73) Patentinhaber: **Euroimmun Medizinische Labordiagnostika AG**
**23560 Lübeck (DE)**

(72) Erfinder:
 • GERLACH, Stefan
   23627 Groß Grönau (DE)
 • KRAUTH, Jens
   23552 Lübeck (DE)
 • MARZAHL, Christian
   91052 Erlangen (DE)
 • KRAUSE, Christoper
   21514 Büchen (DE)
 • DANCKWARDT, Maick
   23919 Rondeshagen (DE)
 • HAHN, Melanie
   23617 Stockelsdorf (DE)
 • VOIGT, Jörn
   23558 Lübeck (DE)

(56) Entgegenhaltungen:
WO-A1-2018/200715    US-A1- 2019 030 371
US-A1- 2019 371 425

 • STEFAN GERLACH ET AL: "Automated Evaluation of Crithidia luciliae Based Indirect Immunofluorescence Tests: A Novel Application of the EUROPattern-Suite Technology", JOURNAL OF IMMUNOLOGY RESEARCH, Bd. 2015, 1. Januar 2015 (2015-01-01), Seiten 1-8, XP055588770, US ISSN: 2314-8861, DOI: 10.1155/2015/742402

**Beschreibung**

Die Erfindung betrifft ein Verfahren und eine Vorrichtung zum Detektieren einer Bindung von Autoantikörpern einer Patientenprobe an doppelsträngige Desoxyribonukleinsäure (DNS) unter Verwendung von Crithidia luciliae Zellen mittels Fluoreszenzmikroskopie und mittels digitaler Bildverarbeitung.

Der Nachweis von Autoantikörpern gegen Desoxyribonucleinsäuren (DNS) ist beispielsweise für die Diagnose des SLE (Synonym: Lupus erythematodes disseminatus) von entscheidender Bedeutung. Hierbei müssen grundsätzlich zwei Typen unterschieden werden: Antikörper gegen dsDNS und Antikörper gegen einzelsträngige, denaturierte DNS (Einzelstrang-DNS, ssDNS). Antikörper gegen dsDNS reagieren mit Epitopen, die im Desoxyribosephosphat-Gerüst der DNS liegen. Dagegen binden sich Antikörper gegen ssDNS vorwiegend an Epitope aus dem Bereich der Purin bzw. Pyrimidinbasen. Sie können aber auch Epitope des Desoxyribosephosphat-Gerüsts erkennen. Anti-dsDNS-Antikörper findet man fast ausschließlich beim SLE. Ihre Prävalenz beträgt, je nach Nachweismethode und Krankheitsaktivität, 20 % bis 90 %. Anti-dsDNS-Antikörper werden mitunter auch bei Patienten mit anderen Autoimmunerkrankungen und Infektionen sowie in seltenen Fällen bei klinisch gesunden Personen nachgewiesen. Letztere entwickeln in 85 % der Fälle einen SLE innerhalb von 5 Jahren nach dem Anti-dsDNS-Erstnachweis. Allerdings lässt sich ein SLE nicht ganz ausschließen, wenn keine Antikörper gegen dsDNS vorliegen. Der SLE ist eine systemische Autoimmunerkrankung aus der Gruppe der Kollagenosen. Die Diagnose richtet sich nach den 1997 modifizierten 11 Kriterien des American College of Rheumatology (ACR). Bei Vorhandensein von 4 der 11 Kriterien kann mit 80- bis 90 %-iger Sicherheit die Diagnose eines SLE gestellt werden.

Eine indirekte Immunfluoreszenz ist ein in-vitro-Test für die Bestimmung humaner Antikörper gegen dsDNS. Es können beispielsweise sogenannte BIOCHIPs als Substrate dienen, die mit Crithidia luciliae Ausstrichen beschichtet sind. Diese werden beispielsweise mit verdünnten Patientenproben inkubiert. Bei positiven Reaktionen binden sich spezifische Antikörper an die Antigene. Gebundene Antikörper (IgG) werden in einem weiteren Inkubationsschritt beispielsweise mit Fluorescein-markierten Anti-Human-Antikörpern angefärbt und im Fluoreszenzmikroskop sichtbar gemacht.

Aus dem Stand der Technik ist es daher bekannt, dass ein Substrat bereitgestellt wird, bei welchem mehrere Crithidia luciliae Zellen auf dem Substrat fixiert sind. Eine solche Fixierung kann beispielsweise mittels Ethanol vorgenommen sein.

Das Substrat wird dann mit einer Patientenprobe, vorzugsweise verdünntem Blutserum, inkubiert, wobei die Patientenprobe potentiell die zu detektierenden Autoantikörper aufweist. Nach dem Stand der Technik können dann die Zellen bzw. das Substrat mit einem sogenannten Konjugat inkubiert werden, welches sekundäre Antikörper aufweist, welche mit einem beispielsweise grünen Fluoreszenzfarbstoff markiert sind.

Es kann dann nach Bestrahlen des inkubierten Substrates mit Anregungslicht eine durch den grünen Fluoreszenzfarbstoff emittierte Fluoreszenzstrahlung als ein Mikroskopbild einer Fluoreszenzmikroskopie erfasst werden.

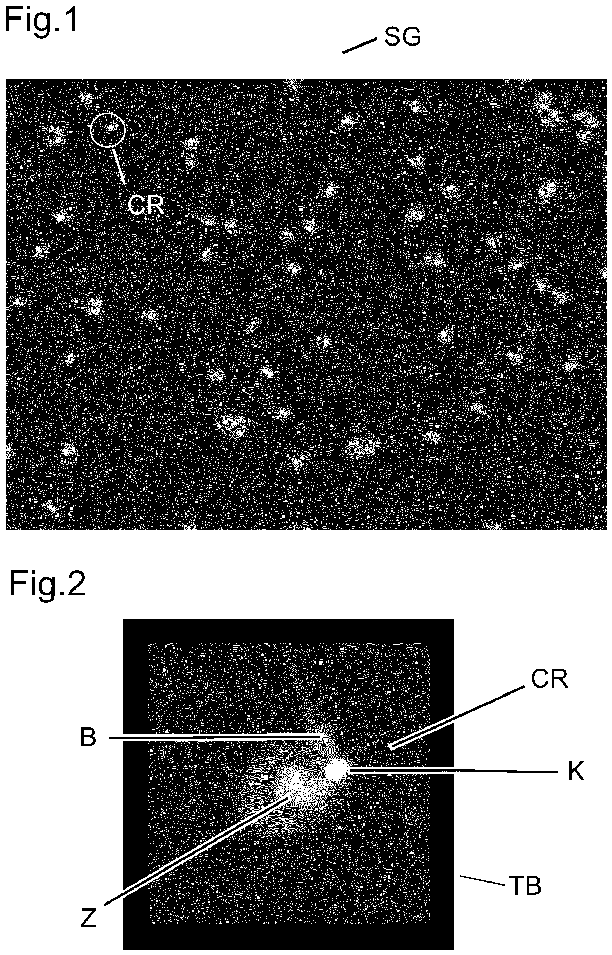

Ein solches Mikroskopbild ist beispielhaft in der Figur 1 dargestellt als das Bild SG.

Eine einzelne Crithidien Zelle CR aus der Figur 1 ist noch einmal in der Figur 2 detaillierter dargestellt. Ein solches Teilbild TB einer Crithidia luciliae Zelle CR zeigt deutlich eine Färbung am Kinetoplasten K, welcher auch als Mitochondrium bezeichnet wird. Weitere Färbungen sind am Zellkern Z als auch am Basalkörper B gegeben.

Für ein zuverlässiges Detektieren einer Bindung von Autoantikörpern aus der Patientenprobe an doppelsträngige DNS ist es entscheidend, eine Färbung von mehreren Kinetoplasten in dem Fluoreszenzbild SB des Substrates zu detektieren. Wie die Figur 2 zeigt, kann eine Bindung bzw. eine Färbung auch für einen Zellkern Z als auch für ein Basalkörper B gegeben sein, so dass mittels Bildverarbeitung der Kinetoplast K zuverlässig hinsichtlich seiner Lage im Bild bestimmt werden muss.

Durch eine Auswertung bezüglich einer jeweiligen Färbung jeweiliger Kinetoplasten jeweiliger Crithidia luciliae Zellen in dem Fluoreszenzbild SB kann dann insgesamt eine im Mittel gesehene Gesamtbindung von Autoantikörpern der Patientenprobe an doppelsträngige DNS ermittelt werden. STEFAN GERLACH ET AL: "Automated Evaluation of Crithidia luciliae Based Indirect Immunofluorescence Tests: A Novel Application of the EUROPattern-Suite Technology", JOURNAL OF IMMUNOLOGY RESEARCH, Bd. 2015, 1. Januar 2015 (2015-01-01), Seiten 1-8, offenbart eine Methode zur Bildverarbeitung basierend auf der Messung eines grünen und roten Fluoreszenzfarbstoffes. Basierend auf den daraus erhaltenen Bildern wird eine Segmentierung der Bilder vorgenommen. Die Teilbilder repräsentieren jeweils eine Crithidia luciliae Zelle. Zellen, an die keine Autoantikörper in der Region der Kinetoplasten binden, werden identifiziert. Anschließend werden Zellen, an die besagte Antikörper binden, unter Verwendung von Trainingsdaten klassifiziert. Daraus kann ein Antikörper Titer berechnet werden.

Aufgabe ist es also, ein Verfahren unter Verwendung digitaler Bildverarbeitung bereitzustellen, um eine Bindung von Autoantikörpern aus einer Patientenprobe an doppelsträngige DNS zu bestimmen, wobei eine Färbung unterschiedlicher Kinetoplastbereiche unterschiedlicher Crithidia luciliae Zellen innerhalb eines Fluoreszenzbildes sicher bestimmt werden kann. Die Erfindung wird durch die Ansprüche definiert.

Vorgeschlagen wird daher ein Verfahren zum Detektieren einer Bindung von Autoantikörpern einer Patientenprobe an doppelsträngige Desoxyribonukleinsäure unter Verwendung von Crithidia luciliae Zellen mittels Fluoreszenzmikroskopie und mittels digitaler Bildverarbeitung. Das Verfahren weist unterschiedliche Schritte auf. Es erfolgt zunächst ein Bereitstellen eines Substrates, welches mehrere Crithidia luciliae Zellen aufweist. Es erfolgt dann ein Inkubieren des Substrates mit der Patientenprobe, welche potentiell die Autoantikörper aufweist. Ferner erfolgt ein Inkubieren des Substrates mit sekundären Antikörpern, welche jeweils mit einem Fluoreszenzfarbstoff markiert sind, welcher vorzugsweise ein grüner Fluoreszenzfarbstoff ist. Es erfolgt ferner ein Erfassen eines Fluoreszenzbildes des Substrates in einem Farbkanal, welcher zu dem Fluoreszenzfarbstoff korrespondiert, wobei der Farbkanal vorzugsweise ein Grünkanal ist. Es erfolgt ferner ein Identifizieren jeweiliger Teilbilder in dem einen Fluoreszenzbild, welches erfasst wurde. Die Teilbilder repräsentieren jeweils eine Crithidia luciliae Zelle. Dieses Identifizieren erfolgt mittels eines ersten, vortrainierten Convolutional Neural Network. Es erfolgt ferner ein jeweiliges Prozessieren wenigstens einer Teilmenge der jeweiligen Teilbilder mittels eines zweiten, vortrainierten Convolutional Neural Networks zur Bestimmung jeweiliger Bindungsmaße, welche einen jeweiligen Grad einer Bindung von Autoantikörpern in einem jeweiligen Kinetoplastbereich einer jeweiligen Crithidia luciliae Zelle eines jeweiligen Teilbildes indizieren. Ferner erfolgt ein Bestimmen eines Gesamtbindungsmaßes bezogen auf eine Bindung von Autoantikörpern der Patientenprobe an doppelsträngige Desoxyribonukleinsäure auf Basis der jeweiligen Bindungsmaße.

Die Patientenprobe ist eine flüssige Patientenprobe, vorzugsweise flüssiges Blut oder flüssige Blutbestandteile. Insbesondere ist die flüssige Patientenprobe flüssiges Blutserum. Vorzugsweise ist die Patientenprobe verdünnt mit sogenanntem Waschpuffer, vorzugsweise sogenanntem PBS Tween.

Das Konjugat weist sekundäre Antikörper auf, welche mit dem Fluoreszenzfarbstoff markiert sind.

Das vorgeschlagene Verfahren ist insbesondere ein Verfahren zur in vitro Bestimmung eines Vorhandenseins von primären Antikörpern in einer flüssigen Patientenprobe.

Das Substrat ist insbesondere eine biologischen Probe, welche tierpathogene Hemoflagellaten der Art Crithidia luciliae aufweist. Diese Einzeller besitzen ein Doppelstrang-DNS haltiges Riesenmitochondrium (Kinetoplast), welches im Wesentlichen keines der übrigen in dem Zellkern enthaltende Antigene aufweist. Primäre Autoantikörper aus der Patientenprobe, die mit dem Kinetoplasten reagieren, sind gegen dsDNS gerichtet.

Die Fluoreszenzmikroskopie ist insbesondere eine sogenannte indirekte Immunfluoreszenzmikroskopie (IIFT Mikroskopie).

Das Substrat wird vorzugsweise mit Anregungsstrahlung beleuchtet, um Fluoreszenzstrahlung des ersten und des zweiten Fluoreszenzfarbstoffes anzuregen. Die Anregungsstrahlung ist vorzugsweise ein blaues Licht und weist somit Wellenlängen in einem Blauspektrum auf. Der Fluoreszenzfarbstoff ist vorzugsweise ein grüner Fluoreszenzfarbstoff, insbesondere des Typs Fluorescein isothiocyanate (FITC).

Zur Erläuterung eines oder mehrerer möglicherweise zu erreichender Vorteile des erfindungsgemäßen Verfahrens werden im Folgenden genauere Ausführung gemacht.

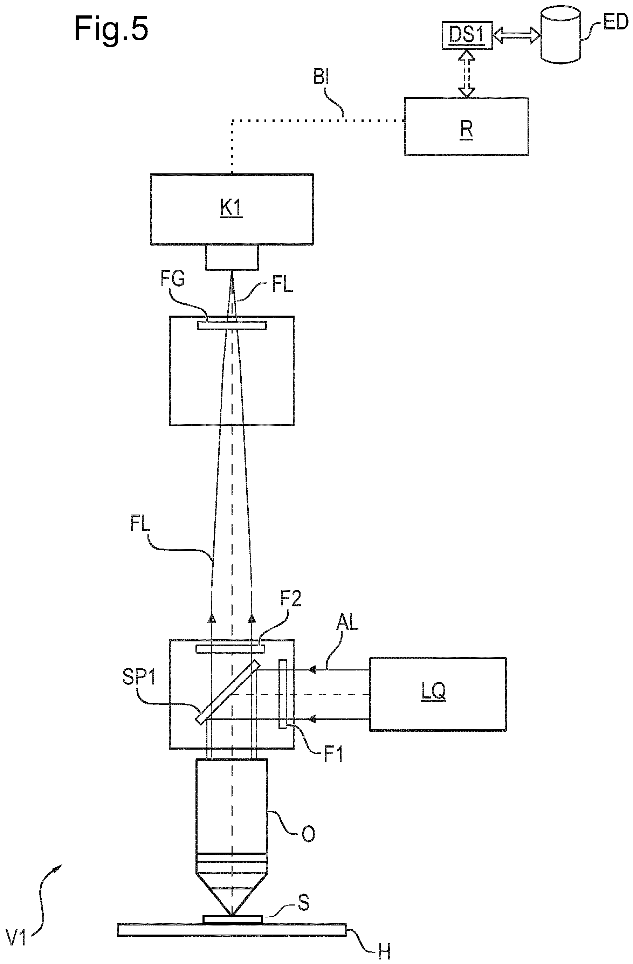

Die Figur 5 zeigt eine Vorrichtung V1, mittels welcher das erfindungsgemäße Verfahren durchgeführt werden kann. Die Vorrichtung V1 kann als ein Fluoreszenzmikroskop bezeichnet werden. Die Vorrichtung V1 weist eine Halterung H für ein Substrat S auf, welches in der zuvor beschriebenen Weise inkubiert wurde. Über eine Optik O wird Anregungslicht AL einer Anregungslichtquelle LQ hin zu einem Substrat S geleitet. Sich ergebende Fluoreszenzstrahlung FL wird dann durch die Optik O zurücktransmittiert und passiert den dichroitischen Spiegel SP1 und einen optionalen optischen Filter F2. Vorzugsweise passiert die Fluoreszenzstrahlung FL einen optischen Filter FG, welcher einen Grünkanal herausfiltert. Eine Kamera K1 ist vorzugsweise eine Monochromkamera, welche dann bei Vorhandensein eines optischen Filters FG die Fluoreszenzstrahlung FL in einem Grünkanal erfasst. Gemäß einer alternativen Ausführungsform ist die Kamera K1 eine Farbbildkamera, welche ohne Verwendung des optischen Filters FG auskommt und mittels einer Bayer-Matrix das Fluoreszenzbild in dem entsprechenden Farbkanal als einen Grünkanal erfasst. Die Kamera K1 stellt die Bildinformation BI bzw. das Fluoreszenzbild an eine Recheneinheit R bereit, welche diese Bildinformation BI verarbeitet.

Die Figur 1 zeigt ein beispielhaftes Fluoreszenzbild SG in einem Grünkanal.

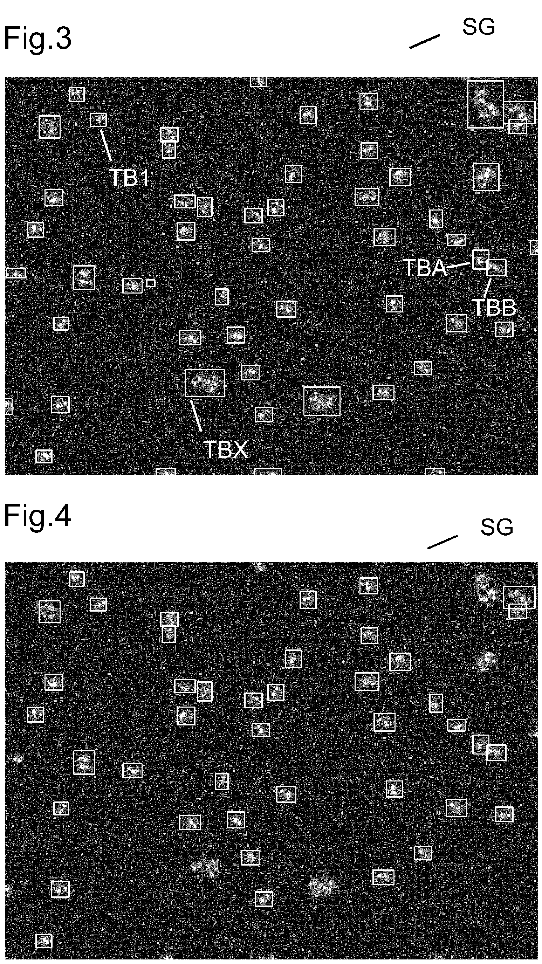

Erfindungsgemäß werden zunächst auf Basis des Fluoreszenzbildes SG solche Teilbilder identifiziert, welche insbesondere wenigstens eine bzw. mindestens eine Crithidia luciliae Zelle aufweisen. Die Figur 3 zeigt hierzu entsprechende durch Rechtecke markierte Teilbilder, insbesondere die Teilbilder TB1, TBA, TBB, TBX. Indiziert ist insbesondere ein bestimmter Teilbildbereich TB1.

Im Sinne dieser Anmeldung kann ein Teilbildbereich auch als ein Teilbild bezeichnet werden.

Ein solches beispielhaftes Teilbild TB, welches das Teilbild TB1 aus der Figur 1 ist, ist in der Figur 2 dargestellt. Wie es in Figur 2 zu erkennen ist, können in einer Crithidia luciliae Zelle bis zu drei signifikant gefärbte Bereiche aufgrund einer Bindung von Antikörpern gegeben sein, nämlich im Bereich des Kinetoplasten K, des Basalkörpers B als auch des Zellkerns Z. Daher muss sichergestellt werden, dass in dem Fluoreszenzbild SG nicht irgendwelche signifikante Färbungen als Bewertungskriterium verwendet werden, sondern nur Färbungen von Kinetoplastbereichen.

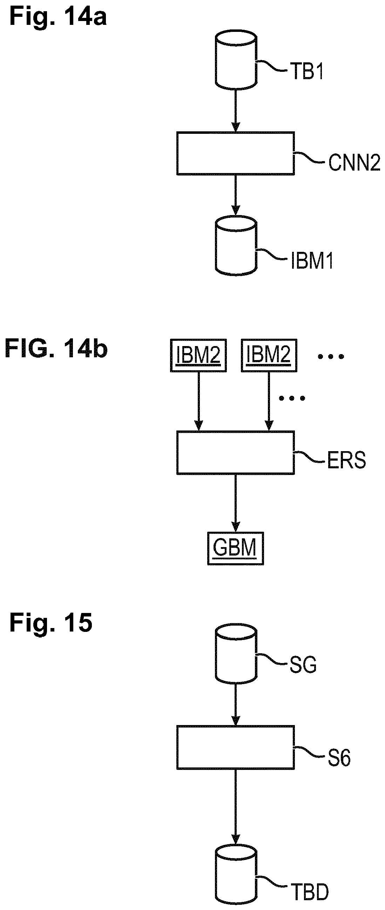

Die Figur 14a zeigt einen weiteren Schritt des erfindungsgemäßen Verfahrens, in welchem für ein bestimmtes Teilbild TB1 mittels eines zweiten Convolutional Neural Network CNN2 ein zugehöriges Bindungsmaß IBM1 bestimmt wird. Das Bindungsmaß IBM1 indiziert einen Grad einer Bindung von Autoantikörpern in einem Kinetoplastbereich der in dem Teilbild dargestellten Crithidia luciliae Zelle.

Die Figur 14b illustriert einen weiteren Schritt ERS des erfindungsgemäßen Verfahrens, in welchem auf Basis unterschiedlicher, jeweiliger Bindungsmaße IBM1, IBM2 von unterschiedlichen, jeweiligen Teilbildbereichen dann ein Gesamtbindungsmaß GBM ermittelt wird, welches bezogen ist auf die zu detektierende Bindung von Autoantikörpern der Patientenprobe an doppelsträngige DNS in dem Substrat.

Das Ziel des erfindungsgemäßen Verfahrens ist also ein Bestimmen der Bindung der primären Autoantikörper an die dsDNS in den jeweiligen Kinetoplastbereich der jeweiligen Crithidia luciliae Zellen mittels Bestimmung entsprechender Färbungen der entsprechenden Kinetoplastbereiche durch den Fluoreszenzfarbstoff in den entsprechenden Teilbildbereichen.

Es ist festzustellen, dass in dem erfindungsgemäßen Verfahren nicht einfach das gesamte Fluoreszenzbild SG aus der Figur 1 einem einzelnen Convolutional Nuronal Network für eine gesamte Erkennung mehrerer gefärbter Kinetoplastbereiche zugeführt wird, sondern dass die Erfindung explizit von einem solchen ganzheitlichen Klassifikationsansatz für das gesamte Fluoreszenzbild SG mittels eines einzigen Convolutional Neural Network abweicht. Erfindungsgemäß erfolgt nämlich zunächst eine Vorprozessierung in der Weise, dass mittels des ersten Convolutional Neural Network in dem Fluoreszenzbild die Teilbildbereiche bzw. Teilbilder identifiziert werden. Wie es in Figur 2 zu erkennen ist, können in einer Crithidia luciliae Zelle bis zu drei signifikant gefärbte Bereiche aufgrund einer Bindung von Antikörpern gegeben sein, nämlich im Bereich des Kinetoplasten K, des Basalkörpers B als auch des Zellkerns Z. Die durch das erste Convolutional Neural Network erkannten Teilbilder werden dann zunächst jeweils für sich separat durch das zweite Convolutional Neural Network CNN2 prozessiert, um für ein jeweiliges Teilbild ein jeweiliges Subbild zu bestimmen, das den Kinetoplastbereich repräsentiert, und um dann insbesondere mittels des Subbildes ein jeweiliges Bindungsmaß zu bestimmen, welches einen jeweiligen Grad einer Bindung von Autoantikörpern in einem jeweiligen Kinetoplastbereich einer jeweiligen Crithidia luciliae Zelle eines jeweiligen Teilbildes indiziert. Mit anderen Worten: Es werden insbesondere diese identifizierten Teilbildbereiche zunächst jeweils für sich separat durch das zweite Convolutional Neural Network CNN2 prozessiert, um für ein jeweiliges Teilbild ein jeweiliges Subbild zu identifizieren und um dann auf Basis des jeweiligen Subbildes ein jeweiliges Bindungsmaß für das jeweilige Teilbild zu bestimmen. Ein solches Bindungsmaß indiziert einen jeweiligen Grad einer Bindung von Autoantikörpern in einem jeweiligen Kinetoplastbereich einer jeweiligen Crithidia luciliae Zelle eines jeweiligen Teilbildes. Auf Basis dieser jeweiligen einzelnen Bindungsmaße der jeweiligen Teilbilder wird dann das Gesamtbindungsmaß ermittelt.

Erfindungsgemäß erfolgt das Identifizieren der Teilbilder in dem einen Fluoreszenzbild mittels des ersten, vortrainierten Convolutional Neural Networks. Dieses ist vorteilhaft, da dieses erste Convolutional Neural Network nicht für die Gesamtaufgabe des Bestimmens eines Gesamtbindungsmaßes bezogen auf die Bindung von Autoantikörpern der Patientenprobe an doppelsträngige Desoxyribonukleinsäure herangezogen wird, sondern da dieses erste Convolutional Neural Network lediglich die Teilaufgabe der Identifikation von Teilbildern, welche jeweils eine Crithidia luciliae Zelle repräsentieren, verwendet wird. Dieses erste Convolutional Neural Network muss also nur in einer solchen Weise vortrainiert sein, dass es entsprechende Teilbilder in dem Fluoreszenzbild identifiziert bzw. lokalisiert. Erfindungsgemäß werden dann die identifizierten Teilbilder jeweils separat durch das zweite Convolutional Neural Network prozessiert, um für jedes der Teilbilder ein individuelles Bindungsmaß von Autoantikörpern an die jeweiligen Kinetoplastbereiche der jeweiligen Crithidia luciliae Zelle aus dem Teilbild zu ermitteln. Somit kann eben das zweite Convolutional Neural Network in der Weise vortrainiert werden, dass es auf die Aufgabe fokussiert bzw. ausgelegt wird, in einem Teilbild mit einer insbesondere einzelnen Crithidia luciliae Zelle dann den Kinetoplastbereich zu identifizieren. Es werden eben nicht die gesamten Bildinformationen des Fluoreszenzbildes in ein einzelnes Convolutional Neural Network eingebracht, sondern es werden separate Convolutional Neural Networks für die genannten separaten Teilaufgaben des Identifizierens der Teilbilder und des Bestimmens der jeweiligen individuellen Bindungsmaße für die jeweiligen Teilbilder verwendet. Hierdurch können diese separaten Convolutional Neural Networks so ausgestaltet und vortrainiert werden, dass sie ihre jeweiligen Aufgaben zuverlässig ausführen, wohingegen eine Auslegung bzw. ein Trainieren eines einzelnen Convolutional Neural Networks zur Prozessierung eines gesamten Fluoreszenzbildes für den Zweck der Bestimmung des Gesamtbindungsmaßes eine extrem hohe Menge an Bildinformationen verarbeiten müsste, um den Zweck der Bestimmung des Gesamtbindungsmaßes zu erfüllen, weshalb dann ein solches einzelnes Convolutional Neural Network möglicherweise fehleranfällig werden würde.

Das vorgeschlagene Verfahren ist ferner vorteilhaft, da in dem einen Fluoreszenzbild des einen Farbkanals, welcher vorzugsweise ein Grünkanal ist, das erste Convolutional Neural Network direkt in diesem einen Fluoreszenzbild die Teilbilder identifiziert bzw. lokalisiert. Es ist also somit erfindungsgemäß nicht notwendig, dass das Substrat mit einer sogenannten Gegenfärbung mittels eines anderen Fluoreszenzfarbstoffes wie beispielsweise Evans Blue zur Erzeugung einer Rotfärbung in einem Rotkanal präpariert sein müsste, sondern es kann eben erfindungsgemäß auf eine solche Gegenfärbung für einen Rotkanal komplett verzichtet werden. Dieses wird dadurch erreicht, dass zu dem Schritt bzw. für die Aufgabe des Identifizierens der Teilbilder, welche jeweils eine Crithidia luciliae Zelle repräsentieren, das erste Convolutional Neural Network ausgelegt bzw. vortrainiert wird.

Mit anderen Worten: Das erfindungsgemäße Verfahren erzielt durch den hier beschriebenen Ansatz eine hohe Genauigkeit bei der Lokalisation der Kinetoplastbereiche beziehungsweise bei einer Detektion einer Verfärbung der Kinetoplastbereiche. Würde das gesamte Fluoreszenzfarbbild des SG aus der Figur 1 einem einzigen Convolutional Neural Network zugeführt werden, um eine Verfärbung eines Kinetoplasten bzw. der verschiedenen Kinetoplastbereiche der verschiedenen Crithidia luciliae Zellen zu detektieren, so könnten hier andere Bereiche wie Basalkörperbereiche oder Zellkernbereiche fehlerhaft als jeweils ein Kinetoplastbereich identifiziert werden und eine Bestimmung einer Bindung von primären Autoantikörpern an dsDNS daher verfälscht werden. Ein gesamtes Fluoreszenzbild SG weist ferner zu einem sehr großen Anteil von ca. 90% lediglich Hintergrundbereiche auf, so dass bei einem Prozessieren eines gesamten Fluoreszenzbildes SG durch ein einzelnes CNN auch die zu dem Hintergrundbereich zugehörigen Bildpixel prozessiert werden müssten, welches das einzelne CNN sehr aufwändig machen würde und eine Prozessierung ineffizient. Ferner müsste das einzelne CNN aus dem gesamten Fluoreszenzbild SG auch noch solche Unterbereiche identifizieren, welche jeweils einen Kinetoplasten repräsentieren, welches aufgrund möglicher Färbungen von Zellkernbereichen oder Basalkörperbereichen aufwändig ist. Es muss nämlich im Falle einer Färbung eines Basalkörperbereiches und/oder eines Zellkernbereiches nicht unbedingt eine Färbung eines Kinetoplastbereiches vorliegen. Aufgrund unterschiedlicher Lagen der Crithidia luciliae Zellen innerhalb des Substrates bzw. des Fluoreszenzfarbbildes SG, wie in der Figur 1 darstellt, ergeben sich eben zu viele Freiheitsgrade für eine sichere Klassifikation einzelner Bildsegmente als Kinetoplast durch ein einzelnes Convolutional Neural Network.

Es erfolgt statt eines Prozessierens eines gesamten Fluoreszenzbildes durch ein einzelnes CNN erfindungsgemäß ein separates Prozessieren durch das zweite Convolutional Neural Network CNN2 für ein jeweiliges Teilbild für sich separat. Das Teilbild wird eben hinsichtlich seiner Lage bezogen auf das gesamte Fluoreszenzbild aufgrund einer Auswertung des Fluoreszenzbildes mittels des ersten Convolutional Neural Network CNN1 bestimmt. Hierdurch wird es ermöglicht, dass das zweite Convolutional Neural Network CNN2 explizit auf einzelne Teilbilder trainiert werden kann, also auf jeweilige Teilbilder mit einzelner Crithidia luciliae Zelle. Das zweite Convolutional Neural Network CNN2 muss dann nur noch in diesem Teilbild die Lage des Kinetoplasten detektieren. Diese Lage kann eben vorzugsweise als ein sogenanntes Subbild bestimmt werden. Es kann dann bezogen auf den einzelnen Kinetoplasten ein Bindungsmaß für diese Zelle bzw. diesen Kinetoplasten durch das zweite Convolutional Neural Network CNN2 bestimmt werden.

Mit abermals anderen Worten: Erfindungsgemäß wird durch Zuführen jeweils nur bestimmter einzelner Teilbildbereiche in das zweite Convolutional Neural Network CNN2 es ermöglicht, dass das zweite Convolutional Neural Network CNN2 lediglich auf eine Analyse eines solchen Teilbildes bzw. einer einzelnen Darstellung einer einzelnen Crithidia luciliae Zelle beschränkt wird, um den Kinetoplasten zu identifizieren und das Bindungsmaß bezogen auf die Autoantikörper zu bestimmen. Würde ein gesamtes Fluoreszenzfarbbild wie das Bild SG aus der Figur 1 einem einzelnen Convolutional Neural Network für eine Klassifikationsaufgabe zur Detektion von unterschiedlichen Kinetoplasten im Zuge einer Trainingsphase zugeführt werden, so wäre dieses einzelne Convolutional Neural Network sehr groß in seinem Freiheitsgrad auszugestalten und es wäre sehr aufwendig und ineffizient, ein solches einzelnes CNN zu trainieren. Dadurch, dass das erfindungsgemäße zweite Convolutional Neural Network CNN2 jeweils nur einen einzelnen Teilbildbereich für sich separat prozessieren muss um dann ein entsprechendes Bindungsmaß zu bestimmen, kann das zweite Convolutional Neural Network CNN2 auf eine Prozessierung eines eine Crtihidia luciliae Zelle repräsentierenden Bildes, wie in der Figur 2 als Bild TB dargestellt, beschränkt werden.

Vorteilhafte Ausführungsformen der Erfindung sind Gegenstand der abhängigen Ansprüche und werden in der folgenden Beschreibung unter Teilweise Bezugnahme auf die Figuren näher erläutert.

Vorzugsweise erfolgt das Identifizieren der jeweiligen Teilbilder in dem einen Fluoreszenzbild dadurch, dass mittels des ersten, vortrainierten Convolutional Neural Networks jeweilige Bildsegmente des Fluoreszenzbildes jeweiligen Bildsegmentklassen aus einer Gruppe von Bildsegmentklassen zugeordnet werden, wobei die Gruppe der Bildsegmentklassen wenigstens die folgenden Bildsegmentklassen umfasst: Zelle und Hintergrund.

Vorzugsweise umfasst die Gruppe der Bildsegmentklassen wenigstens die folgenden Bildsegmentklassen: Zelle, Zellrand und Hintergrund. Vorzugsweise kann die Klasse "Zelle" auch als Klasse "Zell-Leib" bezeichnet werden.

Vorzugsweise weist das Verfahren für ein jeweiliges Teilbild die Schritte auf: Selektieren eines jeweiligen Subbildes des jeweiligen Teilbildes, wobei das jeweilige Subbild einen jeweiligen Kinetoplastbereich einer jeweiligen Crithidia luciliae Zelle repräsentiert, ferner Bestimmen des jeweiligen Bindungsmaßes auf Basis des jeweiligen Subbildes. Das Verfahren weist vorzugsweise ferner auf: Bestimmen des Gesamtbindungsmaßes auf Basis der jeweiligen Bindungsmaße.

Vorzugsweise weist das Verfahren die Schritte auf: Bestimmen wenigstens einer jeweiligen finalen Feature Map für ein jeweiliges Teilbild mittels des zweiten Convolutional Neural Network, ferner Bestimmen eines jeweiligen Konfidenzmaßes bezogen auf ein Vorliegen einer Bindung von Autoantikörpern in einem jeweiligen Kinetoplastbereich für das jeweilige Teilbild bzw. für eine jeweilige finale Feature Map, insbesondere auf Basis der jeweiligen finalen Feature Map, ferner Auswählen einer Teilmenge der Teilbilder bzw. Auswählen einer Teilmenge der jeweiligen finalen Feature Maps auf Basis der bestimmten Konfidenzmaße, ferner jeweiliges Prozessieren der jeweiligen Feature Maps der jeweiligen ausgewählten Teilbilder zur Bestimmung der jeweiligen Bindungsmaße. Das Verfahren weist ferner vorzugsweise auf: Bestimmen des Gesamtbindungsmaßes auf Basis der jeweiligen Bindungsmaße.

Vorzugsweise weist das Verfahren für ein jeweiliges Teilbild aus der ausgewählten Teilmenge die Schritte auf: Selektieren eines jeweiligen Subbildes des jeweiligen Teilbildes auf Basis der zu dem jeweiligen Teilbild korrespondierenden jeweiligen finalen Feature Map, wobei das jeweilige Subbild einen jeweiligen Kinetoplastbereich einer jeweiligen Crithidia luciliae Zelle repräsentiert, sowie ferner Bestimmen des jeweiligen Bindungsmaßes auf Basis des jeweiligen Subbildes. Das Verfahren weist ferner vorzugsweise auf: Bestimmen des Gesamtbindungsmaßes auf Basis der jeweiligen Bindungsmaße.

Vorzugsweise weist das Verfahren für ein jeweiliges Teilbild aus der ausgewählten Teilmenge ferner die Schritte auf: Ermitteln eines jeweiligen Maskierungsoperators auf Basis der jeweiligen finalen Feature Map, ferner Selektieren des jeweiligen Subbildes des jeweiligen Teilbildes mittels Anwendung des jeweiligen Maskierungsoperators auf das jeweilige Teilbild, ferner Bestimmen des jeweiligen Bindungsmaßes auf Basis des jeweiligen Subbildes. Das Verfahren weist ferner vorzugsweise auf: Bestimmen des Gesamtbindungsmaßes auf Basis der jeweiligen Bindungsmaße.

Vorzugsweise ist das Verfahren so ausgestaltet, dass das zweite Convolutional Neural Network im Zuge einer Prozessierung eines Teilbildes in einer ersten Prozessierungsebene auf Basis des Teilbildes mittels eines ersten Convolutional Layer eine erste Menge von resultierenden Feature Maps generiert, ferner in einer zweiten Prozessierungsebene auf Basis der ersten Menge zweidimensionaler Feature Maps mittels eines zweiten Convolutional Layer eine zweite Menge von resultierenden Feature Maps generiert sowie ferner auf Basis der zweiten Menge zweidimensionaler Feature Maps mittels eines dritten Convolutional Layer eine dritte Menge von resultierenden Feature Maps generiert, wobei die zweite Menge eine geringere Anzahl resultierender Feature Maps aufweist als die erste Menge und wobei die dritte Menge eine größere Anzahl resultierender Feature Maps aufweist als die zweite Menge.

Vorzugsweise ist das Verfahren so ausgestaltet, dass das zweite Convolutional Layer und das dritte Convolutional Layer als Teilschritte eines sequentiellen Prozessierungspfades aufeinander abfolgen, wobei in der zweiten Prozessierungsebene ein weiterer Prozessierungspfad parallel zu dem sequentiellen Prozessierungspfad vorhanden ist, in welchem das zweite Convolutional Neural Network auf Basis der ersten Menge zweidimensionaler Feature Maps mittels wenigstens eines vierten Convolutional Layer eine vierte Menge von resultierenden Feature Maps generiert, wobei ferner das zweite Convolutional Neural Network die zu dem Teilbild korrespondierende finale Feature Map auf Basis der dritten und der vierten Menge von resultierenden Feature Maps generiert und wobei ferner die Anzahl aufeinanderfolgender Convolutional Layer in dem parallelen Prozessierungspfad geringer ist als die Anzahl aufeinanderfolgender Convolution Layer in dem sequentiellen Prozessierungspfad

Vorzugsweise weist das Verfahren die Schritte auf: Erfassen eines ersten vorläufigen Fluoreszenzbildes unter Verwendung eines fest vorgegebenen Erfassungsparameters, Feststellen, ob eine Helligkeit des ersten vorläufigen Fluoreszenzbildes eine maximale Helligkeit übersteigt, in dem Fall, dass das erste vorläufige Fluoreszenzbild die maximale Helligkeit nicht übersteigt, Verwenden des ersten vorläufigen Fluoreszenzbildes als das Fluoreszenzbild, in dem Fall, dass das das erste vorläufige Fluoreszenzbild die maximale Helligkeit übersteigt, Erfassen eines zweiten vorläufigen Fluoreszenzbildes und Verwenden des zweiten vorläufigen Fluoreszenzbildes als das Fluoreszenzbild.

Vorgeschlagen wird ferner eine erfindungsgemäße Vorrichtung zum Detektieren einer Bindung von Autoantikörpern einer Patientenprobe an doppelsträngige Desoxyribonukleinsäure unter Verwendung von Crithidia luciliae Zellen mittels Fluoreszenzmikroskopie und mittels digitaler Bildverarbeitung, aufweisend eine Haltevorrichtung für ein Substrat, welches mehrere Crithidia luciliae Zellen aufweist und welches mit einer Patientenprobe aufweisend die Autoantikörper, sowie ferner mit sekundären Antikörpern, welche jeweils mit einem Fluoreszenzfarbstoff markiert sind, inkubiert wurde. Die Vorrichtung weist ferner wenigstens eine Bilderfassungseinheit zum Erfassen eines Fluoreszenzbildes des Substrates auf. Die Vorrichtung weist ferner wenigstens eine Recheneinheit auf, welche ausgebildet ist mittels eines ersten, vortrainierten Convolutional Neural Network jeweilige Teilbilder in dem einen Fluoreszenzbild zu identifizieren, welche jeweils wenigstens eine Crithidia luciliae Zelle repräsentieren, ferner wenigstens eine Teilmenge der jeweiligen Teilbilder jeweils separat zu prozessieren mittels eines vortrainierten zweiten Convolutional Neural Network zur Bestimmung jeweiliger Bindungsmaße, welche einen jeweiligen Grad einer Bindung von Autoantikörpern in einem jeweiligen Kinetoplastbereich einer jeweiligen Crithidia luciliae Zelle eines jeweiligen Teilbildes indizieren, sowie ferner ein Gesamtbindungsmaß einer Bindung von Autoantikörpern der Patientenprobe an doppelsträngige Desoxyribonukleinsäure auf Basis der jeweiligen Bindungsmaße zu bestimmen.

Vorgeschlagen wird ferner eine Recheneinheit, welche ausgebildet ist, im Zuge einer digitalen Bildverarbeitung ein Fluoreszenzbild entgegenzunehmen, welches eine Färbung eines Substrates, welches wiederum mehrere Crithidia luciliae Zellen aufweist, durch einen Fluoreszenzfarbstoff repräsentiert, ferner mittels eines ersten Convolutional Neural Network jeweilige Teilbilder in dem Fluoreszenzbild zu identifizieren, welche jeweils wenigstens eine Crithidia luciliae Zelle repräsentieren, ferner wenigstens eine Teilmenge der jeweiligen Teilbilder jeweils separat zu prozessieren mittels eines vortrainierten zweiten Convolutional Neural Network zur Bestimmung jeweiliger Bindungsmaße, welche einen jeweiligen Grad einer Bindung von Autoantikörpern in einem jeweiligen Kinetoplastbereich einer jeweiligen Crithidia luciliae Zelle eines jeweiligen Teilbildes indizieren, sowie ferner ein Gesamtbindungsmaß einer Bindung von Autoantikörpern der Patientenprobe an doppelsträngige Desoxyribonukleinsäure auf Basis der jeweiligen Bindungsmaße zu bestimmen.

Vorgeschlagen wird ferner eine Datennetzwerkvorrichtung aufweisend wenigstens eine Datenschnittstelle zum Entgegennehmen eines Fluoreszenzbildes, welches eine Färbung eines Substrates, welches wiederum mehrere Crithidia luciliae Zellen aufweist, durch einen Fluoreszenzfarbstoff repräsentiert. Die Datennetzwerkvorrichtung weist ferner eine Recheneinheit auf, welche ausgebildet ist, im Zuge einer digitalen Bildverarbeitung jeweilige Teilbilder in dem einen Fluoreszenzbild mittels eines ersten Convolutional Neural Network zu identifizieren, welche jeweils wenigstens eine Crithidia luciliae Zelle repräsentieren, ferner wenigstens eine Teilmenge der jeweiligen Teilbilder jeweils separat zu prozessieren mittels eines vortrainierten zweiten Convolutional Neural Network zur Bestimmung jeweiliger Bindungsmaße, welche einen jeweiligen Grad einer Bindung von Autoantikörpern in einem jeweiligen Kinetoplastbereich einer jeweiligen Crithidia luciliae Zelle eines jeweiligen Teilbildes indizieren, sowie ferner ein Gesamtbindungsmaß einer Bindung von Autoantikörpern der Patientenprobe an doppelsträngige Desoxyribonukleinsäure auf Basis der jeweiligen Bindungsmaße zu bestimmen.

Vorgeschlagen wird ferner ein Verfahren zur digitalen Bildverarbeitung aufweisend die Schritte: Entgegennehmen eines Fluoreszenzbildes, welches eine Färbung eines Substrates, welches wiederum mehrere Crithidia luciliae Zellen aufweist, durch einen Fluoreszenzfarbstoff, ferner Identifizieren jeweiliger Teilbilder in dem Fluoreszenzbild, welche jeweils wenigstens eine Crithidia luciliae Zelle repräsentieren, mittels eines ersten Convolutional Neural Network, ferner jeweiliges separates Prozessieren wenigstens einer Teilmenge der jeweiligen Teilbilder mittels eines vortrainierten, zweiten Convolutional Neural Network zur Bestimmung jeweiliger Bindungsmaße, welche einen jeweiligen Grad einer Bindung von Autoantikörpern in einem jeweiligen Kinetoplastbereich einer jeweiligen Crithidia luciliae Zelle eines jeweiligen Teilbildes indizieren, sowie ferner Bestimmen eines Gesamtbindungsmaßes einer Bindung von Autoantikörpern der Patientenprobe an doppelsträngige Desoxyribonukleinsäure auf Basis der jeweiligen Bindungsmaße.

Vorgeschlagen wird ferner ein Computerprogrammprodukt, umfassend Befehle, die bei der Ausführung des Programms durch einen Computer diesen veranlassen, das erfindungsgemäße Verfahren zur digitalen Bildverarbeitung durchzuführen

Vorgeschlagen wird ferner ein Datenträgersignal, welches das vorgeschlagene Computerprogrammprodukt überträgt.

Im Folgenden wird die Erfindung anhand spezieller Ausführungsform ohne Beschränkung des allgemeinen Erfindungsgedankens anhand der Figuren näher erläutert. Dabei zeigen:

Figur 1 ein Fluoreszenzbild eines Substrates aufweisend mehrere Crithidia luciliae Zellen in dem Farbkanal

Figur 2 ein Teilbild des Fluoreszenzbildes aus der Figur 1 mit einer Crithidia luciliae Zelle,

Figur 3 das Fluoreszenzbild des ersten Farbkanals mit identifizierten jeweiligen ersten Teilbildbereichen,

Figur 4 das Fluoreszenzbild mit einer Indizierung ausgewählter Teilbilder aus der Gesamtmenge der Teilbilder aus der Figur 3,

Figur 5 ein Ausführungsbeispiel einer erfindungsgemäßen Vorrichtung,

Figur 6 ein zu dem Fluoreszenzbild ermitteltes Binärbild als eine Binärinformation bzw. Binärmaske,

Figur 7a ein Klassifikationsergebnis für einen bestimmten Bildausschnittaus der Figur 3,

Figur 7b ein finales Klassifikationsergebnis einer ersten Art,

Figur 7c ein finales Klassifikationsergebnis einer zweiten Art,

Figur 8a ein beispielhaftes Teilbild,

Figur 8b eine erste finale Feature Map,

Figur 8c eine vorzugsweise zu bestimmende zweite finale Feature Map,

Figur 9a eine interpolierte Version der ersten finalen Feature Map aus der Figur 8b,

Figur 9b einen binärwertigen Maskierungsoperator abgeleitet aus der interpolierten Feature Map aus der Figur 9a,

Figur 9c einen selektierten Subbildbereich eines Subbildes aus dem Teilbildbereich aus der Figur 8a,

Figur 10 Schritte zur Verarbeitung des Fluoreszenzbildes mittels des ersten Convolutional Neural Networks,

Figur 11 detaillierte Schritte aus der Verarbeitung durch das erste Convolutional Neural Network,

Figur 12a eine beispielhafte Darstellung des Fluoreszenzbildes,

Figur 12b eine beispielhafte Darstellung einer ersten Klassifikationsmatrix bzw. einer ersten Klassifikationsmap,

Figur 13 Schritte zur Durchführung des erfindungsgemäßen Verfahrens

Figur 14a ein Grundprinzip zur Ermittlung eines Bindungsmaßes einer Bindung primärer Autoantikörper der Patientenprobe an ein Kinetoplastbereich für einen Teilbildbereich,

Figur 14b eine Prozessierung mehrerer Bindungsmaße zur Bestimmung eines Gesamtbindungsmaßes,

Figur 15 Schritte zur Bestimmung erster Teilbildbereiche in dem ersten Fluoreszenzfarbbild des ersten Farbkanals,

Figur 16a eine beispielhafte Illustration einer bevorzugten Ausführungsform eines zweiten Convolutional Neural Network,

Figur 16b Teilschritte einer ersten Art eines Convolutional Layer des zweiten Convolutional Neural Network,

Figur 16c Teilschritte einer weiteren Art eines Convolutional Layer des zweiten Convolutional Neural Network,

Figur 17 Prozessierungsschritte zur Bestimmung eines Konfidenzmaßes bezogen auf ein Vorliegen einer Bindung von Autoantikörpern an einen Kinetoplastbereich für ein entsprechendes Teilbild auf Basis der zugehörigen Feature Maps,

Figur 18 Schritte zum Auswählen einer Teilmenge der identifizierten Teilbilder auf Basis der Konfidenzmaße der jeweiligen Teilbilder,

Figur 19 Schritte für ein Selektieren eines jeweiligen Subbildes eines jeweiligen Teilbildes sowie für ein Bestimmen eines jeweiligen Bindungsmaßes auf Basis des jeweiligen Subbildes mittels Anwenden eines binärwertigen Maskierungsoperators auf das jeweilige Teilbild,

Figur 20 eine beispielhafte Darstellung einer erfindungsgemäßen Recheneinheit,

Figur 21 eine beispielhafte Darstellung einer erfindungsgemäßen Datennetzwerkvorrichtung,

Figur 22 eine beispielhafte Darstellung eines erfindungsgemäßen Computerprogrammproduktes als auch eines erfindungsgemäßen Datenträgersignals,

Figur 23 ein Ausführungsbeispiel einer ersten Prozessierungsebene des CNN,

Figur 24 ein Ausführungsbeispiel eines ersten Teils einer zweiten Prozessierungsebene des CNN,

Figur 25 ein Ausführungsbeispiel eines zweiten Teils einer zweiten Prozessierungsebene des CNN,

Figur 26 ein Ausführungsbeispiel einer dritten Prozessierungsebene des CNN,

Figur 27 Pixelwerte eines Subbildes,

Figur 28 jeweilige Konfidenzmaßwerte für jeweilige Teilbilder eines Fluoreszenzbildes,

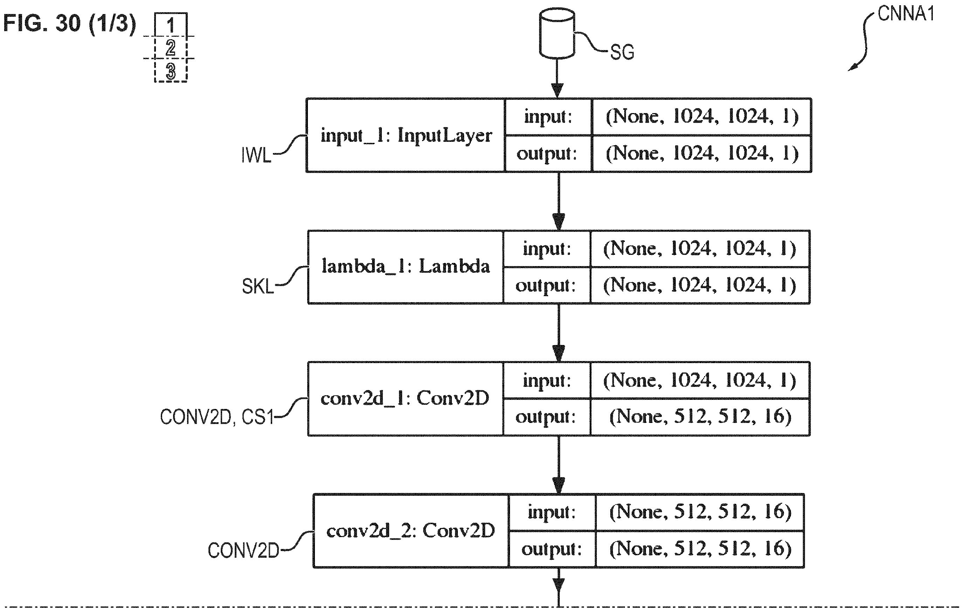

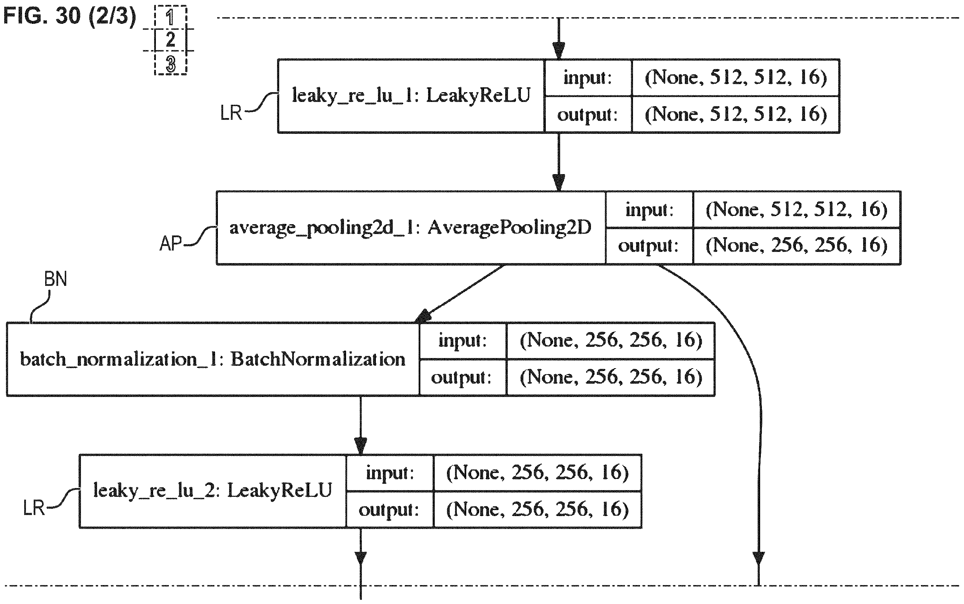

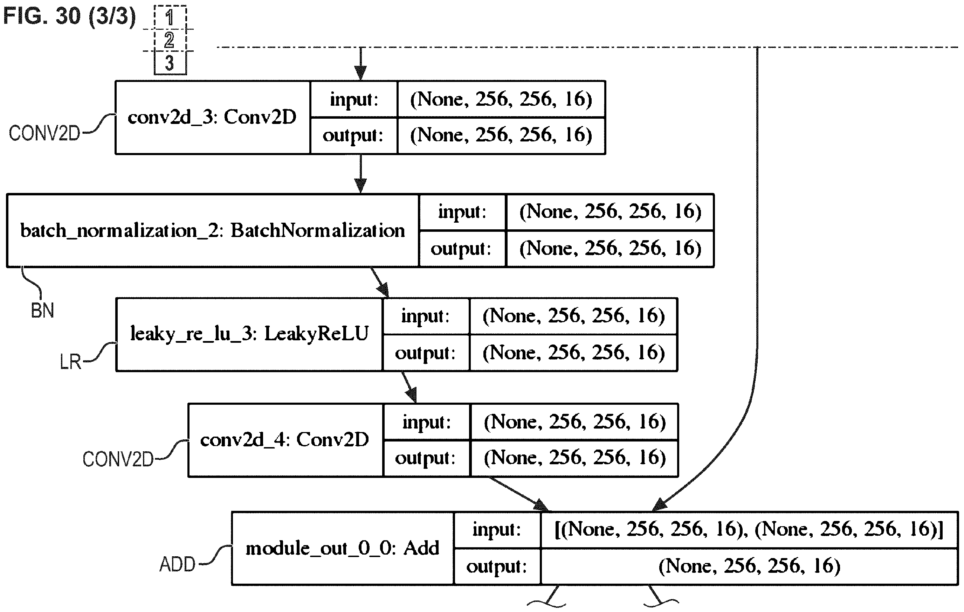

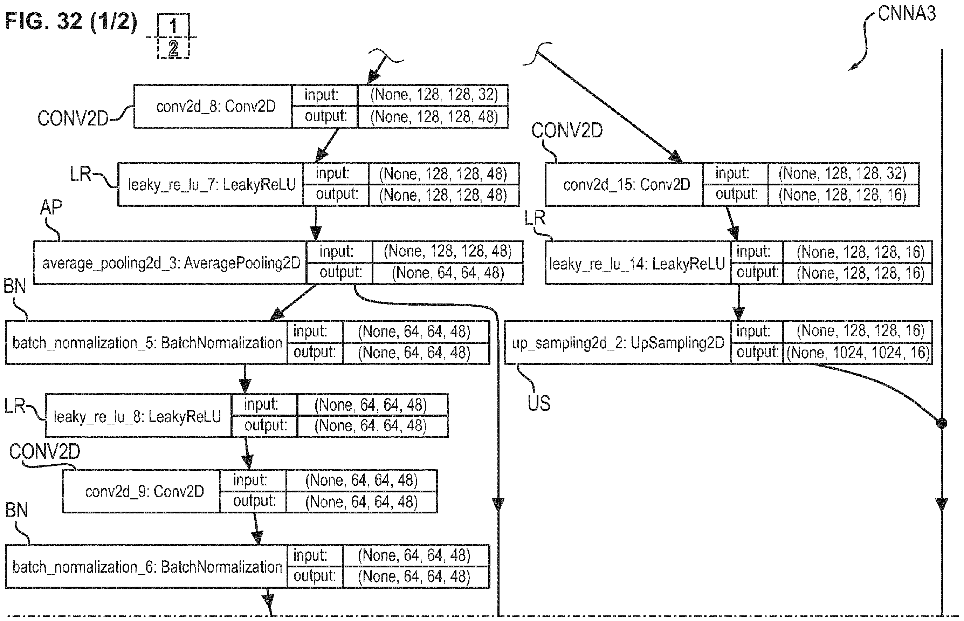

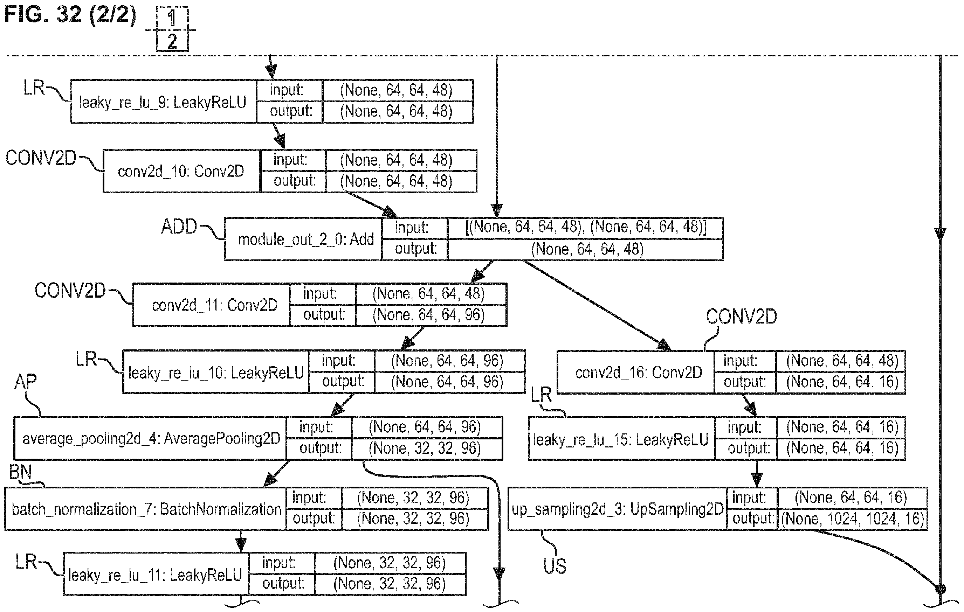

Figur 29 Schritte eines Verfahrens für eine Erfassung eines Fluoreszenzbildes gemäß einer bevorzugten Ausführungsform,

sowie Figuren 30 bis 33 Teilelemente einer Ausführungsform des zweiten Convolutional Neural Network.

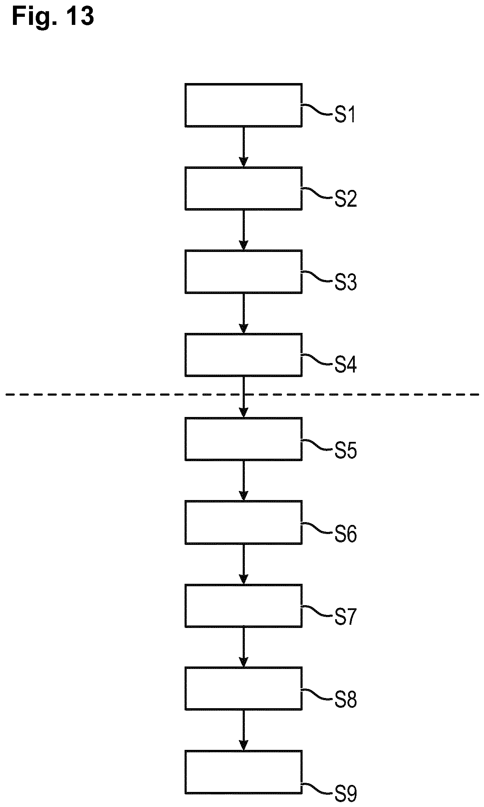

Figur 13 illustriert unterschiedliche Schritte zur Durchführung des erfindungsgemäßen Verfahrens. In einem Schritt S1 wird ein Substrat bereitgestellt, welches mehrere Crithidia luciliae Zellen aufweist. In einem Schritt S2 wird das Substrat mit der Patientenprobe inkubiert. Die Patientenprobe weist potenziell die Autoantikörper auf. In einem gemeinsamen Schritt S3 und S4 wird das Substrat mit sekundären Antikörpern inkubiert, welche jeweils mit einem vorzugsweise grünen Fluoreszenzfarbstoff markiert sind, inkubiert. In einem Schritt S5 erfolgt ein Erfassen eines Fluoreszenzbildes des Substrates in einem Farbkanal, welcher vorzugsweise ein Grünkanal ist, und welcher zu dem Fluoreszenzfarbstoff korrespondiert. In einem Schritt S6 erfolgt ein Identifizieren jeweiliger Teilbilder in dem einen Fluoreszenzbild, welche jeweils eine Crithidia luciliae Zelle repräsentieren, mittels eines ersten, vortrainierten Convolutional Neural Networks.

In einem Schritt S7 erfolgt ein jeweiliges Prozessieren wenigstens einer Teilmenge der jeweiligen Teilbildern mittels eines vortrainierten zweiten Convolutional Neural Network zur Bestimmung jeweiliger Bindungsmaße, welche einen jeweiligen Grad einer Bindung von Autoantikörpern in einem jeweiligen Kinetoplastbereich einer jeweiligen Crithidia luciliae Zelle eines jeweiligen Teilbildes indizieren. In einem Schritt S8 erfolgt ein Bestimmen eines Gesamtbindungsmaßes bezogen auf die Bindung von Autoantikörpern der Patientenprobe an doppelsträngige DNS in dem Substrat auf Basis der jeweiligen Bindungsmaße der jeweiligen Teilbilder. In einem Schritt S9 erfolgt vorzugsweise ein Bereitstellen des Gesamtbindungsmaßes, alternativ oder zusätzlich erfolgt ein Ausgeben und/oder Anzeigen des Gesamtbindungsmaßes.

Die Figur 14a zeigt ein beispielhaftes separates Prozessieren eines individuellen Teilbildbereiches TB1 durch ein zweites Convolutional Neural Network CNN2 zur Bestimmung eines individuellen Bindungsmaßes IBM1. Das Bindungsmaß IBM1 indiziert einen individuellen Grad einer Bindung von Autoantikörpern in einem individuellen Kinetoplastbereich einer individuellen Crithidia luciliae Zelle des individuellen Teilbildbereichs TB1.

Die Figur 14b illustriert ein Bestimmen des Gesamtbindungsmaßes GBM durch einen Ermittlungsschritt ERS, welcher dem Schritt S8 aus der Figur 13 entspricht, um das Gesamtbindungsmaß GBM auf Basis jeweiliger Bindungsmaße IBM1, IBM2 jeweiliger Teilbilder zu bestimmen.

Die Figur 5 illustriert ein Ausführungsbeispiel der erfindungsgemäßen Vorrichtung V1. Die Vorrichtung V1 weist eine Haltevorrichtung H für das Substrat S auf. Anregungslicht AL einer Anregungslichtquelle LQ wird über ein optisches Filter F1 vorgefiltert und dann mittels eines dichroitischen Spiegels SP1 durch eine Optik O hin zum Substrat geleitet. Entstehende Fluoreszenzstrahlung bzw. entstehendes Fluoreszenzlicht FL tritt dann vom Substrat her zurück durch das Objektiv O durch den dichroitischen Spiegel SP1 und durch ein Abschlussfilter F2 hindurch. Das optische Filter F2 filtert eine Wellenlänge der Anregungsstrahlung bzw. des Anregungslichtes AL heraus. Das Fluoreszenzlicht FL wird dann wenigstens einer Bilderfassungseinheit in Form einer Kamera K1 zugeführt. Über einen vorzugsweise zu verwendenden optischen Filter FG wird das Fluoreszenzlicht FL in einem ersten Farbkanal so gefiltert, dass das Fluoreszenzlicht FL nach Passieren des Filters FG einen sogenannten Grünkanal repräsentiert. Die Bilderfassungseinheit K1 ist dann vorzugsweise eine Monochromkamera. In einer alternativen Ausführungsform ist das optische Filter FG nicht vorhanden und die Kamera K1 ist eine Farbbildkamera, welche mit beispielsweise einer Bayer-Matrix ein Grünbild bzw. ein Grünfarbkanalbild herausfiltert und das Fluoreszenzbild als ein vorzugsweises Grünbild erfasst. Über eine Datenschnittstelle DS1 kann die Vorrichtung V1 Ergebnisdaten ED bereitstellen, welche das Gesamtbindungsmaß indizieren.

Eine Recheneinheit R ist ausgebildet, das Fluoreszenzbild in Form digitaler Daten BI entgegenzunehmen. Die Recheneinheit R ist ferner ausgebildet, die Schritte S5 bis S9 des erfindungsgemäßen Verfahrens durchzuführen.

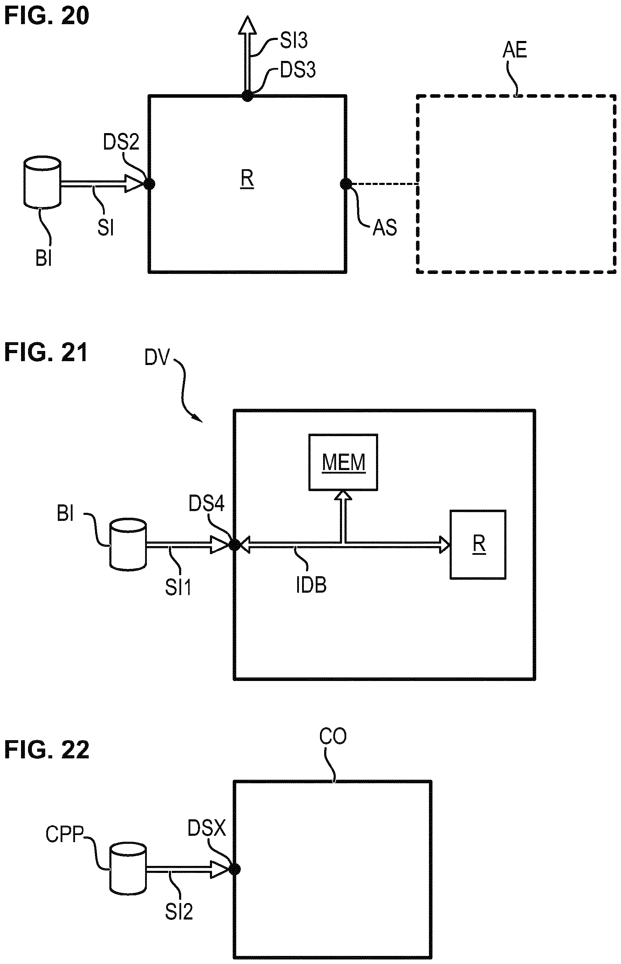

Eine erfindungsgemäße Recheneinheit R kann auch wie in der Figur 20 dargestellt realisiert sein. Hierbei nimmt die Recheneinheit R das Fluoreszenzbild über wenigstens eine Datenschnittstelle DS2 in Form wenigstens eines Datensignals SI entgegen. Nach Durchführung der entsprechenden Schritte S5 bis S8 des erfindungsgemäßen Verfahrens aus der Figur 13 hat die Recheneinheit R das Gesamtbindungsmaß der Bindung von Autoantikörpern der Patientenprobe an doppelsträngige DNS bestimmt. Vorzugsweise weist die Recheneinheit R eine Ausgabeschnittstelle AS zu einer Anzeigeeinheit AE auf, über welche das Gesamtbindungsmaß ausgegeben bzw. angezeigt werden kann. Vorzugsweise weist die Recheneinheit R eine weitere Datenschnittstelle DS3 hin zu einem Datennetzwerk auf, über welche die Recheneinheit das Gesamtbindungsmaßes über ein Datensignal SI3 bereitstellt. Die Datenschnittstellen DS2, DS3 der Recheneinheit R können auch eine gemeinsame Datenschnittstelle sein. Die Datenschnittstellen DS2, DS3 sind vorzugsweise Netzwerkdatenschnittstellen.

Die Recheneinheit R kann auch Teil einer erfindungsgemäßen Datennetzwerkvorrichtung DV sein, wie in der Figur 21 illustriert. Die Datennetzwerkvorrichtung DV weist wenigstens eine Datenschnittstelle DS4 zum Entgegennehmen des Fluoreszenzbildes BI mittels wenigstens eines Datensignals SI1 auf. Die Datennetzwerkvorrichtung DV weist die Recheneinheit R auf, welche vorzugsweise über einen internen Datenbus IDB mit einer Speichereinheit MEM und der Datenschnittstelle DS4 verbunden ist. Die Recheneinheit R ist in jener Weise ausgebildet, wie zuvor in Bezug auf die Figur 20 beschrieben. Die Datennetzwerkvorrichtung DV kann ein einzelner Computer sein oder aber eine sogenannte Cloud-Lösung. Die Datennetzwerkvorrichtung DV führt also mittels der Recheneinheit R ein erfindungsgemäßes Verfahren zur digitalen Bildverarbeitung durch, bei welchem das Fluoreszenzbild BI entgegengenommen wird und bei welchem die Recheneinheit R die Schritte S5 bis S8 aus der Figur 13 durchführt.

Die Figur 22 illustriert ein erfindungsgemäßes Computerprogrammprodukt CPP, welches Befehle umfasst, die bei der Ausführung des Programms durch einen Computer CO diesen veranlassen, ein erfindungsgemäßes Verfahren der digitalen Bildverarbeitung durchzuführen.

Das Computerprogrammprodukt CPP kann in Form eines Datenträgersignals SI2 bereitgestellt werden und durch einen Computer CO mittels einer an dem Computer CO befindlichen Datenschnittstelle DSX entgegengenommen werden. Das Datenträgersignal SI2 überträgt also das Computerprogrammprodukt CPP.

Die Figur 15 illustriert das Prozessieren des Fluoreszenzbildes SG in dem Schritt S6 zur Gewinnung der Teilbilddaten TBD, welche die Teilbilder repräsentieren bzw. indizieren. Die Figur 3 zeigt das Fluoreszenzbild SG mit indizierten Teilbildern TB1, TBA, TBB, TBX, welche mittels jeweiliger Rechtecke in dem Fluoreszenzbild SG eingetragen sind. Weitere, nicht näher benannte Teilbilder sind mittels weiterer Rechtecke in dem Fluoreszenzbild SG eingetragen. Die Figur 4 zeigt das Fluoreszenzbild SG mit speziell ausgewählten Teilbildern. Beispielsweise ist das Teilbild TBX aus der Figur 3 keines, welches zur Bestimmung einer Teilmenge bzw. Untermenge von Teilbildern ausgewählt wurde, da dieses Teilbild TBX aus der Figur 3 in der Figur 4 nicht mehr indiziert ist. Dieser Schritt des Auswählens einer Teilmenge von Teilbildern wird im Späteren noch genauer erläutert.

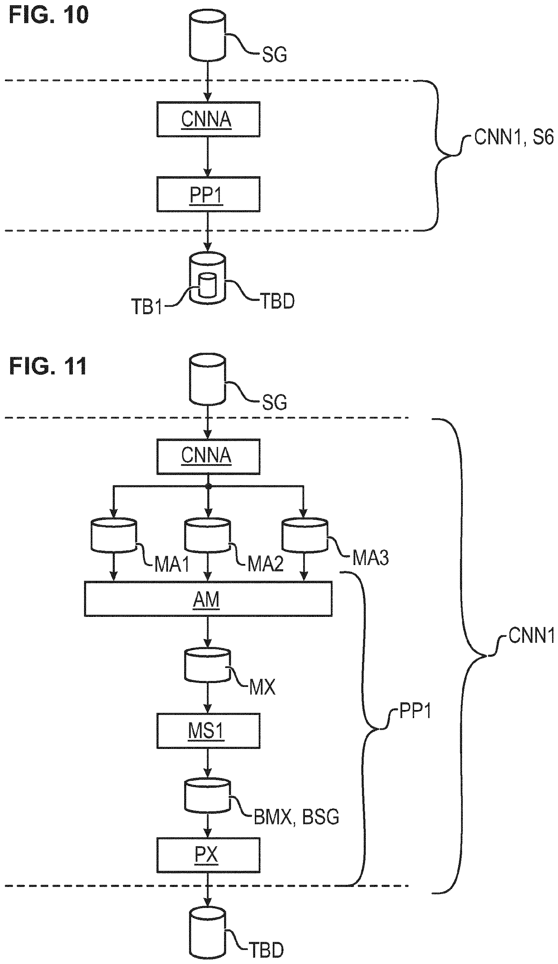

Der in der Figur 15 dargestellte Schritt S6 ist in der Figur 10 näher erläutert. Der Schritt S6 wird durch das erste Convolutional Neural Network CNN1 durchgeführt, welches vorzugsweise ein Convolutional Neural Network CNNA sowie ein sogenanntes Postprocessing PP1 als jeweilige Teilschritte aufweist. Die mittels des ersten, vortrainierten Convolutional Neural Networks CNN1 ermittelten Teilbilddaten TBD weisen dann die identifizierten Teilbilder wie beispielsweise das Teilbild TB1 auf bzw. indizieren diese Teilbilder.

Die Figur 11 zeigt weitere Details zu dem ersten Convolutional Neural Network CNN1.

Das in dem ersten Convolutional Neural Network CNN1 verwendete Convolutional Neural Network CNNA als erster Teilschritt wird später unter Bezugnahme auf die Figuren 30 bis 33 noch näher erläutert.

Dieses Convolutional Neural Network CNNA nimmt das Fluoreszenzbild SG entgegen und ordnet diese jeweiligen Bildsegmente bzw. diese jeweiligen Pixel des Fluoreszenzbildes SG jeweiligen Bildsegmentklassen zu. Diese Bildsegmentklassen bilden eine Gruppe von Bildsegmentklassen, welche wenigstens die Bildsegmentklassen Zelle und Hintergrund umfasst. Vorzugsweise umfasst diese Gruppe der Bildsegmentklassen die Bildsegmentklassen Zelle, Zellrand und Hintergrund.

Das Fluoreszenzbild SG kann als eine Information

$$SG(m), m = 1 \dots M, M = 1048576$$

aufgefasst werden, bei welcher der Index m der Pixelindex ist bzw. der Bildsegmentindex und bei welcher bei einer Auflösung von 1024x1024 sich der Parameter M zu dem Wert M=1048576 ergibt. Der Fachmann erkennt, dass bei Auswahl einer anderen Bildauflösung für das Fluoreszenzbild SG sich ein anderer Wert für den Parameter M ergibt. Das Fluoreszenzbild kann auch herunterskaliert werden.

Das Convolutional Neural Network CNNA ermittelt dann jeweilige Matrizen bzw. Maps MA1, MA2, MA3 für die jeweiligen Bildsegmentklassen. Es kann also eine Map bzw. eine Matrix MA1, MA2, MA3 für vorzugsweise K=3 Bildsegmentklassen geschrieben werden als

$$MAk, k = 1 \dots K$$

Die Auflösung einer Map ist vorzugsweise von gleicher Auflösung wie für das Fluoreszenzbild SG, sodass gilt

$$MAk(m), m = 1 \dots M$$

mit vorzugsweise M = 1048567.

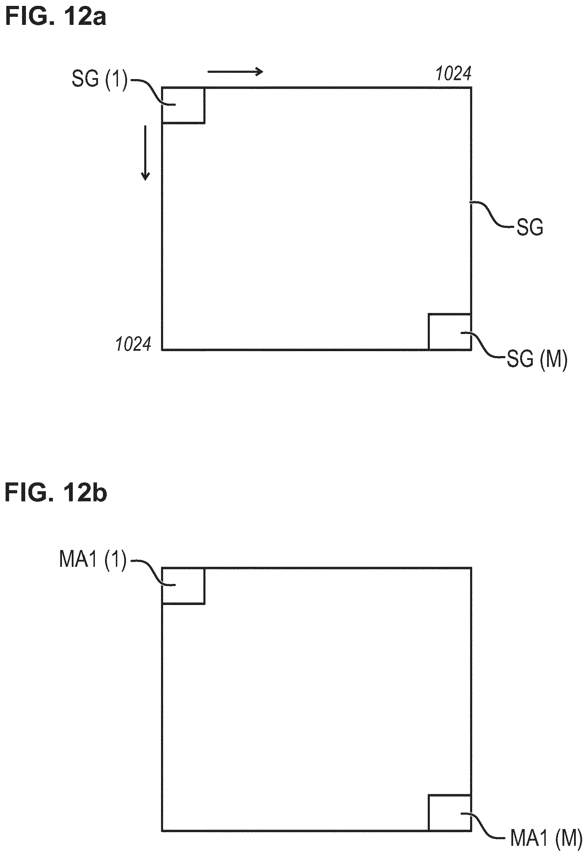

Die Figur 12a zeigt hierzu eine beispielhafte Darstellung des Fluoreszenzbildes SG mit einer Indizierung des ersten Pixels SG (1) und des letzten Pixels SG (M).

Figur 12b zeigt hierzu dann die Map bzw. Matrix MA1 der ersten Bildsegmentklasse in korrespondierender Weise. Entsprechende korrespondierende Maps bzw. Matrizen MA2, MA3 können für die weiteren Bildsegmentklassen dann aufgestellt werden.

In einem einzelnen Eintrag MA1 (1) weist dann die Map MA1 ein Konfidenzmaß auf, welches indiziert, dass das korrespondierende Bildsegment bzw. das Pixel SG (1) zu der ersten Bildsegmentklasse der Map bzw. der Matrix MA1 gehört.

Entsprechend kann dies für die Maps bzw. Matrizen MA2, MA3 der entsprechenden zweiten bzw. dritten Bildsegmentklasse aufgestellt werden.

Die in der Figur 11 ebenfalls dargestellten Maps bzw. Matrizen MA1, MA2, MA3 werden dann in einem weiteren Schritt AM einer sogenannten Argmax-Operation unterzogen, welche dann für ein jeweiliges Bildsegment bzw. ein jeweiliges Pixel aus dem Fluoreszenzbild SG ermittelt, zu welcher Bildsegmentklasse dieses Pixel bzw. dieses Bildsegment am wahrscheinlichsten gehört.

Diese erfolgt durch Bildung einer Klassifikationsmatrix MX mit

$$MX(m), m = 1 \dots M$$

Es wird die dann zur Bestimmung der Klassifikationsmatrix MX die Argmax-Operation ausgeführt entsprechend

$$MX(m) = \underset{k}{argmax} \{MAk(m), \dots, MAK(m)\}$$

Die Werte der Klassifikationsmatrix MX bzw. ein einzelner Wert MX(m) ist dann aus der Menge {1, 2, 3}, wobei beispielsweise eine 1 indiziert, dass das korrespondierende Bildsegment bzw. das korrespondierende Pixel aus dem Fluoreszenzbild SG zu der Klasse Hintergrund gehört. Beispielsweise indiziert der Wert 2, dass das korrespondierende Pixel bzw. das korrespondierende Bildsegment aus dem Fluoreszenzbild SG zu einem Zellrand gehört. Vorzugsweise indiziert der Wert 3, dass das korrespondierende Pixel bzw. das korrespondierende Bildsegment aus dem Fluoreszenzbild zu der Klasse Zelle bzw. Zellleib gehört.

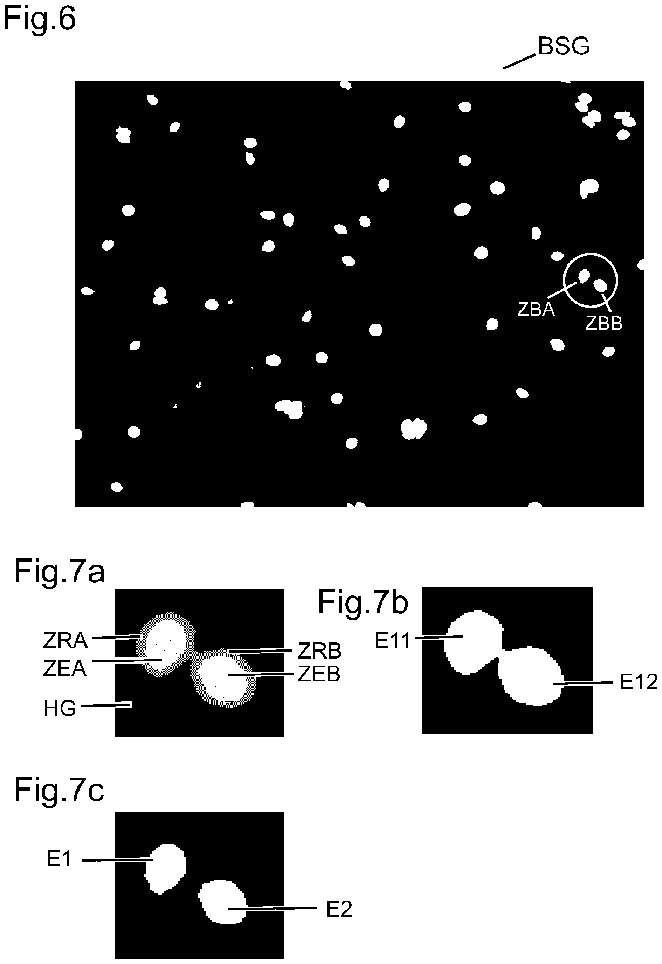

Es erfolgt dann noch vorzugsweise ein sogenannter Mapping Step MS1, welcher die Klassifikationsmatrix MX überführt in eine binäre Klassifikationsmatrix BMX, welche auch als binäre Bildinformation BSG aufgefasst werden kann. Ein solches Resultat einer binären Bildinformation BSG ist in der Figur 6 dargestellt. Eine solche binäre Bildinformation BSG bzw. BMX kann auch geschrieben werden als

$$BMX(m), m = 1 \dots M.$$

Das Mapping in dem Mapping Step MS1 erfolgt dann vorzugsweise derart, dass all jene Bildsegmente bzw. Pixel mit Index m aus der Matrix MX, welche dem Hintergrund zugeordnet sind, also der Klasse 1 angehören, dann in der Binärmatrix BMX final auch dem Hintergrund zugeordnet werden und auf den Wert 0 gesetzt werden. All jene Bildsegmente bzw. Pixel, welche in der Matrix MX dem Zellrand zugeordnet wurden, also der Klasse 2 angehören, werden in der Binärmatrix BMX final auch dem Hintergrund zugeordnet und auf den Wert 0 gesetzt. All jene Bildsegmente bzw. Pixel, welche in der Matrix MX der Zelle bzw. dem Zellleib zugeordnet wurden, also der Klasse 3 angehören, werden in der Binärmatrix BMX final als Zellbereich zugeordnet und auf den Wert 1 gesetzt. Dies erfolgt gemäß der Vorschrift:

$$BMX(m) = \begin{cases} 0 \ \text{für } MX(m) = 1 \lor 2 \\ 1 \ \text{für } MX(m) = 3 \end{cases}$$

Die Figur 7a zeigt für ganz bestimmte Zellen ZBA, ZBB aus der Figur 6 die Klassifikationsergebnisse aus der Mapping Matrix MX vor dem Mapping Step MS1. Pixel, welche dem Hintergrund zugeordnet sind, sind schwarz dargestellt. Pixel, welche dem Zellrand zugeordnet sind, sind als ZRA grau dargestellt. Pixel, welche dem Zellleib bzw. der Zelle zugeordnet sind, sind weiß als ZEA dargestellt. Dieses erfolgt in der Figur 7a auch für die Zelle ZBB mittels der Klassifikation der Pixel bzw. Bildsegmente und ist mittels der Information ZEB und ZRB dargestellt. Aufgrund des Mapping Steps MS1 erfolgt dann eine Separation der Zellen ZBA und ZBB in vorteilhafter Weise, sodass die finale Information aus der Figur 7c ernannt wird, sodass diese beiden Zellen ZBA, ZBB getrennt werden konnten und nun als Bereiche E1, E2 ermittelt werden konnten. Erfolgt das Mapping in nicht korrekter Weise, indem z.B. der Zellrand ZRA, ZRB dem Zellbereich bzw. Zellleib ZEA, ZEB zugeordnet wird, so kann es dazu kommen, dass die beiden Zellen ZBA, ZBB als ein Gesamtbereich aufgefasst werden, welche in der Figur 7 als Bereich mit den Unterbereichen E11, E21 dargestellt ist.

Das Binärbild BSG aus der Figur 6 wird dann in einem weiteren Post-Processing Schritt PX aus der Figur 11 prozessiert, um die Teilbilder zu ermitteln und als Teilbilddaten TBD bereitzustellen. Auf den Schritt des Post-Processing PX wird nun genauer eingegangen. Es wird zunächst mittels des Verfahrens "Satoshi Suzuki and others. Topological structural analysis of digitized binary images by border following. Computer Vision, Graphics, and Image Processing, 30(1):32-46, 1985" nachbearbeitet, um relevante Konturen in dem Bild BSR aufzufinden. Ferner werden dann sogenannte bounding boxes mittels der Funktion *boundingRect* aus der Sektion "Structural analysis and shape descriptors" aus der Datenbank OpenCV (https://opencv.org) nachbearbeitet. Die bounding boxes haben hierbei eine Größe von 150x150 Pixel.

Vorzugsweise kann das Fluoreszenzbild SG aus der Figur 1 in seiner Qualität bewertet werden, indem nach Ermittlung der Konturen in dem Binärbild BSG aus der Figur 6 wenigstens zehn identifizierte Zellbereiche bzw. Teilbilder eine bestimmte Morphologie als auch eine gewisse Mindestgröße aufweisen müssen. Nur in dem Fall, dass wenigstens zehn Zellen diese Kriterien beiden erfüllen, erfolgt dann eine weitere Prozessierung der Fluoreszenzbilder ohne Fehlerausgabe in dem Verfahren. Erfüllen weniger als zehn identifizierte Zellbereiche bzw. Teilbilder die beiden Kriterien nicht, so wird das Verfahren vorzugsweise abgebrochen und eine Fehlermeldung ausgegeben.

Die Figuren 30, 31, 32 sowie 33 zeigen Teilelemente CNNA1, CNNA2, CNNA3, CNNA4, welche gemeinsam betrachtet in entsprechender Abfolge das Convolutional Neural Network CNNA aus den Figuren 10 bzw. 11 bilden. Das Fluoreszenzbild SG wird gemäß Figur 30 von dem Teil CNNA1 des Convolutional Neural Networks entgegengenommen. Das gesamte Convolutional Neural Network CNNA wird, wie aus den Figuren 30 bis 33 ersichtlich wird, durch eine Abfolge einer Mehrzahl von Schritten bzw. Prozessierungsoperation eines neuronalen Netzes ausgebildet, wobei unterschiedliche Arten von Schritten vorkommen. Der erste Schritt INL stellt das sogenannte Inputlayer dar, in welchem das Fluoreszenzbild SG von beispielsweise einer Dimensionalität 1024×1024 entgegengenommen wird. Das Fluoreszenzbild SG aus der Figur 1 kann vorzugsweise eine erste Dimensionalität bzw. Auflösung aufweisen, welche vorzugsweise höher ist als die Auflösung 1024 × 1024, und für den Zweck einer Vorverarbeitung auf eine zweite Dimensionalität bzw. Auflösung von 1024× 1024 herunterskaliert bzw. umskaliert werden. Es erfolgt vorzugsweise in einem Skalierungsschritt SKL eine Skalierung der Helligkeitswerte des Fluoreszenzbildes SG auf den Wertebereich 0 bis 1.

Für jeden einzelnen abfolgenden Schritt ist im Detail angegeben, von welcher Dimensionalität eine Eingangsgröße ist und von welcher Dimensionalität eine Ausgangsgröße eines Schrittes ist. Hierbei kann für jeden einzelnen Schritt in der oberen Zeile "Input" in den darauffolgenden Klammern durch den zweiten und den dritten Eintrag die Dimensionalität der Eingangsgröße entnommen werden. Ferner kann durch den vierten Eintrag in den Klammern entnommen werden, wie viele Eingangsgrößen in einem einzelnen Schritt entgegengenommen werden. Beispielsweise wird in dem Schritt CS1 eine zweidimensionale Faltung durchgeführt, sodass die Ausgangsgröße "Output" Input 16 verschiedene Ausgangsgrößen generiert, da 16 Faltungskernels verwendet werden für die eine einzige Eingangsgröße, wobei die Ausgangsgrößen jeweils eine Dimensionalität von $512 \times 512$ aufweisen. Hierdurch kann also ein Fachmann für jeden Prozessierungsschritt aus den hier angegebenen Parametern klar ableiten, von welcher Dimensionalität Eingangs- und Ausgangsgrößen sind und wie viele Faltungskernel gegebenenfalls verwendet werden müssen.

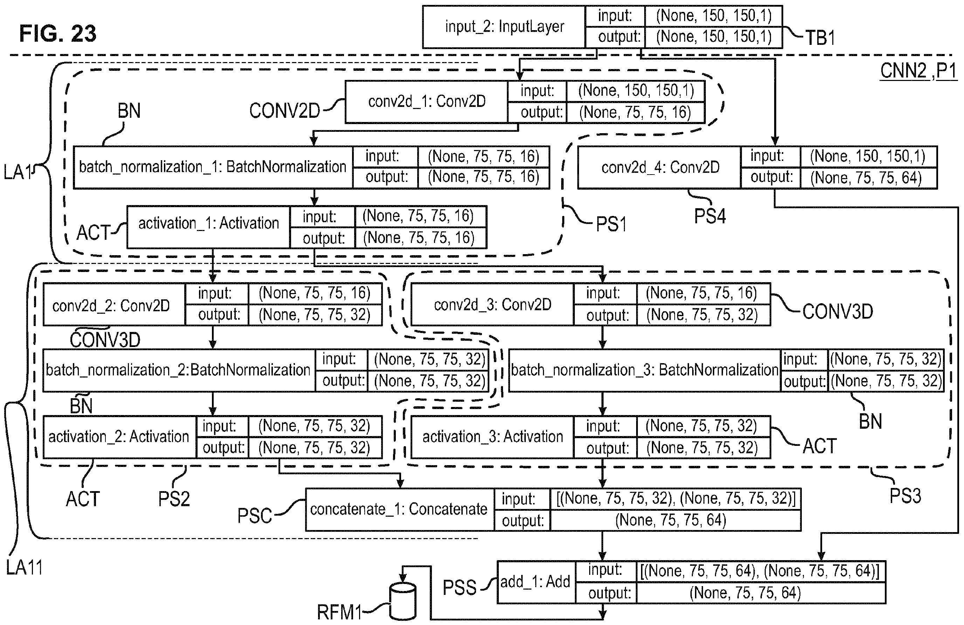

Derartige Ausführungen befinden sich auch in der späteren Beschreibung unter Bezug auf das zweite Convolutional Neural Network CNN2 unter Bezugnahme auf die Figuren 23, 24, 25, 26.

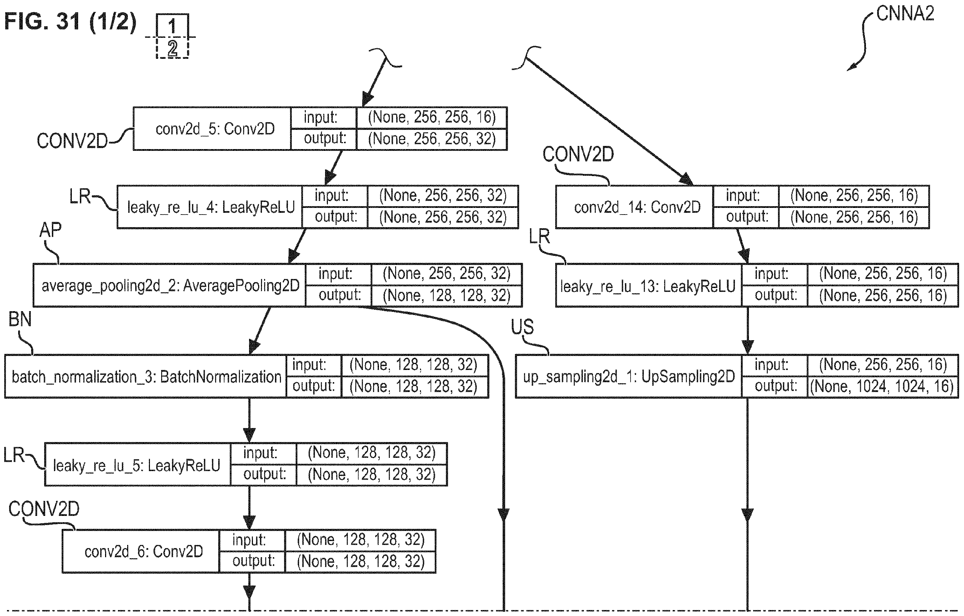

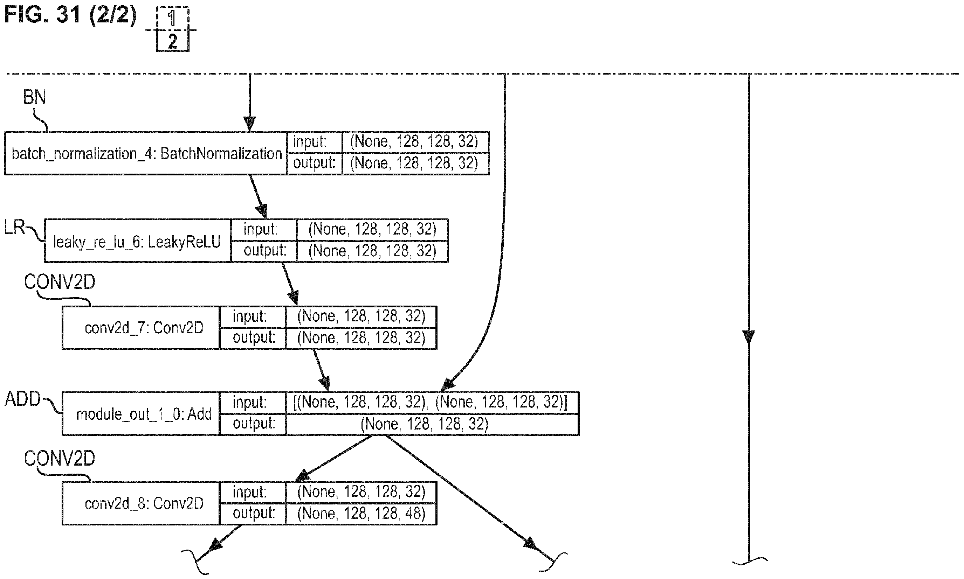

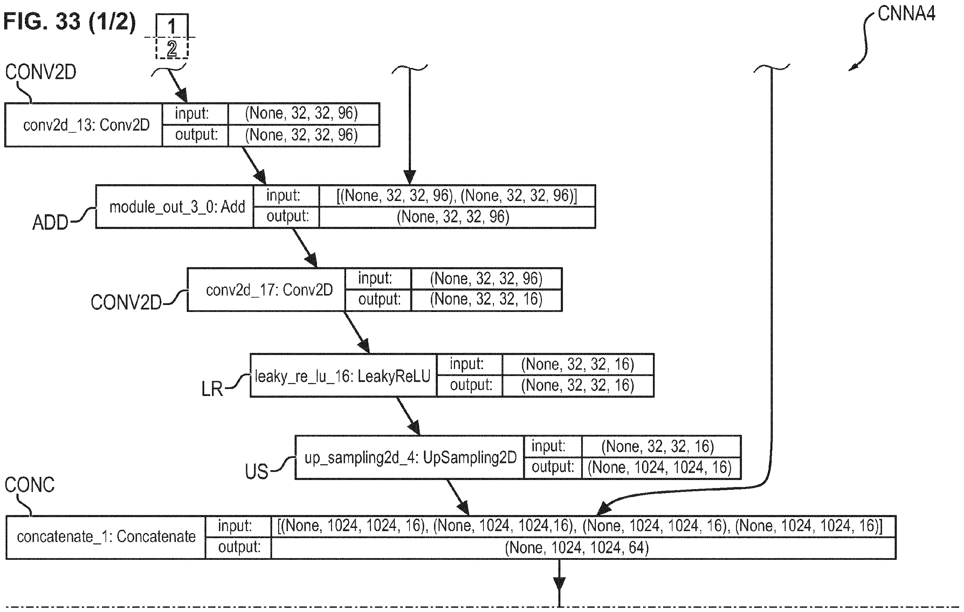

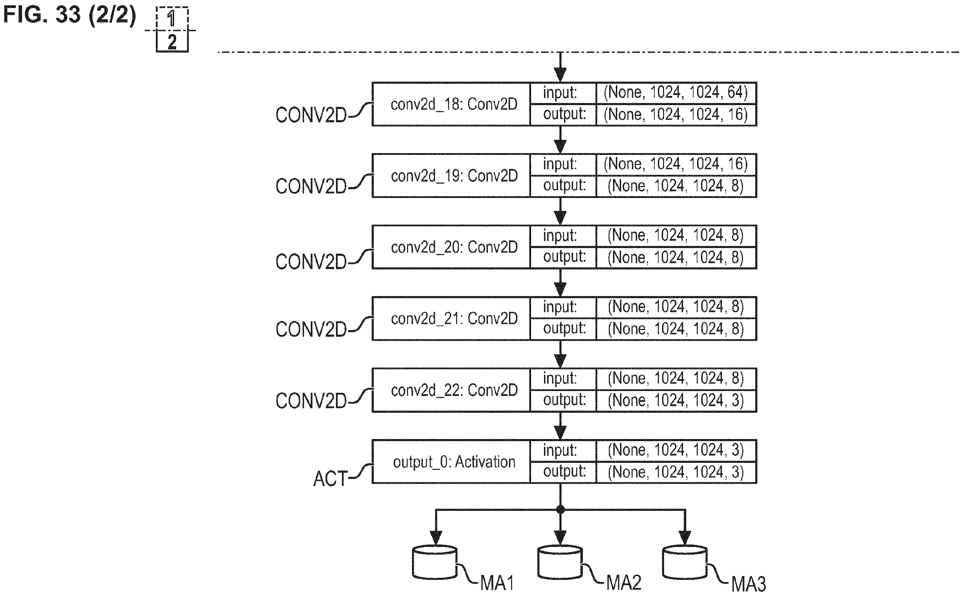

Das Convolutional Neural Network CNNA mit seinen Bestandteilen CNNA1 bis CNNA4 der Figuren 30 bis 32 weist unterschiedliche Arten von Prozessierungsschritten auf. Ein Prozessierungsschritt CONV2D stellt eine zweidimensionale Faltung mit einer bestimmten Anzahl von Faltungskernel dar. Eine Funktion LR stellt eine Aktivierungsfunktion als eine Leaky Rectified Linear Unit Activation Function dar. Eine Funktion AP stellt ein sogenanntes Average Pooling dar. Eine Funktion BN stellt eine sogenannte Batch Normalization dar. Ein Prozessierungsschritt bzw. eine Funktion ADD stellt eine elementweise Addition von mehreren Eingangsgrößen zur Generierung einer einzelnen Ausgangsgröße dar. Ein Prozessierungsschritt US, wie beispielsweise in der Figur 31 dargestellt, stellt ein sogenanntes Up-Sampling dar. Ein Prozessierungsschritt ACT aus der Figur 33 stellt eine sogenannte Aktivierungsfunktion dar, welche beispielsweise durch eine Sigmoidfunktion oder eine sogenannte Softmaxfunktion gegeben sein kann. Am Ausgang des neuronalen Netzes CNNA bzw. am Ausgang des Teilnetzes CNNA4 ergeben sich dann die drei Matrizen MA1, MA2, MA3, welche bereits unter Bezug auf die Figur 11 näher erläutert wurden.

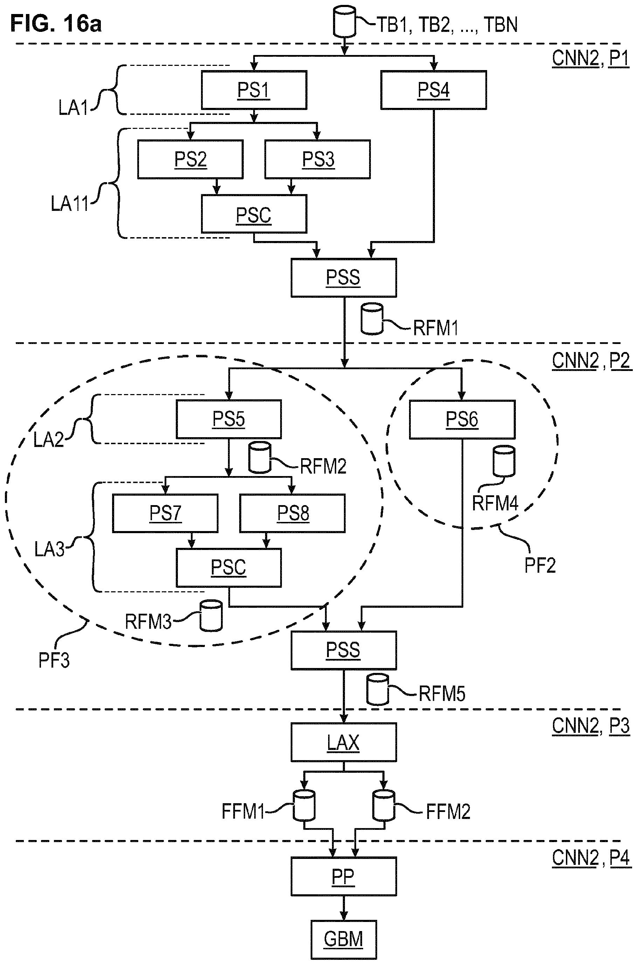

Die Figur 16a illustriert unterschiedliche Prozessierungsebenen P1, P2, P3, P4 des zweiten Convolutional Neural Network CNN2 zur Bestimmung des Gesamtbindungsmaßes GBM auf Basis mehrerer Teilbildbereiche TB1, ..., TBN wie zuvor ausgeführt. Das CNN2 prozessiert hierbei jeweilige Teilbildbereiche TB1, TB2, ..., TBN mit Index N für sich, um dann für einen jeweiligen Teilbildbereich TB1, TB2, ..., TBN ein jeweiliges individuelles Bindungsmaß IBM1, IBM2, ..., IBMN zu generieren, wie zuvor unter Bezug auf die Figur 14a verdeutlicht.

Die Figur 16a zeigt hierbei, dass im Zuge dieser Bestimmung der jeweiligen individuellen Bindungsmaße für jedes einzelne Teilbild TB1, ... dann eine jeweilige finale Feature Map FFM1 als auch vorzugsweise eine weitere finale Feature Map FFM2 generiert wird. Das CNN2 kann so ausgestaltet werden, dass lediglich eine einzige finale Feature Map FFM1 eines einzigen Kanals vorgesehen ist und generiert wird.

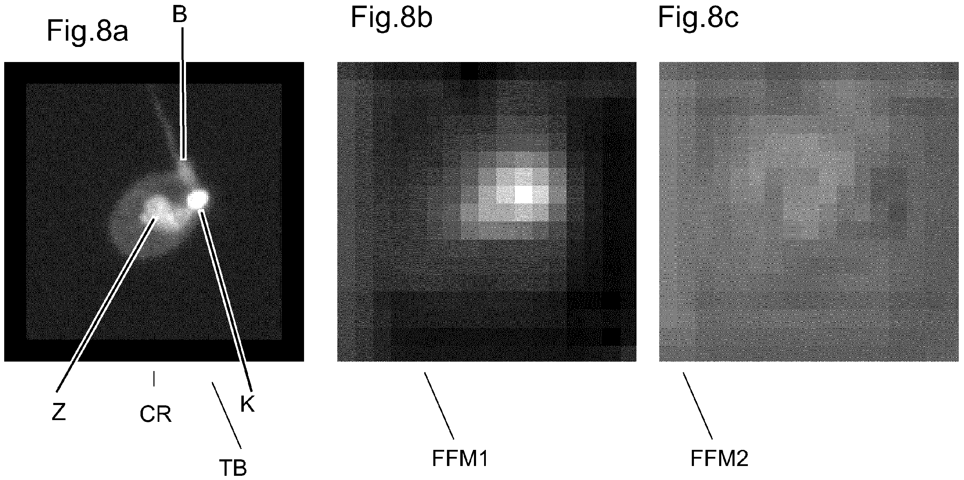

Das Ergebnis der Prozessierungsebene P3 aus der Figur 16a sind also für ein Teilbild TB mit Index N eine finale Feature Map FFM1 als auch vorzugsweise eine weitere finale Feature Map FFM2. Die erste finale Feature Map FFM1 ist für das Teilbild TB aus der Figur 8a in der Figur 8b dargestellt. Die zweite finale Feature Map FFM2 für das Teilbild TB ist in der Figur 8c dargestellt.

Das Convolutional Neural Network CNN2 löst die Aufgabe einer sogenannten "Single-Label Classification", also ob der Kinetoplast in dem Teilbild eine Färbung aufweist oder nicht. Die finale Feature Map FFM1 repräsentiert eine Aktivierung in einem ersten Klassifikationskanal bezogen auf eine positive Entscheidung der "Single Label Classification", also dass der Kinetoplastbereich gefärbt ist. Die vorzugsweise vorzusehende finale Feature Map FFM2 repräsentiert die dazu korrespondierende Aktivierung bezogen auf eine negative Entscheidung, also dass der Kinetoplast nicht wesentlich gefärbt ist.

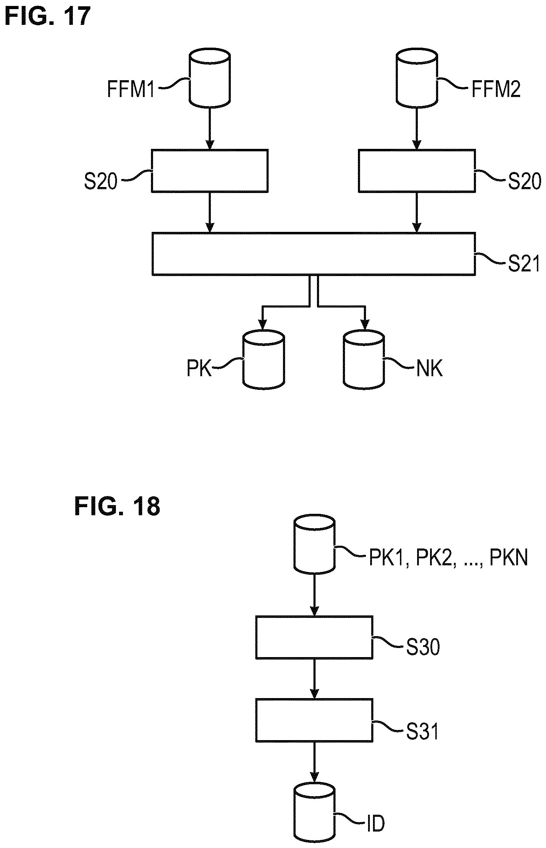

Gemäß der Figur 17 wird auf Basis der ersten finalen Feature Map FFM1 als auch vorzugsweise der zweiten finalen Feature Map FFM2 dann ein positives Konfidenzmaß PK bestimmt bezogen auf eine Färbung des Kinetoplastbereiches bzw. auf ein Vorliegen einer Bindung von Autoantikörpern in dem entsprechenden Kinetoplastbereich K des betrachteten Teilbildes TB. Vorzugsweise wird auf Basis der ersten finalen Feature Map FFM1 als auch vorzugsweise der zweiten finalen Feature Map FFM2 ein negatives Konfidenzmaß NK bestimmt bezogen auf eine Färbung des Kinetoplastbereiches bzw. auf ein Vorliegen einer Bindung von Autoantikörpern in dem entsprechenden Kinetoplastbereich K des betrachteten Teilbildes TB.

Vorzugsweise kann auf Basis lediglich der ersten finalen Feature Map ein Konfidenzmaß bezogen ein Vorliegen einer Bindung von Autoantikörpern in einem jeweiligen Kinetoplastbereich für das jeweilige Teilbild TB bestimmt werden, ohne die zweite finale Feature Map FFM2 verwenden zu müssen. Es kann dann beispielsweise in einem Schritt S20 die Feature Map FFM1 einem sogenannten Max-Pooling zugeführt werden, welches für die finale Feature Map FFM1 den maximalen Pixelwert als einen einzelnen Skalarwert ermittelt. Vorzugsweise kann dieser Skalarwert als das Konfidenzmaß verwendet werden. Vorzugsweise kann auf Basis dieses Skalarwertes dann beispielsweise mittels einer sogenannten Sigmoid-Funktion ein Wert als das Konfidenzmaß ermittelt werden. Vorzugsweise kann auf Basis dieses Skalarwertes dann beispielsweise mittels einer sogenannten Rectified Linear Unit Activation Funktion ein Wert als das Konfidenzmaß ermittelt werden.

Vorzugsweise werden beide jeweiligen Feature Maps FFM1, FFM2 in jeweiligen Schritten S20 jeweils einem sogenannten Max-Pooling zugeführt, welches für eine jeweilige finale Feature Map jeweils den maximalen Pixelwert als einen jeweiligen einzelnen Skalarwert ermitteln. Auf Basis dieser Skalarwerte kann dann in einer sogenannten Softmax-Funktion in einem Schritt S21 eine positive Wahrscheinlichkeit PK als das Konfidenzmaß bezogen auf das Vorliegen der Bindung von Autoantikörpern in dem Kinetoplastbereich bzw. bezogen auf eine Färbung des Kinetoplastbereiches bestimmt werden. Die negative Wahrscheinlichkeit NK kann ebenfalls durch die Softmax-Funktion bestimmt werden. Positive Wahrscheinlichkeit PK und negative Wahrscheinlichkeit NK bilden vorzugsweise addiert eine Summe vom Wert 1. Auf diese Weist kann also für ein jeweiliges Teilbild TB durch Ermittlung der ersten finalen Feature Map FFM1 und vorzugsweise auch der zweiten finalen Feature Map FFM2 dann gemäß der Figur 17 ein jeweiliges Konfidenzmaß bezogen auf das Vorliegen der Bindung von Autoantikörpern in den Kinetoplastbereich des jeweiligen Teilbildes bestimmt werden.

Zu der Softmax-Funktion alternative Funktionen sind beispielsweise die Sigmoid-Funktion, die Rectified Linear Unit Activation Funktion oder die Leaky Rectified Linear Unit Activation Funktion.

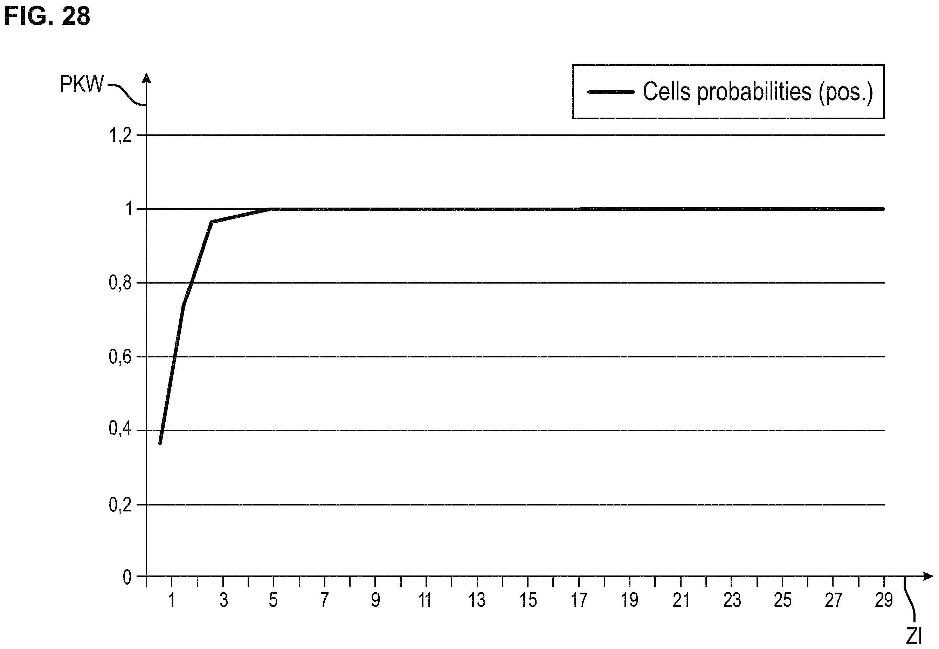

Die Figur 18 zeigt Schritte für ein Auswählen einer Teilmenge der identifizierten Teilbilder aus dem Fluoreszenzbild auf Basis der jeweiligen positiven Konfidenzmaße der jeweiligen Teilbilder. Die jeweiligen positiven Konfidenzmaße PK1, PK2, PK3,....., PKN mit Index 1, ..., N von N jeweiligen Teilbildern werden in einem Schritt S30 hinsichtlich ihrer Wertigkeit aufsteigend sortiert. Beispielhaft zeigt die Figur 28 für 29 verschiedene Crithidia luciliae Zellen bzw. für 29 verschiedene Teilbilder die jeweils zugehörigen positiven Konfidenzmaße PK, welche bezüglich ihrer Werte PKW in aufsteigender Wertigkeit über einem entsprechenden Sortierindex 1, ..., 29 aufgetragen sind. Es werden dann in dem Schritt S31 jene Konfidenzmaße und ihre jeweils dazugehörigen Teilbilder ausgewählt, deren zugehörige Konfidenzmaßwerte die 50 % der höchsten Konfidenzmaßwerte PKW darstellen. Diese Untermenge der dazugehörigen Teilbilder wird dann durch Ausgabe der entsprechenden Teilbild-Indizes mittels eines Datensatzes ID ausgegeben bzw. indiziert. Anhand der Indizes aus dem Datensatz ID können dann die entsprechenden Teilbilder und ihre zugehörigen Bindungsmaße für eine Bestimmung des Gesamtbindungsmaßes ausgewählt werden.

Das Gesamtbindungsmaß wird dann auf Basis jener Bindungsmaße bestimmt, welche zu den ausgewählten Teilbildern gehören. Eine solche Ermittlung des Gesamtbindungsmaßes findet insbesondere in einem Schritt des Post-Processing PP innerhalb der vierten Prozessierungsebene P4 des zweite Convolutional Neural Network statt, wie in Figur 16a dargestellt.

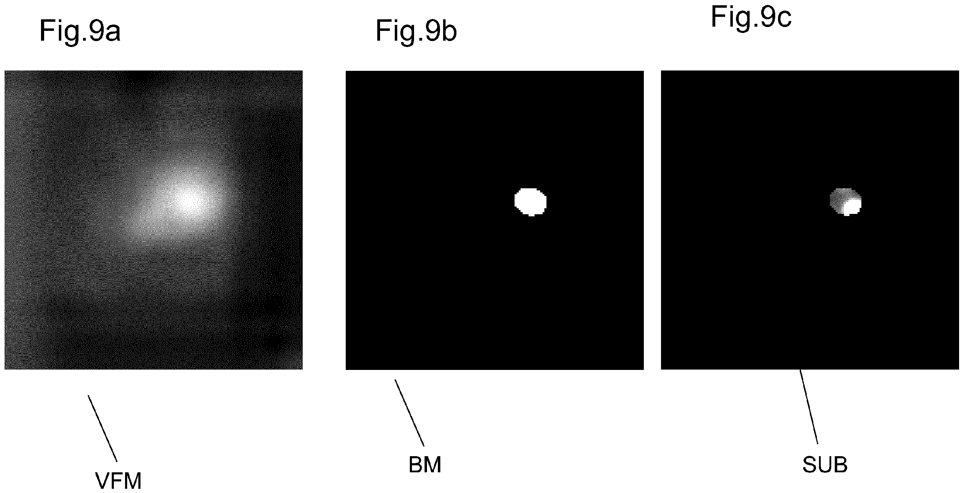

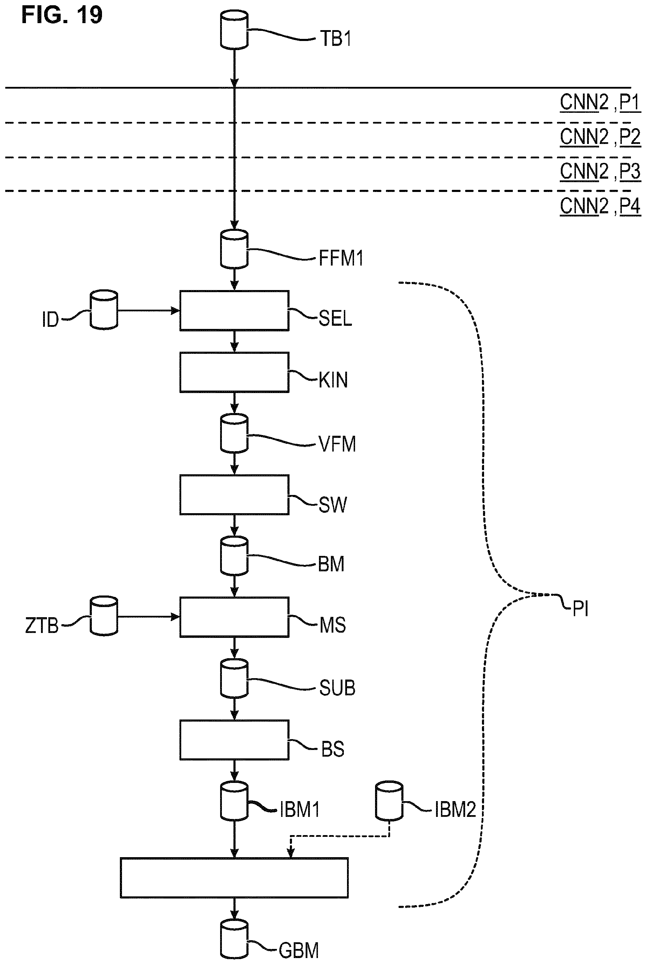

Die Figur 19 zeigt noch einmal eine beispielhafte Prozessierung eines einzelnen Teilbildes TB1 durch das zweite Convolutional Neural Network CNN2. Zunächst wird das Teilbild TB1 durch die ersten drei Prozessierungsebenen P1 bis P3 des zweiten Convolutional Neural Network prozessiert, sodass die finale Feature Map FFM1 und vorzugsweise auch die zweite finale Feature Map bereitgestellt werden. In einem Auswahlschritt SEL erfolgt dann auf Basis der unter Bezug auf die Figuren 17 und 18 erläuterten Indexdaten ID das potentielle Auswählen des Teilbildes TB1 in die Teilmenge der Teilbilder. Ist das Teilbild TB1 in diese Teilmenge ausgewählt worden, so erfolgt dann das weitere Prozessieren als Post-Processing PP in der vierten Prozessierungsebene P4. Hier erfolgt nach Bestimmen der positiven Konfidenzmaße und nach dem Auswählen der Teilmenge der Teilbilder dann ein jeweiliges Post-Processing PI der jeweiligen ersten Feature Maps FFM1 der ausgewählten jeweiligen Teilbilder. Beispielhaft ist hier also für ein individuelles Teilbild TB1 ein Post-Processing PI zur Bestimmung eines individuellen Bindungsmaßes IBM1 gezeigt. Hierbei erfolgt für das Teilbild TB1 ein Selektieren eines jeweiligen Subbildes auf Basis der finalen Feature Map FFM1. Das Subbild präsentiert einen jeweiligen Kinetoplastbereich der entsprechenden Crithidia luciliae Zelle in dem Teilbild. Ein solches Teilbild TB ist beispielhaft in Figur 8a dargestellt. Die zugehörige erste Feature Map FFM1 aus der Figur 8b wird dann in einem Schritt KIN mittels kubischer Interpolation in ihrer Größe und Auflösung auf das Teilbild TB aus der Figur 8a angepasst, sodass sich die vergrößerte Feature Map VFM aus der Figur 9a ergibt.

In einem Schwellenwertschritt SB wird dann eine binärwertigen Maske BM ermittelt, welche in der Figur 9b dargestellt ist. Hierbei wird vorzugsweise als Schwellenwert jener Wert angewendet, welcher die Hälfte der maximal möglichen Grauwertintensität einer Feature Map darstellt. Liegt also ein Grauwert einer Feature Map VFM zwischen den Werten 0 und 1, so wird als Schwellenwert der Wert 0,5 angewendet.

In einem Schritt MS wird dann der Maskierungsoperator BM aus der Figur 9b auf das Teilbild TB angewendet, sodass sich das entsprechende Subbild SUB aus der Figur 9c ergibt. Das vorgeschlagene zweite Convolutional Neural Network CNN2 ist also in der Lage, für jedes jeweilige Teilbild TB eine finale Feature Map FFM1 bereitzustellen, welche mittels ihrer Werte einen Subbildbereich für das Teilbild indiziert, der dem Kinetoplasten entspricht. Das zweite Convolutional Neural Network ist also in der Lage ein entsprechendes Subbild aus dem Teilbild herauszuschneiden bzw. zu selektieren, welches dann für die Bestimmung eines Bindungsmaßes einer Bindung von Autoantikörpern an doppelsträngige DNS an den Kinetoplasten herangezogen werden kann.

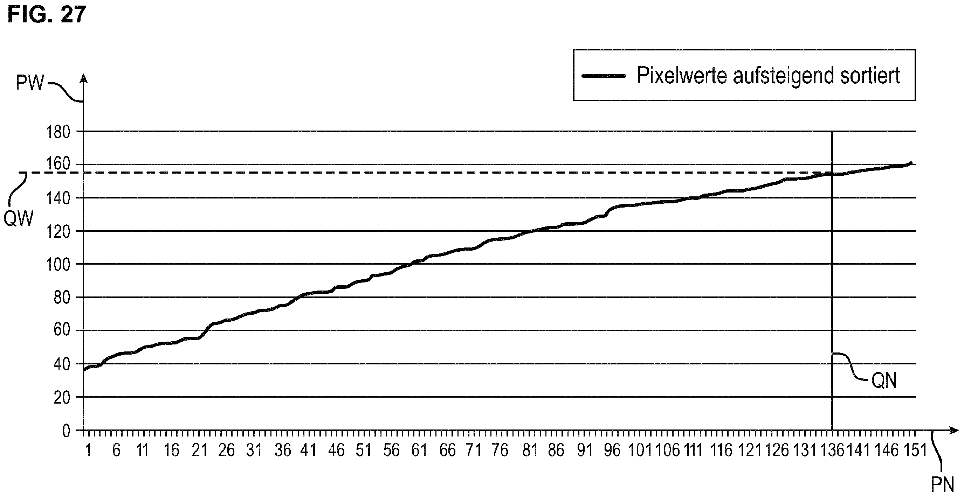

Gemäß der Figur 19 wird dann in einem Bestimmungsschritt BS das Bindungsmaß IBM1 für das Teilbild TB1 bestimmt. Dies erfolgt, indem die Pixelwerte aus dem Subbild SUB betrachtet werden und jener Wert gewählt wird, welcher das 90% Quantil für die Pixelwerte aus dem Subbild definiert. Figur 27 zeigt hierzu für die Pixel des Subbildes SUB aus der Figur 9c die entsprechenden Pixelwerte PW in aufsteigender Größe mit entsprechendem Sortierungsindex PN. Der Wert QW für den Index QN ist jener, für welchen 90% der Pixelwerte aus dem Subbild SUB kleiner als der Wert QW sind.

In einem danach folgenden Schritt wird dann auf Basis der mehreren individuellen Bindungsmaße IBM1, IBM2 ... der individuellen Teilbilder aus der ausgewählten Teilmenge das Gesamtbindungsmaß GBM bestimmt.

Die Figur 16a zeigt eine beispielhafte Ausführungsform des zweiten Convolutional Neural Network CNN2 mit mehreren Prozessierungsebenen P1, P2, P3, P4 für eine jeweils separate Prozessierung von Teilbildern TB1, ..., TBN mit Index N.

In einer ersten Prozessierungsebene P1 generiert das Convolutional Neural Network CNN2 auf Basis eines Teilbildes TB1 mittels wenigstens eines ersten Convolutional Layer LA1 und mittels Anwendung mehrerer zweidimensionaler Faltungskernel eine erste Menge von zweidimensionalen resultierenden Feature Maps RFM1. Diese Feature Maps RFM1 müssen nicht direkt aus dem Convolutional Layer LA1 hervorgehen, sondern können mittels weiterer Prozessierungsschritte PS2, PS3, PSC generiert werden.

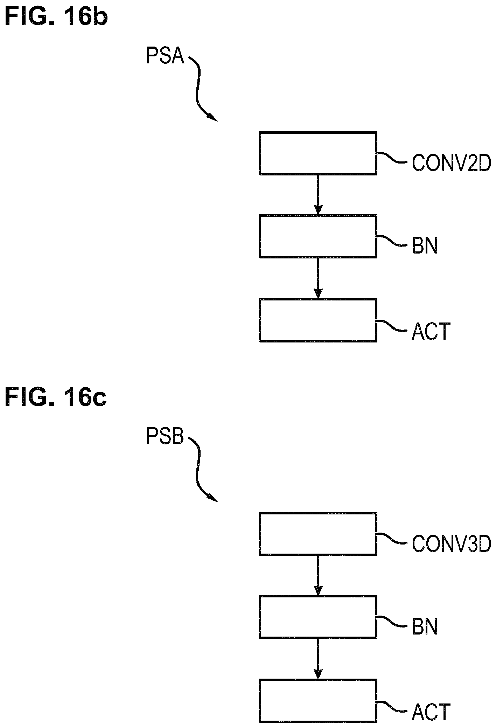

In dem Convolutional Layer LA1 erfolgt eine Prozessierung in einem Schritt PS1 mit einer Abfolge unterschiedlicher Teilschritte. Der Schritt PS1 ist vom Typ eines Schrittes PSA, welcher im Detail in der Figur 16b dargestellt ist. Zunächst erfolgt eine zweidimensionale Faltung CONV2D des eingehenden Teilbildes mit mehreren Faltungskernel mittels jeweiliger zweidimensionaler Faltungen. Eine anschließende Batch-Normalization erfolgt in einem Schritt BN. Es folgt dann eine sogenannte Activation in einem Schritt ACT.

Im Sinne dieser Anmeldung weist ein Convolutional Layer des zweiten Convolutional Neural Network CNN2 ein Layer zur Faltung einer oder mehrerer Feature Maps mit einem oder mehreren Faltungskernel auf. Solch ein Layer zur Faltung kann vorzugsweise dann innerhalb des Convolutional Layer von einem Layer einer Batch-Normalization und/ oder einem Layer einer Activation gefolgt sein.

In einer zweiten Prozessierungsebene P2 aus der Figur 16a erfolgt dann auf Basis der ersten Menge von Feature Maps RFM1 mittels wenigstens eines zweiten Convolutional Layer LA2 und mittels Anwendung mehrerer dreidimensionaler Faltungskernel eine Generierung einer zweiten Menge von zweidimensionalen resultierenden Feature Maps RFM2.

Auf Basis der zweiten Menge RFM2 erfolgt dann mittels wenigstens eines dritten Convolutional Layer LA3 und mittels Anwendung mehrerer dreidimensionaler Faltungskernel eine Generierung einer dritten Menge von zweidimensionalen resultierenden Feature Maps RFM3 dargestellt. Diese dritte Menge RFM3 geht direkt oder indirekt in die weitere Prozessierung der dritten Ebene P3 ein. In der dritten Ebene erfolgt eine Bestimmung der ersten finalen Feature Map FFM1 sowie vorzugsweise der zweiten finalen Feature Map FFM2 auf Basis der dritten Menge RFM3 mittels weiterer Convolutional Layer LAX.

Die zweite Menge RFM2 weist eine geringere Anzahl an Feature Maps auf als die erste Menge RFM1. Ferner weist die dritte Menge RFM3 eine größere Anzahl resultierender Feature Map auf als die zweite Menge RFM2. Ein Faltungskernel kann auch als ein Faltungskern oder als ein Faltungsoperator bezeichnet werden.

Durch die Reduktion der Anzahl der Feature Maps in dem zweiten Convolutional Layer LA2 erfolgt ein sogenanntes Squeezing. Die Feature Maps der ersten Menge RFM1 bzw. deren Merkmale werden mittels der Faltungskernel in einen Unterraum projiziert, da die dreidimensionalen Faltungskernel auf Merkmalskorrelation zwischen den Feature Maps reagieren. Es werden also nur die dominantesten Merkmale aus den Feature Maps der ersten Menge RFM1 beibehalten und in Feature Maps der zweiten Menge RFM2 hineinprojiziert. Weniger dominante und weniger aussagekräftige Merkmale werden also hierdurch herausgefiltert.

Durch die Erhöhung der Anzahl von Feature Maps durch das dritte Convolutional Layer LA3 von der zweiten Menge RFM2 hin zu der dritten Menge RFM3 werden die zuvor reduzierten Merkmale bzw. Informationen auf unterschiedliche Merkmalsräume und unterschiedliche Feature Maps verteilt, wobei die Merkmale unterschiedlich kombiniert werden können aufgrund der Freiheitsgrade der in dem Dritten Convolutional Layer LA3 verwendeten dreidimensionalen Faltungskernel. Dieses entspricht einem sogenannten Expandieren bzw. einem "Expand".

Auf das erste Convolutional Layer LA1 kann in der ersten Prozessierungsebene P1 ein weiteres Convolutional Layer LA11 folgen. Dieses Layer LA11 verwendet die in dem Layer LA1 erstellten Feature Maps. Vorzugsweise weist das Layer LA11 parallel zueinander angeordnete Prozessionsschritte PS2, PS3 auf. Diese Prozessionsschritte PS2, PS3 sind jeweils von der Art des Prozessionsschrittes PSB aus der Figur 16 C. In einem Teilschritt CONV3D des Schrittes PSB erfolgt eine dreidimensionale Faltung der Feature Maps mit jeweiligen dreidimensionalen Faltungskerneln. Es folgt dann in einem weiteren Teilschritt BN eine sogenannte Batch-Normalization. Es folgt ferner in einem Schritt ACT eine sogenannte Activation.

Die sich aus den Schritten PS2 sowie PS3 des Layer LA11 ergebenden Feature Maps werden dann in einem Konkatenierungs-Schritt PSC miteinander konkateniert; mit anderen Worten: die Feature Maps werden aneinandergereiht.

Vorzugsweise erfolgt in der ersten Prozessionsstufe P1 ferner eine Faltung des Teilbildes TB1 in einem Schritt PS4 mit zweidimensionalen Faltungskernel. Der Schritt PS4 ist von der Art des Teilschrittes CONV2D aus der Figur 16b.

Es können vorzugsweise die sich aus dem Layer LA11 und die aus dem Schritt PS4 ergebenden Feature Maps in der Weise miteinander verknüpft werden, dass die Einträge der Feature Maps jeweils elementweise miteinander addiert werden. Hierdurch ergibt sich also keine Veränderung der Dimensionalität der Feature Maps sondern die einzelnen Elemente Feature Maps aus dem Layer LA11 werden mit den einzelnen Elementen der Feature Maps aus dem Schritt PS4 elementweise addiert.

Der Schritt PS5 aus dem zweiten Convolutional Layer LA2 ist von der Art Schrittes PSB aus der Figur 16c.

Vorzugsweise werden die Feature Maps aus dem Convolutional Layer LA2 in dem dritten Convolutional Layer LA3 in der Weise prozessiert, dass in entsprechenden Schritten PS7 sowie PS8 als auch in dem Schritt PSC die Feature Maps in einer analogen Weise verarbeitet werden wie jene aus dem Convolutional Layer 11, wobei eine Anzahl verwendeter Faltungskernel und eine Dimensionalität der Faltungskernel voneinander abweichen können. Die Schritte PS7 sowie PS8 sind von der Art des Schrittes PSB aus der Figur 16c. Durch ein elementweises Addieren der jeweiligen Feature Maps aus den jeweiligen Schritten PS7 und PS8 wird dann durch einen Schritt PSC eine dritte Menge an Feature Maps RFM3 generiert.

In der zweiten Prozessierungsebene P2 folgen das zweite Convolutional Layer LA2 und das dritte Convolutional Layer LA3 als Teilschritte eines sequenziellen Prozessierungspfades PF1 aufeinander ab. In der zweiten Prozessierungsebene P2 ist ferner parallel zu dem sequenziellen Prozessierungspfad PF1 ein weiterer Prozessierungspfad PF2 vorhanden, in welchem das CNN2 auf Basis der ersten Menge RFM2 mittels wenigstens eines vierten Convolutional Layer LA4 und mittels Anwendung mehrerer dreidimensionaler Faltungskernel eine vierte Menge RFM4 von zweidimensionalen resultierenden Feature Maps generiert. Dies erfolgt durch einen Schritt PS6, welcher von der Art des Teilschrittes CONV3D aus der Figur 16c ist.

Eine durch den Schritt PSS in der Prozessierungsebene P2 bestimmte Menge an Feature Maps RFM5 wird dann wiederum mittels eines Schrittes PSS aus der dritten Menge RFM3 von Feature Maps und der vierten Menge RFM4 von Feature Maps generiert. Diese Menge von Feature Maps RFM5 kann dann in einer dritten Prozessierungsebene P3 verwendet werden, um mittels weiterer Schritte LAX, welche später noch detailliert erläutert werden, die erste finale Feature Map FFM1 und vorzugsweise die zweite finale Feature Map FFM2 zu generieren.

In einer weiteren Prozessierungsebene PS4 erfolgt dann das sogenannte Post-Processing, wie in der Figur 19 im Detail erläutert.

Das CNN2 generiert also die zu dem Teilbild TB korrespondierende finale Feature Map FFM1 auf Basis der dritten Menge RFM3 von Feature Maps und auf Basis der vierten Menge RFM4 von Feature Maps. Die Anzahl der aufeinanderfolgenden Convolutional Layer LA4 in dem parallelen Prozessierungspfad PF2 ist hierbei geringer als die Anzahl der aufeinander folgenden Convolutional Layer LA2, LA3 in dem sequenziellen Prozessierungspfad PF1. Der parallele Prozessierungspfad PF2 weist also weniger Convolutional Layer auf als der sequenzielle Pfad PF1. Hierduch wird es im Zuge eines Trainings des zweite Convolutional Neural Network ermöglicht, dass bei einer Neuberechnung einzelner Gewichte der Faltungskernel im Zuge einer Backpropagation das Problem des sogenannten "Vanishing Gradient" vermieden bzw. reduziert wird.

Wie zuvor in Bezug auf die Figur 16a dargelegt, kann das CNN2 aus vier Prozessierungsebenen bestehen.

Figur 23 zeigt hierzu eine detaillierte Ausführungsform der ersten Prozessierungsebene P1, welche zu der ersten Prozessierungsebene P1 aus der Figur 16a korrespondiert.

Für jeden einzelnen Schritt ist im Detail angegeben, von welcher Dimensionsalität eine Eingangsgröße in Form eines Teilbildes oder einer Menge von Feature Maps ist. Hierbei kann für jeden einzelnen Schritt in der oberen Zeile "Input" in darauffolgenden Klammern durch den zweiten und dritten Eintrag die Dimensionalität der Eingangsgröße bzw. Eingangsgrößen entnommen werden. Beispielsweise sind die Teilbilddaten TB1 von einer Dimensionalität von 150 × 150 Pixel. Es gibt für die Daten TB1 nur eine einzige Eingangsgröße, welches in dem vierten bzw. letzten Eintrag in den Klammern durch das Element "1" indiziert wird. Die Bilddaten TB1 sind in ihrem Wertebereich vorzugsweise auf einen Wertebereich von 0 bis 1 normiert.

In dem Schritt PS wird beispielsweise diese Eingangsgröße TB1 dann in der Weise mit Faltungskernel prozessiert, dass sich Feature Maps von einer Dimensionalität von 75 × 75 Pixeln ergeben. Der letzte Eintrag in der unteren Zeile "Output" indiziert hierbei die Anzahl der generierten Feature Maps der resultierenden Menge von Feature Maps. Hierdurch kann ein Fachmann also für jeden Prozessierungsschritt aus den hier angegebenen Parametern klar ableiten, wieviele Faltungskernel auf eingehende Daten TB1 oder eingehende Feature Maps angewendet werden müssen, um auf eine bestimmte Anzahl ausgehender Feature Maps zu kommen. In dem Beispiel des Schrittes PS4 sind dies 64 Faltungskernel. Ferner kann ein Fachmann aufgrund der angegebenen Dimensionalität der eingehenden Feature Maps und der angegebene Dimensionalität der ausgehenden Feature Maps ableiten, inwiefern ein sogenanntes "Striding", also ein Versatz während des Faltens einer Feature Map mit einem Faltungskernel, um eine bestimmte Anzahl von Pixel vorgenommen werden muss. In dem Beispiel des Schrittes PS4 ist dies ein Striding des Wertes 2.

Dem Fachmann werden als durch die Angaben aus der Figur 23 klare Anweisungen gegeben, um die Prozessierungsebene P1 des Convolutional Neural Network CNN2 auszugestalten.

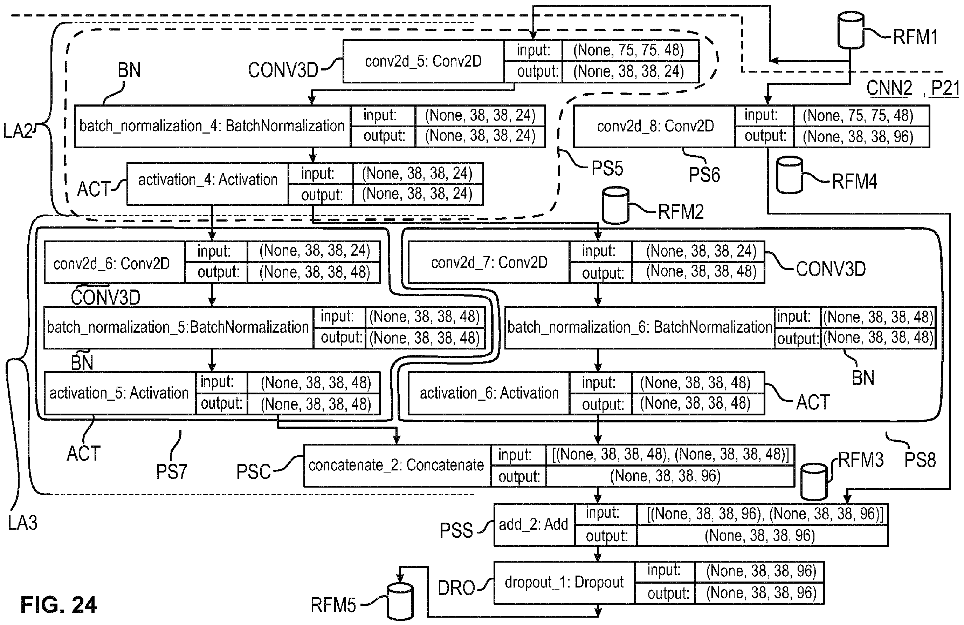

Die Figur 24 zeigt einen ersten Teil P21 der Prozessierungsebene P2 aus der Figur 16a. Hierbei entspricht die Struktur der Teil-Prozessierungsebene P21 im Wesentlichen der Prozessierungsebene P2 aus der Figur 16a. Zusätzlich ist hier noch ein so genannter Schritt eines "Dropout" für die Traingsphase vorgesehen, in welchem einzelne Einträge der Feature Maps während des Trainings, nicht aber während der tatsächlichen Klassifikation in der Testphase, in ihren Pixelwerten auf den Wert "0" (null) gesetzt werden. Der Drop-Faktor ist hierbei vorzugsweise ein Wert von 50 %, sodass die Hälfte der Pixelwerte wird auf null gesetzt werden. Die Pixelwerte werden zufällig gewählt, indem ihre Indizes mittels einer Zufallsfunktion ausgewählt werden.

Es ergibt sich dann in der Teil-Prozessierungsebene P21 die Menge RFM5 an Feature Maps, wie zuvor in der Figur 16a in der Prozessierungsebene P2 gezeigt.

Vorzugsweise kann das Convolutional Neural Network CNN2 eine weitere Teil-Prozessierungsebene P22 aufweisen, welche zuvor in der Figur 16a nicht dargestellt wurde. Hierbei wird die Menge an Feature Maps RFM5 weiterverarbeitet, um eine modifizierte Menge an Feature Maps RFM51 zu generieren.

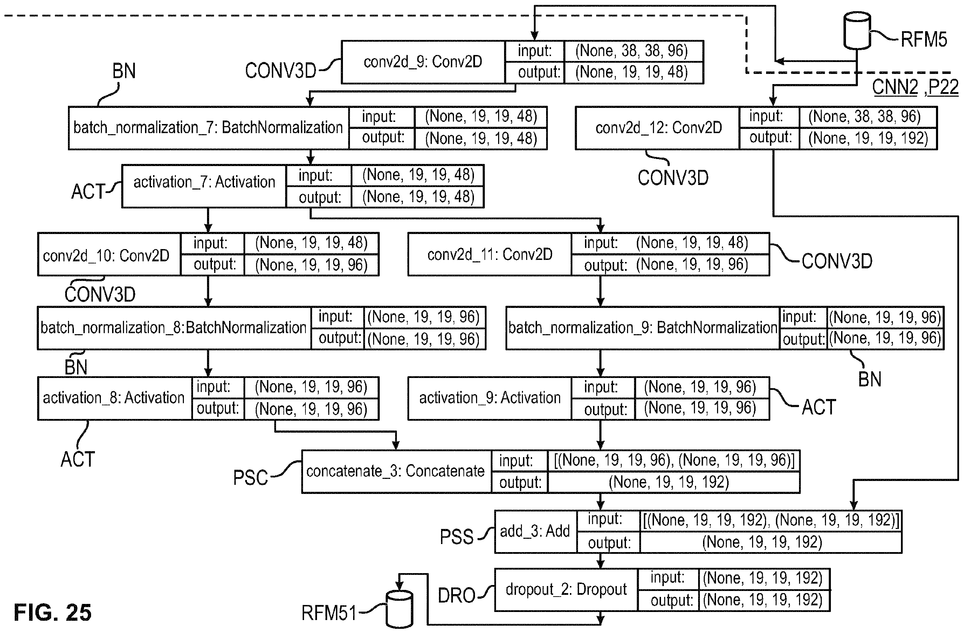

Auch hier sind in Figur 25 genaue Angaben für einen Fachmann enthalten, in welcher Weise diese modifizierte Menge an Features Maps RFM51 zu generieren ist. Auch hier erfolgt ein sogenanntes Dropout DRO während der Trainingsphase aber nicht der Testphase.

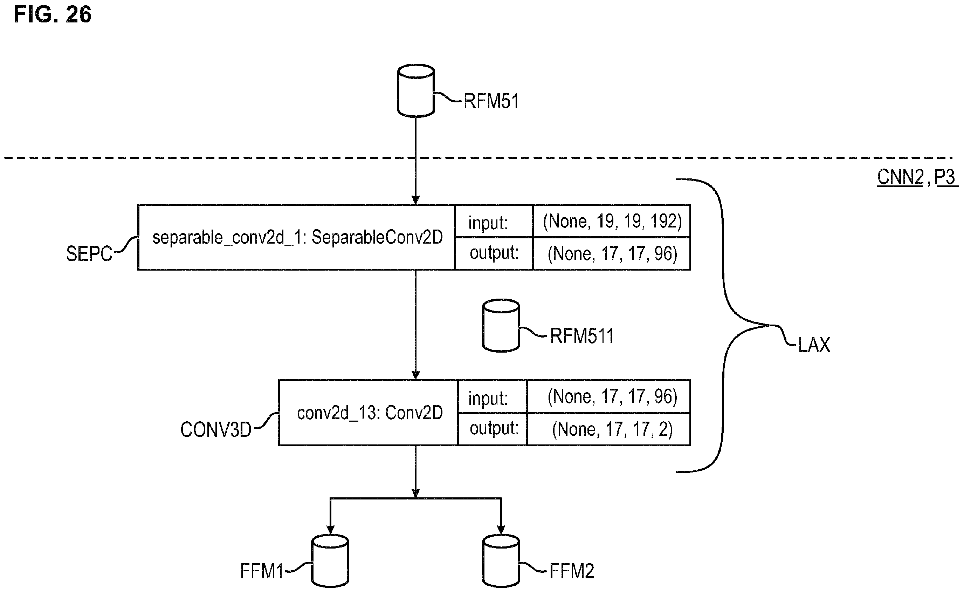

Die Figur 26 zeigt eine Ausführungsform der dritten Prozessierungsebene P3 zur Generierung der ersten finalen Feature Map FFM1 als auch vorzugsweise der zweiten finalen Feature Map FFM2. Die Feature Map FFM1 als auch vorzugsweise die Feature Map FFM2 wernde indirekt auf Basis der dritten Menge an Feature Maps RFM3 und der vierten Menge RFM4 an Feature Maps generiert. Hierbei weist der zuvor in Figur 16a dargestellte Prozessierungsschritt LAX Teilschritte auf. In einem ersten Teilschritt SEPC erfolgt eine sogenannte "depthwise convolution" der eingehenden Feature Maps RFM51, bei welcher jede einzelne zweidimensionale Feature Map aus der Menge RFM51 jeweils für sich mit einem zweidimensionalen Faltungskernel gefaltet wird, woraus sich eine Menge an zweidimensionalen Feature Maps RFM511 ergibt. Es erfolgt dann direkt im Anschluss in einem weiteren Teilschritt CONV3D eine Faltung der sich aus dem Schritt SEPC ergebenden mehreren zweidimensionalen Feature Maps der Menge RFM511 mit einem dreidimensionalen Faltungskernel der Dimensionalität 1x1xG , wobei G die Anzahl der Feature Maps der Menge ist, so dass hieraus die erste finale Feature Map FFM1 bestimmt wird. Vorzugsweise erfolgt in dem weiteren Teilschritt CONV3D ferner auch eine weitere Faltung der sich aus dem Schritt SEPC ergebenden mehreren zweidimensionalen Feature Maps der Menge RFM511 mit einem weiteren dreidimensionalen Faltungskernel der Dimensionalität 1x1xG , wobei G die Anzahl der Feature Maps der Menge ist, so dass hieraus die erste finale Feature Map FFM2 bestimmt wird.

An die dritte Prozessierungsebene P3 aus der Figur 26 schließt sich dann das sogenannte Post-Processing PP an, wie in der Figur 16a in der Prozessierungsebene P4 angedeutet und wie in der Figur 19 im Detail erläutert.

Für eine Implementation eines oder mehrerer Ausführungsbeispiele der hier vorgeschlagenen Convolutional Neural Networks CNN1, CNN2 kann ein Fachmann auf eine sogenannte Open Source Deep-Learning Bibliothek namens "Keras" zurückgegriffen werden. Detaillierte Informationen findet der Fachmann unter https://keras.io . Die Ausführungsform des vorgeschlagenen CNN2 mit den Prozessierungsebenen P1, P21, P22 und P3 aus den Figuren 23 bis 26 sowie der Prozessierungsebene P4, wie im Detail in Figur 19 illustriert, wurde für Versuchszwecke mittels der sogenannte Open Source Deep-Learning Bibliothek "Keras" erstellt.

Es wurden für ein Training der Convolutional Neural Networks CNN1, CNN2 verschiedene Datensätze an Fluoreszenzbildern verwendet.

Das Training des ersten Convolutional Neural Network CNN1 basierte auf 34.000 Gesamtbildern bzw. Fluoreszenzbilder mit entsprechenden Ground-Truth Daten der drei Bildsegmentklassen Zellleib, Zellrand und Hintergrund. Für das Training wurden 95% der 34.000 Fluoreszenzbilder Bilder verwendet, also 32.300 Fluoreszenzbilder. Während des Trainings wurden dem ersten Convolutional Neural Network diese 32.300 und mittels Cross-Entropy Loss dahingehend optimiert, die Ground-Truth Masken zu reproduzieren. Das Training wurde mit einer Start- Learning Rate von 1e-3 für 100 Epochen durchgeführt. Die Learning Rate war variabel und von der aktuellen Epoche abhängig. Für einen anschließenden Test des ersten Convolutional Neural Network CNN1 wurden dann die verbleibenden 5% der 34.000 Fluoreszenbilder Bilder verwendet , also 1.700 Fluoreszenzbilder. Es lagen auch entsprechende Ground-Truth Daten der drei Bildsegmentklassen Zellleib, Zellrand und Hintergrund vor. Die Klassifikation der Bildsegmente erfolgte auf Pixelebene bzw. Pixelgenauigkeit. Es wurde eine Genauigkeit der Klassifikation der Bildsegmente bzw. Pixel bezogen auf die 3 Klassen von 99.6% erreicht. Eine weitere evaluierte Metrik war die Mean Intersection Over Union (Miou), denn sie beachtet das starke Ungleichgewicht zwischen der Häufigkeit von Hintergrund- und Zell-Pixeln. Es wurde eine Miou von 80 % erreicht.

Der erste Datensatz an Fluoreszenzbildern war ein solcher, bei welchem es bekannt war, dass die zur Inkubation verwendeten Patientenproben Autoantikörper aufwiesen und somit in den Fluoreszenzbildern die Kinetoplastbereiche eine relevante Färbung aufwiesen. Der zweite Datensatz an Fluoreszenzbildern war ein solcher, bei welchem es bekannt war, dass die zur Inkubation verwendeten Patientenproben keine Autoantikörper aufwiesen und somit in den Fluoreszenzbildern die Kinetoplastbereiche keine relevante Färbung aufwiesen.

Das Training des zweiten Convolutional Neural Network CNN2 basierte auf 24.980 Teilbildern, welche den "positiven" Testfall (positiver Befund bzw. positiver Patient) repräsentierten, sowie 15.957 Teilbildern, welche den "negativen" Testfall (negativer Befund bzw. negativer Patient) repräsentierten. Für das Training selber wurden von den gesamten 24.980 positiven Teilbildern dann 17.427 positive Teilbilder verwendet sowie von den gesamten 15.957 negativen Teilbildern dann 11.233 negative Teilbilder, jeweils mit entsprechender Ground-Truth Information. Das zweite Convolutional Neural Network CNN2 wurde in 100 Epochen unter Verwendung einer Learning-Rate von 1e-3 trainiert. Für die Testung wurden von den gesamten 24.980 positiven Teilbildern dann 7543 positive Teilbilder verwendet sowie von den gesamten 15.957 negativen Teilbildern dann 4724 negative Teilbilder. Die durch das zweite Convolutional Neural Network CNN2 im Testfall gefällten Entscheidungen ergaben dann 6602 true positive Entscheidungen und 4623 true negative Entscheidungen. Ferner ergaben sich 101 false positive Entscheidungen und 941 false negative Entscheidungen. Dieses entspricht einer Sensitivität von 87,52% und einer Spezifität von 97, 86%.

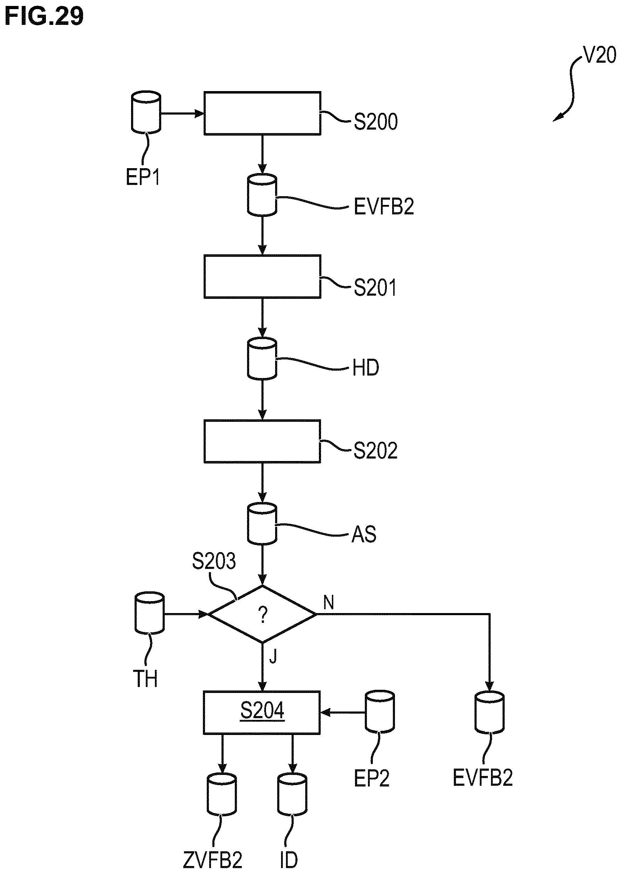

Die Figur 29 zeigt ein Verfahren V20, in welchem vorzugsweise durchzuführende Schritte zur Erfassung des Fluoreszenzbildes durchgeführt werden.

In einem Schritt S200 wird mittels wenigstens eines fest vorgegebenen Erfassungsparameters EP1, welches vorzugsweise ein Gain-Parameter ist, ein erstes vorläufiges Fluoreszenzbild EVFB2 erfasst.

In einem Schritt S201 wird dann ein Histogramm über die Pixelwerte des Bildes EVFB2 gebildet, sodass das Histogrammdaten HD ermittelt und bereitgestellt werden.

In einem Schritt S202 wird dann festgestellt, wie hoch die Anzahl von Pixeln ist, welche hinsichtlich einer Helligkeit eine bestimmte Sättigung überschreiten. Für einen beispielhaften Quantisierungsbereich der Pixelwerte von 0 bis 255 wird beispielsweise festgestellt, wie viele Pixel einen Pixelwert bzw. einen Grauwert von 255 aufweisen. Diese Anzahl der Pixel, welche in der Helligkeits-Sättigung sind, werden als Daten AS bereitgestellt.

Es wird dann in einem Schritt S203 überprüft, ob die Anzahl der Pixel AS, welche in einem Sättigungsbereich sind, einen vorgegebenen Schwellenwert TH überschreitet. Liegt keine Schwellenwertüberschreitung vor (siehe Verzweigung "N"), so wird das erste vorläufige Fluoreszenzbild EVFB2 als das Fluoreszenzbild SG verwendet, siehe Figur 4. Überschreitet die Anzahl der Pixel, welche in der Sättigung sind, den Schwellenwert TH (siehe Verzweigung "J"), so wird in einem Schritt S204 ein zweites vorläufiges Fluoreszenzbild ZVFB2 unter Verwendung wenigstens eines zweiten fest vorgegebenen Erfassungsparameters EP2 erfasst. Der fest vorgegebene Erfassungsparameter EP2 unterscheidet sich von dem zuvor fest vorgegebenen ersten Erfassungsparameter EP1. Vorzugsweise ist der Erfassungsparameter EP2 ein Gain-Parameter und ist geringer als der erste Erfassungsparameter EP1, sodass das Bild ZVFB2 weniger stark belichtet ist als das Bild EVFB2. Es wird dann das erfasste zweite vorläufige Fluoreszenzbild EVFB2 als das Fluoreszenzbild SG verwendet, siehe Figur 4. Vorzugsweise werden ferner Indikatordaten bereitgestellt, welche indizieren, dass die Helligkeit des ersten vorläufigen Fluoreszenzbildes EVFB2 eine maximale Helligkeit überstiegen hat und es sich bei dem Fluoreszenzbild SG um ein mit reduzierter Helligkeit erfasstes zweites vorläufiges Fluoreszenzbild ZVFB2 handelt.

Es kann dann das zweite vorläufige Fluoreszenzbild EVFB2 in üblicher Weise als das Fluoreszenzbild SG in dem vorgeschlagenen Verfahren verwendet werden. Hierbei ermittelt vorzugsweise das Convolutional Neural Network CNN2 mittels eines Konfidenzmaßes PK, ob für eine Mindestanzahl an Teilbildbereichen, vorzugsweise wenigstens zehn Teilbildbereiche, eine Färbung von Kinetoplastbereichen vorhanden ist. Ist dies der Fall, so gibt das Convolutional Neural Network CNN2 als Gesamtbindungsmaß die maximale Helligkeit bzw. den maximalen Helligkeitswert, vorzugsweise 255, aus. Stellt das Convolutional Neural Network CNN2 fest, dass die Kinetoplastbereiche nicht wirklich gefärbt sind, so wird das zweite Fluoreszenzbild SG als insgesamt negativ bewertet.

Obwohl manche Aspekte im Zusammenhang mit einer Vorrichtung beschrieben wurden, versteht es sich, dass diese Aspekte auch eine Beschreibung der entsprechenden Verfahren darstellen, sodass ein Block oder ein Bauelement einer Vorrichtung auch als ein entsprechender Verfahrensschritt oder als ein Merkmal eines Verfahrensschrittes zu verstehen ist. Analog dazu stellen Aspekte, die im Zusammenhang mit einem oder als ein Verfahrensschritt beschrieben wurden, auch eine Beschreibung eines entsprechenden Blocks oder Details oder Merkmals einer entsprechenden Vorrichtung dar.

Je nach bestimmten Implementierungsanforderungen können Ausführungsbeispiele der Erfindung die Recheneinheit R oder die Datennetzwerkvorrichtung in Hardware und/oder in Software umsetzen. Eine Umsetzung einer hier genannten Recheneinheit R kann hier als wenigstens eine Recheneinheit erfolgen oder aber durch mehrere Recheneinheiten im Verbund. Die Implementierung kann unter Verwendung eines digitalen Speichermediums, beispielsweise einer Floppy-Disk, einer DVD, einer Blu-Ray Disc, einer CD, eines ROM, eines PROM, eines EPROM, eines EEPROM oder eines FLASH-Speichers, einer Festplatte oder eines anderen magnetischen oder optischen Speichers durchgeführt werden, auf dem elektronisch lesbare Steuersignale gespeichert sind, die mit einer programmierbaren Hardwarekomponente derart zusammenwirken können oder zusammenwirken, dass das jeweilige Verfahren durchgeführt wird.

Eine programmierbare Hardwarekomponente kann als Recheneinheit durch einen Prozessor, einen Computerprozessor (CPU = Central Processing Unit), einen Computer, ein Computersystem, einen anwendungsspezifischen integrierten Schaltkreis (ASIC = Application-Specific Integrated Circuit), einen integrierten Schaltkreis (IC = Integrated Circuit), ein Ein-Chip-System (SOC = System on Chip), ein programmierbares Logikelement oder ein feldprogrammierbares Gatterarray mit einem Mikroprozessor (FPGA = Field Programmable Gate Array) gebildet sein.

Das digitale Speichermedium kann daher maschinen- oder computerlesbar sein. Manche Ausführungsbeispiele umfassen also einen Datenträger, der elektronisch lesbare Steuersignale aufweist, die in der Lage sind, mit einem programmierbaren Computersystem oder einer programmierbare Hardwarekomponente derart zusammenzuwirken, dass eines der hierin beschriebenen Verfahren durchgeführt wird.

Allgemein können Ausführungsbeispiele oder Teile der Ausführungsbeispiele der vorliegenden Erfindung als Programm, Firmware, Computerprogramm oder Computerprogrammprodukt mit einem Programmcode oder als Daten implementiert sein, wobei der Programmcode oder die Daten dahin gehend wirksam ist bzw. sind, eines der Verfahren oder ein Teil eines Verfahrens durchzuführen, wenn das Programm auf einem Prozessor oder einer programmierbaren Hardwarekomponente abläuft.

**Patentansprüche**

1. Verfahren zum Detektieren einer Bindung von Autoantikörpern einer Patientenprobe an doppelsträngige Desoxyribonukleinsäure unter Verwendung von Crithidia luciliae Zellen mittels Fluoreszenzmikroskopie und mittels digitaler Bildverarbeitung, aufweisend

   - Bereitstellen eines Substrates (S), welches mehrere Crithidia luciliae Zellen (CR) aufweist,
   - Inkubieren des Substrates (S) mit der Patientenprobe, welche potentiell die Autoantikörper aufweist,
   - Inkubieren des Substrates (S) mit sekundären Antikörpern, welche jeweils mit einem Fluoreszenzfarbstoff markiert sind, welcher vorzugsweise ein grüner Fluoreszenzfarbstoff ist,
   - Erfassen eines Fluoreszenzbildes (SG) des Substrates (S) in einem Farbkanal, welcher zu dem Fluoreszenzfarbstoff korrespondiert, wobei der Farbkanal vorzugsweise ein Grünkanal ist,

   **gekennzeichnet durch**,

   - Identifizieren jeweiliger Teilbilder (TB) in dem einen Fluoreszenzbild (SG), welche jeweils wenigstens eine Crithidia luciliae Zelle (CR) repräsentieren, mittels eines ersten, vortrainierten Convolutional Neural Network (CNN1)
   - jeweiliges Prozessieren wenigstens einer Teilmenge der jeweiligen Teilbilder (TB) mittels eines zweiten, vortrainierten Convolutional Neural Network (CNN2), wobei das zweite Convolutional Neural Network (CNN2) für ein jeweiliges Teilbild ein jeweiliges Subbild bestimmt, welches einen Kinetoplastbereich repräsentiert, zur Bestimmung jeweiliger Bindungsmaße (IBM1, IBM2) auf Basis jeweiliger Subbilder, wobei ein jeweiliges Bindungsmaß einen jeweiligen Grad einer Bindung von Autoantikörpern in einem jeweiligen Kinetoplastbereich (K) einer jeweiligen Crithidia luciliae Zelle (CR) eines jeweiligen Teilbildes (TB) indizieren,
   - Bestimmen eines Gesamtbindungsmaßes (GBM) bezogen auf die Bindung von Autoantikörpern der Patientenprobe an doppelsträngige Desoxyribonukleinsäure auf Basis der jeweiligen Bindungsmaße (IBM1, IBM2).

2. Verfahren nach Anspruch 1,
   wobei das Identifizieren der jeweiligen Teilbilder (TB) in dem einen Fluoreszenzbild (SG) dadurch erfolgt, dass mittels des ersten, vortrainierten Convolutional Neural Network (CNN1) jeweilige Bildsegmente des Fluoreszenzbildes jeweiligen Bildsegmentklassen aus einer Gruppe von Bildsegmentklassen zugeordnet werden, wobei die Gruppe der Bildsegmentklassen wenigstens die folgenden Bildsegmentklassen umfasst

   - Zelle und
   - Hintergrund.

3. Verfahren nach Anspruch 1,
   wobei das Identifizieren der jeweiligen Teilbilder (TB) in dem einen Fluoreszenzbild (SG) dadurch erfolgt, dass mittels des ersten, vortrainierten Convolutional Neural Network (CNN1) jeweilige Bildsegmente des Fluoreszenzbildes jeweiligen Bildsegmentklassen aus einer Gruppe von Bildsegmentklassen zugeordnet werden, wobei die Gruppe der Bildsegmentklassen wenigstens die folgenden Bildsegmentklassen umfasst

   - Zellleib,
   - Zellrand und
   - Hintergrund.

4. Verfahren nach Anspruch 1,

   aufweisend, für ein jeweiliges Teilbild (TB),

   - Selektieren eines jeweiligen Subbildes (SUB) des jeweiligen Teilbildes (TB), wobei das jeweilige Subbild (SUB) einen jeweiligen Kinetoplastbereich (K) einer jeweiligen Crithidia luciliae Zelle (CR) repräsentiert,
- Bestimmen des jeweiligen Bindungsmaßes (IBM1) auf Basis des jeweiligen Subbildes (SUB),

sowie ferner aufweisend Bestimmen des Gesamtbindungsmaßes (GBM) auf Basis der jeweiligen Bindungsmaße (IBM1, IBM2).

5. Verfahren nach Anspruch 1,
aufweisend

- Bestimmen einer jeweiligen finalen Feature Map (FFM1) für ein jeweiliges Teilbild (TB) mittels des zweiten Convolutional Neural Network (CNN2),
- Bestimmen eines jeweiligen Konfidenzmaßes (PKN) bezogen auf ein Vorliegen einer Bindung von Autoantikörpern in einem jeweiligen Kinetoplastbereich (K) für das jeweilige Teilbild (TB),
- Auswählen einer Teilmenge der Teilbilder (TB) auf Basis der bestimmten Konfidenzmaße (PKN),
- jeweiliges Prozessieren der jeweiligen Feature Maps der jeweiligen ausgewählten Teilbilder zur Bestimmung der jeweiligen Bindungsmaße (IBM1, IBM2),
- Bestimmen des Gesamtbindungsmaßes (GBM) auf Basis der jeweiligen Bindungsmaße (IBM1, IBM2) der jeweiligen ausgewählten Teilbilder.

6. Verfahren nach Anspruch 5,

aufweisend, für ein jeweiliges Teilbild (TB) aus der ausgewählten Teilmenge,

- Selektieren eines jeweiligen Subbildes (SUB) des jeweiligen Teilbildes (TB) auf Basis einer zu dem jeweiligen Teilbild (TB) korrespondierenden jeweiligen finalen Feature Map (FFM1), wobei das jeweilige Subbild (SUB) einen jeweiligen Kinetoplastbereich (K) einer jeweiligen Crithidia luciliae Zelle (CR) repräsentiert,
- Bestimmen des jeweiligen Bindungsmaßes (IBM1) auf Basis des jeweiligen Subbildes (SUB),

sowie ferner aufweisend

- Bestimmen des Gesamtbindungsmaßes (GBM) auf Basis der jeweiligen Bindungsmaße (IBM1, IBM2).

7. Verfahren nach Anspruch 6,

aufweisend für ein jeweiliges Teilbild (TB) aus der ausgewählten Teilmenge,

- Ermitteln eines jeweiligen Maskierungsoperators (BM) auf Basis der jeweiligen finalen Feature Map (FFM1),
- Selektieren des jeweiligen Subbildes (SUB) des jeweiligen Teilbildes (TB) mittels Anwendung des jeweiligen Maskierungsoperators (BM) auf das jeweilige Teilbild (TB),
- Bestimmen des jeweiligen Bindungsmaßes (IBM1) auf Basis des jeweiligen Subbildes (SUB),

sowie aufweisend

- Bestimmen des Gesamtbindungsmaßes (GBM) auf Basis der jeweiligen Bindungsmaße (IBM1, IBM2).

8. Verfahren nach Anspruch 5,
wobei das zweite Convolutional Neural Network (CNN2) im Zuge einer Prozessierung eines Teilbildes (TB)

- in einer ersten Prozessierungsebene (P1) auf Basis des Teilbildes (TB) mittels wenigstens eines ersten Convolutional Layer (LA1) und mittels Anwendung mehrerer zweidimensionaler Faltungskernel eine erste Menge von resultierenden Feature Maps (RFM1) generiert,
- in einer zweiten Prozessierungsebene (P2)

  ∘ auf Basis der ersten Menge zweidimensionaler Feature Maps (RFM1) mittels wenigstens eines zweiten Convolutional Layer (LA2) und mittels Anwendung mehrerer dreidimensionaler Faltungskernel eine zweite Menge von resultierenden Feature Maps (RFM2) generiert,
  ∘ sowie ferner auf Basis der zweiten Menge zweidimensionaler Feature Maps (RFM2) mittels wenigstens eines dritten Convolutional Layer (LA3) und mittels Anwendung mehrerer dreidimensionaler Faltungskernel eine dritte Menge von resultierenden Feature Maps (RFM3) generiert,

wobei die zweite Menge (RFM2) eine geringere Anzahl resultierender Feature Maps aufweist als die erste Menge (RFM1) und wobei die dritte Menge (RFM3) eine größere Anzahl resultierender Feature Maps aufweist als die zweite Menge (RFM2).

9. Verfahren nach Anspruch 8,

wobei in der zweiten Prozessierungsebene (P2) das zweite Convolutional Layer (LA2) und das dritte Convolutional Layer (LA3) als Teilschritte eines sequentiellen Prozessierungspfades (PF1) aufeinander abfolgen,

wobei in der zweiten Prozessierungsebene (P2) ein weiterer Prozessierungspfad (PF2) parallel zu dem sequentiellen Prozessierungspfad (PF1) vorhanden ist, in welchem das zweite Convolutional Neural Network (CNN2) auf Basis der ersten Menge (RFM1) zweidimensionaler Feature Maps mittels wenigstens eines vierten Convolutional Layer (LA4) eine vierte Menge (RFM4) von resultierenden Feature Maps generiert,

wobei das zweite Convolutional Neural Network (CNN2) die zu dem Teilbild (TB) korrespondierende finale Feature Map (FFM1) auf Basis der dritten (RFM3) und der vierten Menge (RFM4) von resultierenden Feature Maps generiert,

und wobei die Anzahl aufeinanderfolgender Convolution Layer in dem parallelen Prozessierungspfad (PF2) geringer ist als die Anzahl aufeinanderfolgender Convolution Layer in dem sequentiellen Prozessierungspfad (PF1).

10. Verfahren nach Anspruch 1, aufweisend

   - Erfassen eines ersten vorläufigen Fluoreszenzbildes (EVFB2) in dem Farbkanal unter Verwendung eines fest vorgegebenen Erfassungsparameters (EP1),
   - Feststellen, ob eine Helligkeit des vorläufigen Fluoreszenzbildes (EVFB2) des Farbkanals eine maximale Helligkeit übersteigt,
   - in dem Fall, dass das erste vorläufige Fluoreszenzbild des Farbkanals (EVB2) die maximale Helligkeit nicht übersteigt, Verwenden des ersten vorläufigen Fluoreszenzbildes (EVFB2) als das eine Fluoreszenzbild (SG),
   - in dem Fall, dass das das erste vorläufige Fluoreszenzbild des Farbkanals (EVB2) die maximale Helligkeit übersteigt, Erfassen eines zweiten vorläufigen Fluoreszenzbildes in dem Farbkanal (ZVFB2) und Verwenden des zweiten vorläufigen Fluoreszenzbildes des Farbkanals (ZVFB2) als das eine Fluoreszenzbild (SG).

11. Vorrichtung (V1) zum Detektieren einer Bindung von Autoantikörpern einer Patientenprobe an doppelsträngige Desoxyribonukleinsäure unter Verwendung von Crithidia luciliae Zellen mittels Fluoreszenzmikroskopie und mittels digitaler Bildverarbeitung, aufweisend

   - eine Haltevorrichtung (H) für ein Substrat (S), welches mehrere Crithidia luciliae Zellen (CR) aufweist und welches mit einer Patientenprobe,

aufweisend die Autoantikörper, sowie ferner mit sekundären Antikörpern, welche jeweils mit einem Fluoreszenzfarbstoff markiert sind, welcher vorzugsweise ein grüner Fluoreszenzfarbstoff ist, inkubiert wurde,

   - wenigstens eine Bilderfassungseinheit (K1, K2) zum Erfassen eines Fluoreszenzbildes (SG) des Substrates (S) in einem Farbkanal, welcher vorzugsweise ein grüner Fluoreszenzfarbstoff ist,

sowie ferner aufweisend wenigstens eine Recheneinheit (R), welche ausgebildet ist,

   - in dem einen Fluoreszenzbild (SG) jeweilige Teilbilder (TB), welche jeweils wenigstens eine Crithidia luciliae Zelle (CR) repräsentieren, mittels eines ersten, vortrainierten Convolutional Neural Network (CNN1) zu identifizieren,
   - wenigstens eine Teilmenge der jeweiligen Teilbilder (TB) jeweils zu prozessieren mittels eines zweiten, vortrainierten Convolutional Neural Network (CNN2), wobei das zweite Convolutional Neural Network (CNN2) für ein jeweiliges Teilbild ein jeweiliges Subbild bestimmt, welches einen Kinetoplastbereich repräsentiert, zur Bestimmung jeweiliger Bindungsmaße (IBM1, IBM2) auf Basis jeweiliger Subbilder, wobei ein jeweiliges Bindungsmaß einen jeweiligen Grad einer Bindung von Autoantikörpern in einem jeweiligen Kinetoplastbereich (K) einer jeweiligen Crithidia luciliae Zelle (CR) eines jeweiligen Teilbildes (TB) indizieren
   - und ein Gesamtbindungsmaß (GBM) einer Bindung von Autoantikörpern der Patientenprobe an doppelsträngige Desoxyribonukleinsäure auf Basis der jeweiligen Bindungsmaße (IBM1, IBM2) zu bestimmen.

12. Recheneinheit (R), welche ausgebildet ist im Zuge einer digitalen Bildverarbeitung

   - ein Fluoreszenzbild (SG) entgegenzunehmen, welches eine Färbung eines Substrates, welches wiederum mehrere Crithidia luciliae Zellen (CR) aufweist, durch einen Fluoreszenzfarbstoff repräsentiert, welcher vorzugsweise ein grüner Fluoreszenzfarbstoff ist,
   - jeweilige Teilbilder (TB) in dem einen Fluoreszenzbild (SG) zu identifizieren, welche jeweils wenigstens eine Crithidia luciliae Zelle (CR) repräsentieren, mittels eines ersten, vortrainierten Convolutional Neural Network (CNN1),
   - wenigstens eine Teilmenge der jeweiligen Teilbilder (TB) jeweils zu prozessieren mittels eines zweiten, vortrainierten Convolutional Neural Network (CNN2), wobei das zweite Convolutional Neural Network (CNN2) für ein jeweiliges Teilbild ein jeweiliges Subbild bestimmt, welches einen Kinetoplastbereich repräsentiert, zur Bestimmung jeweiliger Bindungsmaße (IBM1, IBM2) auf Basis jeweiliger Subbilder, wobei ein jeweiliges Bindungsmaß einen jeweiligen Grad einer Bindung von Autoantikörpern in einem jeweiligen Kinetoplastbereich (K) einer jeweiligen Crithidia luciliae Zelle (CR) eines jeweiligen Teilbildes (TB) indizieren

- sowie ein Gesamtbindungsmaß (GBM) einer Bindung von Autoantikörpern der Patientenprobe an doppelsträngige Desoxyribonukleinsäure auf Basis der jeweiligen Bindungsmaße (IBM1, IBM2) zu bestimmen.

13. Datennetzwerkvorrichtung (DV), aufweisend wenigstens eine Datenschnittstelle (DS4) zum Entgegennehmen eines Fluoreszenzbildes (BI1, SG), welches eine Färbung eines Substrates, welches wiederum mehrere Crithidia luciliae Zellen aufweist, durch einen Fluoreszenzfarbstoff repräsentiert, welcher vorzugsweise ein grüner Fluoreszenzfarbstoff ist, sowie ferner wenigstens eine Recheneinheit (R), welche ausgebildet ist im Zuge einer digitalen Bildverarbeitung

- jeweilige Teilbilder (TB) in dem einen Fluoreszenzbild (SG), welche jeweils wenigstens eine Crithidia luciliae Zelle (CR) repräsentieren, mittels eines ersten, vortrainierten Convolutional Neural Network (CNN1) zu identifizieren,
- wenigstens eine Teilmenge der jeweiligen Teilbilder (TB) jeweils zu prozessieren mittels eines zweiten, vortrainierten Convolutional Neural Network (CNN2), wobei das zweite Convolutional Neural Network (CNN2) für ein jeweiliges Teilbild ein jeweiliges Subbild bestimmt, welches einen Kinetoplastbereich repräsentiert, zur Bestimmung jeweiliger Bindungsmaße (IBM1, IBM2) auf Basis jeweiliger Subbilder, wobei ein jeweiliges Bindungsmaß einen jeweiligen Grad einer Bindung von Autoantikörpern in einem jeweiligen Kinetoplastbereich (K) einer jeweiligen Crithidia luciliae Zelle (CR) eines jeweiligen Teilbildes (TB) indizieren
- sowie ein Gesamtbindungsmaß (GBM) einer Bindung von Autoantikörpern der Patientenprobe an doppelsträngige Desoxyribonukleinsäure auf Basis der jeweiligen Bindungsmaße (IBM1, IBM2) zu bestimmen.

14. Verfahren zur digitalen Bildverarbeitung, aufweisend

- Entgegennehmen eines Fluoreszenzbildes (SG), welches eine Färbung eines Substrates (S), welches wiederum mehrere Crithidia luciliae Zellen (CR) aufweist, durch einen Fluoreszenzfarbstoff repräsentiert, welcher vorzugsweise ein grüner Fluoreszenzfarbstoff ist
- Identifizieren jeweiliger Teilbilder (TB) in dem Fluoreszenzbild (SG), welche jeweils wenigstens eine Crithidia luciliae Zelle (CR) repräsentiert, mittels eines ersten, vortrainierten Convolutional Neural Network (CNN1),
- jeweiliges Prozessieren wenigstens einer Teilmenge der jeweiligen Teilbilder (TB) mittels eines zweiten, vortrainierten Convolutional Neural Network (CNN2) wobei das zweite Convolutional Neural Network (CNN2) für ein jeweiliges Teilbild ein jeweiliges Subbild bestimmt, welches einen Kinetoplastbereich repräsentiert, zur Bestimmung jeweiliger Bindungsmaße (IBM1, IBM2), auf Basis jeweiliger Subbilder, wobei ein jeweiliges Bindungsmaß einen jeweiligen Grad einer Bindung von Autoantikörpern in einem jeweiligen Kinetoplastbereich (K) einer jeweiligen Crithidia luciliae Zelle (CR) eines jeweiligen Teilbildes (TB) indizieren,
- Bestimmen eines Gesamtbindungsmaß (GBM) einer Bindung von Autoantikörpern der Patientenprobe an doppelsträngige Desoxyribonukleinsäure auf Basis der jeweiligen Bindungsmaße (IBM1, IBM2).

15. Computerprogrammprodukt (CPP), umfassend Befehle, die bei der Ausführung des Programms durch einen Computer diesen veranlassen, das Verfahren zur digitalen Bildverarbeitung nach Anspruch 14 durchzuführen.

16. Datenträgersignal (SI2), welches das Computerprogrammprodukt (CPP) nach Anspruch 15 überträgt.

**Claims**

1. Method for detecting a binding of autoantibodies from a patient sample to double-stranded deoxyribonucleic acid using Crithidia luciliae cells by means of fluorescence microscopy and by means of digital image processing,

comprising

- provision of a substrate (S) which has multiple Crithidia luciliae cells (CR),
- incubation of the substrate (S) with the patient sample which potentially has the autoantibodies,
- incubation of the substrate (S) with secondary antibodies which have each been labelled with a fluorescent dye which is preferably a green fluorescent dye,
- acquisition of a fluorescence image (SG) of the substrate (S) in a colour channel which corresponds to the fluorescent dye, wherein the colour channel is preferably a green channel,

**characterized by**

- identification by means of a first pretrained convolutional neural network (CNN1) of respective sub-images (TB) in the one fluorescence image (SG) that each represent at least one Crithidia luciliae cell (CR),
- respective processing of at least one subset of the respective sub-images (TB) by means of a second pretrained convolutional neural network (CNN2) whereby the second convolutional neural network (CNN2) determines a respective subordinate image representing the kinetoplast region for a respective sub-image for determining respective binding measures (IBM1, IBM2) on the basis of respective subordinate images, whereby a respective binding measure indicates a respective extent of a binding of autoantibodies in a respective kinetoplast region (K) of a respective Crithidia luciliae cell (CR) of a respective sub-image (TB),
- determination of an overall binding measure (GBM) with regard to the binding of autoantibodies from the patient sample to double-stranded deoxyribonucleic acid on the basis of the respective binding measures (IBM1, IBM2).

2. Method according to Claim 1, wherein the identification of the respective sub-images (TB) in the one fluorescence image (SG) is effected by assigning respective image segments of the fluorescence image to respective image segment classes from a group of image segment classes by means of the first pretrained convolutional neural network (CNN1), wherein the group of the image segment classes comprises at least the following image segment classes

- cell and
- background.

3. Method according to Claim 1, wherein the identification of the respective sub-images (TB) in the one fluorescence image (SG) is effected by assigning respective image segments of the fluorescence image to respective image segment classes from a group of image segment classes by means of the first pretrained convolutional neural network (CNN1), wherein the group of the image segment classes comprises at least the following image segment classes

- cell body,
- cell edge and
- background.

4. Method according to Claim 1,

comprising, for a respective sub-image (TB),

- selection of a respective subordinate image (SUB) of the respective sub-image (TB), wherein the respective subordinate image (SUB) represents a respective kinetoplast region (K) of a respective Crithidia luciliae cell (CR),
- determination of the respective binding measure (IBM1) on the basis of the respective subordinate image (SUB),

and comprising furthermore determination of the overall binding measure (GBM) on the basis of the respective binding measures (IBM1, IBM2).

5. Method according to Claim 1, comprising

- determination of a respective final feature map (FFM1) for a respective sub-image (TB) by means of the second convolutional neural network (CNN2),
- determination of a respective confidence measure (PKN) with regard to a presence of a binding of autoantibodies in a respective kinetoplast region (K) for the respective sub-image (TB),
- selection of a subset of the sub-images (TB) on the basis of the determined confidence measures (PKN),
- respective processing of the respective feature maps of the respective selected sub-images for determining the respective binding measures (IBM1, IBM2),
- determination of the overall binding measure (GBM) on the basis of the respective binding measures (IBM1, IBM2) of the respective selected sub-images.

6. Method according to Claim 5,

comprising, for a respective sub-image (TB) from the selected subset,

- selection of a respective subordinate image (SUB) of the respective sub-image (TB) on the basis of a respective final feature map (FFM1) corresponding to the respective sub-image (TB), wherein the respective subordinate image (SUB) represents a respective kinetoplast region (K) of a respective Crithidia luciliae cell (CR),
- determination of the respective binding measure (IBM1) on the basis of the respective subordinate image (SUB),

and comprising furthermore

- determination of the overall binding measure (GBM) on the basis of the respective binding measures (IBM1, IBM2).

7. Method according to Claim 6,

comprising, for a respective sub-image (TB) from the selected subset,

- ascertainment of a respective masking operator (BM) on the basis of the respective final feature map (FFM1),
- selection of the respective subordinate image (SUB) of the respective sub-image (TB) by means of application of the respective masking operator (BM) to the respective sub-image (TB),
- determination of the respective binding measure (IBM1) on the basis of the respective subordinate image (SUB),

and comprising

- determination of the overall binding measure (GBM) on the basis of the respective binding measures (IBM1, IBM2).

8. Method according to Claim 5,
wherein, in the course of a processing of a sub-image (TB), the second convolutional neural network (CNN2),

- in a first processing level (P1), generates a first set of resultant feature maps (RFM1) on the basis of the sub-image (TB) by means of at least one first convolutional layer (LA1) and by means of application of multiple two-dimensional convolution kernels,
- in a second processing level (P2),

  ◦ generates a second set of resultant feature maps (RFM2) on the basis of the first set of two-dimensional feature maps (RFM1) by means of at least one second convolutional layer (LA2) and by means of application of multiple three-dimensional convolution kernels,
  ◦ and furthermore generates a third set of resultant feature maps (RFM3) on the basis of the second set of two-dimensional feature maps (RFM2) by means of at least one third convolutional layer (LA3) and by means of application of multiple three-dimensional convolution kernels,

wherein the second set (RFM2) has a smaller number of resultant feature maps than the first set (RFM1) and wherein the third set (RFM3) has a larger number of resultant feature maps than the second set (RFM2).

9. Method according to Claim 8,

wherein, in the second processing level (P2), the second convolutional layer (LA2) and the third convolutional layer (LA3) are in a sequence as sub-steps of a sequential processing path (PF1),
wherein, in the second processing level (P2), there is in parallel to the sequential processing path (PF1) a further processing path (PF2) in which the second convolutional neural network (CNN2) generates a fourth set (RFM4) of resultant feature maps on the basis of the first set (RFM1) of two-dimensional feature maps by means of at least one fourth convolutional layer (LA4),
wherein the second convolutional neural network (CNN2) generates on the basis of the third (RFM3) and the fourth set (RFM4) of resultant feature maps the final feature map (FFM1) corresponding to the sub-image (TB),
and wherein the number of successive convolution layers in the parallel processing path (PF2) is smaller than the number of successive convolution layers in the sequential processing path (PF1).

10. Method according to Claim 1,
comprising

- acquisition of a first preliminary fluorescence image (EVFB2) in the colour channel using a predefined acquisition parameter (EP1),
- establishment of whether a brightness of the first preliminary fluorescence image (EVFB2) of the colour channel exceeds a maximum brightness,
- in the event of the first preliminary fluorescence image of the colour channel (EVFB2) not exceeding the maximum brightness, use of the first preliminary fluorescence image (EVFB2) as the one fluorescence image (SG),
- in the event of the first preliminary fluorescence image of the colour channel (EVFB2) exceeding the maximum brightness, acquisition of a second preliminary fluorescence image in the colour channel (ZVFB2) and use of the second preliminary fluorescence image of the colour channel (ZVFB2) as the one fluorescence image (SG).

11. Device (V1) for detecting a binding of autoantibodies from a patient sample to double-stranded deoxyribonucleic acid using Crithidia luciliae cells by means of fluorescence microscopy and by means of digital image processing, comprising

- a mounting device (H) for a substrate (S) which has multiple Crithidia luciliae cells (CR) and which has been incubated with a patient sample having the autoantibodies and, furthermore, with secondary antibodies which have each been labelled with a fluorescent dye which is preferably a green fluorescent dye,
- at least one image acquisition unit (K1, K2) for acquiring a fluorescence image (SG) of the substrate (S) in a colour channel which corresponds to the fluorescent dye, wherein the colour channel is preferably a green channel,

and comprising furthermore at least one computing unit (R) which is designed

- to identify by means of a first pretrained convolutional neural network (CNN1) respective sub-images (TB) in the one fluorescence image (SG) that each represent at least one Crithidia luciliae cell (CR),
- to respectively process at least one subset of the respective sub-images (TB) by means of a second pretrained convolutional neural network (CNN2), whereby the second convolutional neural network (CNN2) determines a respective subordinate image representing the kinetoplast region for a respective sub-image for determining respective binding measures (IBM1, IBM2) on the basis of respective subordinate images, whereby a respective binding measure indicates a respective extent of a binding of autoantibodies in a respective kinetoplast region (K) of a respective Crithidia luciliae cell (CR) of a respective sub-image (TB),
- and to determine an overall binding measure (GBM) of a binding of autoantibodies from the patient sample to double-stranded deoxyribonucleic acid on the basis of the respective binding measures (IBM1, IBM2).

12. Computing unit (R) which is designed, in the course of a digital image processing,

- to receive a fluorescence image (SG) which represents a staining of a substrate, which in turn has multiple Crithidia luciliae cells (CR), by a fluorescent dye which is preferably a green fluorescent dye,

- to identify by means of a first pretrained convolutional neural network (CNN1) respective sub-images (TB) in the one fluorescence image (SG) that each represent at least one Crithidia luciliae cell (CR),
- to respectively process at least one subset of the respective sub-images (TB) by means of a second pretrained convolutional neural network (CNN2), whereby the second convolutional neural network (CNN2) determines a respective subordinate image representing the kinetoplast region for a respective sub-image for determining respective binding measures (IBM1, IBM2) on the basis of respective subordinate images, whereby a respective binding measure indicates a respective extent of a binding of autoantibodies in a respective kinetoplast region (K) of a respective Crithidia luciliae cell (CR) of a respective sub-image (TB),
- and to determine an overall binding measure (GBM) of a binding of autoantibodies from the patient sample to double-stranded deoxyribonucleic acid on the basis of the respective binding measures (IBM1, IBM2).

13. Data network device (DV) comprising at least one data interface (DS4) for receiving a fluorescence image (BI1, SG) which represents a staining of a substrate, which in turn has multiple Crithidia luciliae cells, by a fluorescent dye which is preferably a green fluorescent dye and furthermore at least one computing unit (R) which is designed, in the course of a digital image processing,

- to identify by means of a first pretrained convolutional neural network (CNN1) respective sub-images (TB) in the one fluorescence image (SG) that each represent at least one Crithidia luciliae cell (CR),
- to respectively process at least one subset of the respective sub-images (TB) by means of a second pretrained convolutional neural network (CNN2), whereby the second convolutional neural network (CNN2) determines a respective subordinate image representing the kinetoplast region for a respective sub-image for determining respective binding measures (IBM1, IBM2) on the basis of respective subordinate images, whereby a respective binding measure indicates a respective extent of a binding of autoantibodies in a respective kinetoplast region (K) of a respective Crithidia luciliae cell (CR) of a respective sub-image (TB),
- and to determine an overall binding measure (GBM) of a binding of autoantibodies from the patient sample to double-stranded deoxyribonucleic acid on the basis of the respective binding measures (IBM1, IBM2).

**14.** Method for digital image processing, comprising

- receiving of a fluorescence image (SG) which represents a staining of a substrate (S), which in turn has multiple Crithidia luciliae cells (CR), by a fluorescent dye which is preferably a green fluorescent dye,
- identification by means of a first pretrained convolutional neural network (CNN1) of respective sub-images (TB) in the fluorescence image (SG) that each represent at least one Crithidia luciliae cell (CR),
- respective processing of at least one subset of the respective sub-images (TB) by means of a second pretrained convolutional neural network (CNN2), whereby the second convolutional neural network (CNN2) determines a respective subordinate image representing the kinetoplast region for a respective sub-image for determining respective binding measures (IBM1, IBM2) on the basis of respective subordinate images, whereby a respective binding measure indicates a respective extent of a binding of autoantibodies in a respective kinetoplast region (K) of a respective Crithidia luciliae cell (CR) of a respective sub-image (TB),
- determination of an overall binding measure (GBM) of a binding of autoantibodies from the patient sample to double-stranded deoxyribonucleic acid on the basis of the respective binding measures (IBM1, IBM2).

**15.** Computer program product (CPP) comprising commands which, upon execution of the program by a computer, prompt said computer to carry out the method for digital image processing according to Claim 14.

**16.** Data carrier signal (SI2) which transmits the computer program product (CPP) according to Claim 15.

**Revendications**

**1.** Procédé de détection de la liaison d'auto-anticorps d'un échantillon de patient à l'acide désoxyribonucléique double brin à l'aide de cellules Crithidia luciliae par microscopie à fluorescence et par traitement d'images numériques, ledit procédé comportant les étapes suivantes :

- fournir un substrat (S) qui contient plusieurs cellules Crithidia luciliae (CR),
- laisser incuber le substrat (S) avec l'échantillon de patient qui comporte potentiellement les auto-anticorps,
- laisser incuber le substrat (S) avec des anticorps secondaires qui sont chacun marqués par un colorant fluorescent qui est de préférence un colorant fluorescent vert,
- acquérir une image de fluorescence (SG) du substrat (S) dans un canal chromatique qui correspond au colorant fluorescent, le canal chromatique étant de préférence un canal vert,

**caractérisé par** les étapes suivantes :

- -identifier dans l'image de fluorescence (SG) des images partielles respectives (TB) qui représentent chacune au moins une cellule Crithidia luciliae (CR), à l'aide d'un premier réseau neuronal convolutif pré-entraîné (CNN1),
- effectuer un traitement respectif sur au moins un sous-ensemble des images partielles respectives (TB) à l'aide d'un deuxième réseau neuronal convolutif pré-entraîné (CNN2), le deuxième réseau neuronal convolutif (CNN2) déterminant pour une image partielle respective une sous-image respective, qui représente une région kinétoplaste, afin de déterminer des dimensions de liaison respectives (IBM1, IBM2) sur la base de sous-images respectives, une mesure de liaison respective indiquant un degré respectif de la liaison d'auto-anticorps dans une région kinétoplaste respective (K) d'une cellule Crithidia luciliae respective (CR) d'une image partielle respective (TB),
- déterminer une mesure de liaison totale (GBM) rapportée à la liaison d'auto-anticorps de l'échantillon de patient à l'acide désoxyribonucléique double brin sur la base des mesures de liaison respectives (IBM1, IBM2).

**2.** Procédé selon la revendication 1, l'identification des images partielles respectives (TB) dans l'image de fluorescence (SG) étant effectuée du fait que le premier réseau neuronal convolutif pré-entraîné (CNN1) permet d'associer des segments d'image respectifs de l'image de fluorescence à des classes de segments d'image respectives d'un groupe de classes de segments d'image, le groupe de classes de segments d'image comprenant au moins les classes de segments d'image suivantes

- cellule et
- fond.

**3.** Procédé selon la revendication 1, l'identification des images partielles respectives (TB) dans l'image de fluorescence (SG) étant effectuée du fait que le premier réseau neuronal convolutif pré-entraîné (CNN1) permet d'associer des segments d'image respectifs de l'image de fluorescence à des classes de segments d'image respectives d'un groupe de classes de segments d'image, le groupe de classes de segments d'image comprenant au moins les classes de segments d'image suivantes

- corps cellulaire,
- bord de cellule et
- fond.

4. Procédé selon la revendication 1,

comportant, pour une image partielle respective (TB), les étapes suivantes

- sélectionner une sous-image respective (SUB) de l'image partielle respective (TB), la sous-image respective (SUB) représentant une région kinétoplaste respective (K) d'une cellule Crithidia luciliae respective (CR),
- déterminer la mesure de liaison respective (IBM1) sur la base de la sous-image respective (SUB),

et comportant en outre l'étape suivante : déterminer la mesure de liaison totale (GBM) sur la base des mesures de liaison respectives (IBM1, IBM2).

5. Procédé selon la revendication 1,
comportant les étapes suivantes

- déterminer une carte de caractéristiques finale respective (FFM1) pour une image partielle respective (TB) à l'aide du deuxième réseau neuronal convolutif (CNN2),
- déterminer une mesure de confiance respective (PKN) rapportée à la présence d'une liaison d'auto-anticorps dans une région kinétoplaste respective (K) pour l'image partielle respective (TB),
- sélectionner un sous-ensemble d'images partielles (TB) sur la base des mesures de confiance déterminées (PKN),
- effectuer un traitement respectif sur des cartes de caractéristiques respectives des images partielles sélectionnées respectives afin de déterminer les mesures de liaison respectives (IBM1, IBM2),
- déterminer la mesure de liaison totale (GBM) sur la base des mesures de liaison respectives (IBM1, IBM2) des images partielles sélectionnées respectives.

6. Procédé selon la revendication 5,

comportant, pour une image partielle respective (TB) du sous-ensemble sélectionné, les étapes suivantes

- sélectionner une sous-image respective (SUB) de l'image partielle respective (TB) sur la base d'une carte de caractéristiques finale respective (FFM1) correspondant à l'image partielle respective (TB), la sous-image respective (SUB) représentant une région kinétoplaste respective (K) d'une cellule Crithidia luciliae respective (CR),
- déterminer la mesure de liaison respective (IBM1) sur la base de la sous-image respective (SUB),

et comprenant en outre l'étape suivante

- déterminer la mesure de liaison totale (GBM) sur la base des mesures de liaison respectives (IBM1, IBM2).

7. Procédé selon la revendication 6,

comportant, pour une image partielle respective (TB) du sous-ensemble sélectionné, les étapes suivantes

- déterminer un opérateur de masquage respectif (BM) sur la base de la carte de caractéristiques finale respective (FFM1),
- sélectionner la sous-image respective (SUB) de l'image partielle respective (TB) par application de l'opérateur de masquage respectif (BM) à l'image partielle respective (TB),
- déterminer la mesure de liaison respective (IBM1) sur la base de la sous-image respective (SUB),

et comportant l'étape suivante

- déterminer la mesure de liaison totale (GBM) sur la base des mesures de liaison respectives (IBM1, IBM2).

8. Procédé selon la revendication 5,
le deuxième réseau neuronal convolutif (CNN2) générant au cours du traitement d'une image partielle (TB)

- dans un premier niveau de traitement (P1), un premier ensemble de cartes de caractéristiques résultantes (RFM1) sur la base de l'image partielle (TB) à l'aide d'au moins une première couche convolutive (LA1) et par application de plusieurs noyaux de convolution bidimensionnels,
- dans un deuxième niveau de traitement (P2)

° un deuxième ensemble de cartes de caractéristiques résultantes (RFM2) sur la base du premier ensemble de cartes de caractéristiques bidimensionnelles (RFM1) à l'aide d'au moins une deuxième couche convolutive (LA2) et par application de plusieurs noyaux de convolution tridimensionnels,

◦ et en outre un troisième ensemble de cartes de caractéristiques résultantes (RFM3) sur la base du deuxième ensemble de cartes de caractéristiques bidimensionnelles (RFM2) à l'aide d'au moins une troisième couche convolutive (LA3) et par application de plusieurs noyaux de convolution tridimensionnels,

le deuxième ensemble (RFM2) comportant un plus petit nombre de cartes de caractéristiques résultantes que le premier ensemble (RFM1) et le troisième ensemble (RFM3) comportant un plus grand nombre de cartes de caractéristiques résultantes que le deuxième ensemble (RFM2).

**9.** Procédé selon la revendication 8,

dans le deuxième niveau de traitement (P2), la deuxième couche convolutive (LA2) et la troisième couche convolutive (LA3) se succédant en tant que sous-étapes d'un chemin de traitement séquentiel (PF1),

dans le deuxième niveau de traitement (P2), un autre chemin de traitement (PF2), parallèle au chemin de traitement séquentiel (PF1), étant présent dans lequel le deuxième réseau neuronal convolutif (CNN2) génère un quatrième ensemble (RFM4) de cartes de caractéristiques résultantes sur la base du premier ensemble (RFM1) de cartes de caractéristiques bidimensionnelles à l'aide d'au moins une quatrième couche convolutive (LA4),

le deuxième réseau neuronal convolutif (CNN2) générant la carte de caractéristiques finale (FFM1), correspondant à l'image partielle (TB), sur la base du troisième ensemble (RFM3) et du quatrième ensemble (RFM4) de cartes de caractéristiques résultantes,

et le nombre de couches de convolution consécutives dans le chemin de traitement parallèle (PF2) étant inférieur au nombre de couches de convolution consécutives dans le chemin de traitement séquentiel (PF1).

**10.** Procédé selon la revendication 1, comportant les étapes suivantes

- acquérir une première image de fluorescence préliminaire (EVFB2) dans le canal chromatique à l'aide d'un paramètre d'acquisition spécifié (EP1),
- déterminer si une luminosité de l'image de fluorescence préliminaire (EVFB2) du canal chromatique dépasse une luminosité maximale,
- dans le cas où la première image de fluorescence préliminaire du canal chromatique (EVB2) ne dépasse pas la luminosité maximale, utiliser la première image de fluorescence préliminaire (EVFB2) comme image de fluorescence (SG),
- dans le cas où la première image de fluorescence préliminaire du canal chromatique (EVB2) dépasse la luminosité maximale, acquérir une deuxième image de fluorescence préliminaire dans le canal chromatique (ZVFB2) et utiliser la deuxième image de fluorescence préliminaire du canal chromatique (ZVFB2) comme image de fluorescence (SG).

**11.** Dispositif (V1) destiné à détecter la liaison d'auto-anticorps d'un échantillon de patient à l'acide désoxyribonucléique double brin à l'aide de cellules Crithidia luciliae par microscopie à fluorescence et par traitement d'images numériques, ledit dispositif comportant

- un dispositif de retenue (H) destiné à un substrat (S) qui comporte plusieurs cellules Crithidia luciliae (CR) et qui a été incubé avec un échantillon de patient contenant les auto-anticorps, ainsi qu'avec des anticorps secondaires qui sont marqués chacun avec un colorant fluorescent qui est de préférence un colorant fluorescent vert,
- au moins une unité d'acquisition d'image (K1, K2) destinée à acquérir une image de fluorescence (SG) du substrat (S) dans un canal chromatique qui est de préférence un colorant fluorescent vert,

et comportant en outre au moins une unité de calcul (R) qui est conçue

- pour identifier, dans l'image de fluorescence (SG), des images partielles respectives (TB), qui représentent chacune au moins une cellule Crithidia luciliae (CR), à l'aide d'un premier réseau neuronal convolutif pré-entraîné (CNN1),
- pour traiter au moins un sous-ensemble des images partielles respectives (TB) à l'aide d'un deuxième réseau neuronal convolutif pré-entraîné (CNN2),
- le deuxième réseau neuronal convolutif (CNN2) déterminant, pour une image partielle respective, une sous-image respective qui représente une région kinétoplaste afin de déterminer des mesures de liaison respectives (IBM1, IBM2) sur la base de sous-images respectives, une mesure de liaison respective indiquant un degré respectif de liaison d'auto-anticorps dans une région kinétoplaste respective (K) d'une cellule Crithidia luciliae respective (CR) d'une image partielle respective (TB)
- et pour déterminer une mesure de liaison totale (GBM) de la liaison d'auto-anticorps de l'échantillon de patient à l'acide désoxyribonucléique double brin sur la base des mesures de liaison respectives (IBM1, IBM2).

12. Unité de calcul (R) qui est conçue pour, au cours du traitement d'images numériques,

   - recevoir une image de fluorescence (SG) qui représente une coloration d'un substrat, qui comporte là encore plusieurs cellules Crithidia luciliae (CR), par un colorant fluorescent qui est de préférence un colorant fluorescent vert,
   - identifier, dans l'image de fluorescence (SG), des images partielles respectives (TB) qui représentent chacune au moins une cellule Crithidia luciliae (CR), à l'aide d'un premier réseau neuronal convolutif pré-entraîné (CNN1),
   - traiter au moins un sous-ensemble des images partielles respectives (TB) à l'aide d'un deuxième réseau neuronal convolutif pré-entraîné (CNN2), le deuxième réseau neuronal convolutif (CNN2) déterminant pour une image partielle respective une sous-image respective, qui représente une région kinétoplaste, afin de déterminer des mesures de liaison respectives (IBM1, IBM2) sur la base de sous-images respectives, une mesure de liaison respective indiquant un degré respectif de liaison d'auto-anticorps dans une région kinétoplaste respective (K) d'une cellule Crithidia luciliae respective (CR) d'une image partielle respective (TB),
   - et déterminer une mesure de liaison totale (GBM) de la liaison d'auto-anticorps de l'échantillon de patient à l'acide désoxyribonucléique double brin sur la base des mesures de liaison respectives (IBM1, IBM2).

13. Dispositif formant réseau de données (DV), comportant au moins une interface de données (DS4) destinée à recevoir une image de fluorescence (BI1, SG) qui représente une coloration d'un substrat, qui comporte là encore plusieurs cellules Crithidia luciliae, par un colorant fluorescent qui est de préférence un colorant fluorescent vert, et en outre au moins une unité de calcul (R) qui est conçue pour, au cours du traitement d'image numérique,

   - identifier, dans une image de fluorescence (SG), des images partielles respectives (TB), qui représentent chacune au moins une cellule Crithidia luciliae (CR), à l'aide d'un premier réseau neuronal convolutif pré-entraîné (CNN1),
   - traiter au moins un sous-ensemble des images partielles respectives (TB) à l'aide d'un deuxième réseau neuronal convolutif pré-entraîné (CNN2), le deuxième réseau neuronal convolutif (CNN2) déterminant pour une image partielle respective une sous-image respective, qui représente une région kinétoplaste, afin de déterminer des mesures de liaison respectives (IBM1, IBM2) sur la base de sous-images respectives, une mesure de liaison respective indiquant un degré respectif de liaison d'auto-anticorps dans une région kinétoplaste respective (K) d'une cellule Crithidia luciliae respective (CR) d'une image partielle respective (TB),
   - et déterminer une mesure de liaison totale (GBM) de la liaison d'auto-anticorps de l'échantillon de patient à l'acide désoxyribonucléique double brin sur la base des mesures de liaison respectives (IBM1, IBM2).

14. Procédé de traitement d'images numérique, comprenant les étapes suivantes

   - recevoir une image de fluorescence (SG) qui représente une coloration d'un substrat (S), qui comporte là encore plusieurs cellules Crithidia luciliae (CR), par un colorant fluorescent qui est de préférence un colorant fluorescent vert,
   - identifier, dans l'image de fluorescence (SG), des images partielles respectives (TB) qui représentent chacune au moins une cellule Crithidia luciliae (CR), à l'aide d'un premier réseau neuronal convolutif pré-entraîné (CNN1),
   - effectuer un traitement respectif sur au moins un sous-ensemble des images partielles respectives (TB) à l'aide d'un deuxième réseau neuronal convolutif pré-entraîné (CNN2), le deuxième réseau neuronal convolutif (CNN2) déterminant pour une image partielle respective une sous-image respective, qui représente une région kinétoplaste, afin de déterminer des mesures de liaison respectives (IBM1, IBM2) sur la base de sous-images respectives, une mesure de liaison respective indiquant un degré respectif de liaison d'auto-anticorps dans une région kinétoplaste respective (K) d'une cellule Crithidia luciliae respective (CR) d'une image partielle respective (TB),
   - déterminer une mesure de liaison totale (GBM) de la liaison d'auto-anticorps de l'échantillon de patient à l'acide désoxyribonucléique double brin sur la base des mesures de liaison respectives (IBM1, IBM2).

15. Progiciel (CPP),
   comprenant des instructions qui, lorsque le programme est exécuté par un ordinateur, ordonnent à celui-ci de mettre en oeuvre le procédé de traitement d'images numérique selon la revendication 14.

16. Signal porteur de données (SI2) que transmet le progiciel (CPP) selon la revendication 15.

## Fig.1

## Fig.2

Fig.3

Fig.4

# Fig.5

Fig.6

Fig.7a

Fig.7b

Fig.7c

Fig.8a   Fig.8b   Fig.8c

Fig.9c

SUB

Fig.9b

BM

Fig.9a

VFM

**FIG. 10**

**FIG. 11**

## FIG. 12a

SG (1)

1024

SG

1024

SG (M)

## FIG. 12b

MA1 (1)

MA1 (M)

## Fig. 13

**Fig. 14a**

TB1

CNN2

IBM1

**FIG. 14b**

IBM2    IBM2    · · ·

· · ·

ERS

GBM

**Fig. 15**

SG

S6

TBD

**FIG. 16a**

**FIG. 16b**

PSA

CONV2D

BN

ACT

**FIG. 16c**

PSB

CONV3D

BN

ACT

**FIG. 17**

**FIG. 18**

**FIG. 19**

**FIG. 20**

**FIG. 21**

**FIG. 22**

**FIG. 23**

| input_2: InputLayer | input: | (None, 150, 150,1) |
|---|---|---|
| | output: | (None, 150, 150,1) | ⌐TB1

CNN2 ,P1

| conv2d_1: Conv2D | input: | (None, 150, 150,1) |
|---|---|---|
| | output: | (None, 75, 75, 16) |

BN    CONV2D

| batch_normalization_1: BatchNormalization | input: | (None, 75, 75, 16) |
|---|---|---|
| | output: | (None, 75, 75, 16) |

LA1

| conv2d_4: Conv2D | input: | (None, 150, 150,1) |
|---|---|---|
| | output: | (None, 75, 75, 64) |

| activation_1: Activation | input: | (None, 75, 75, 16) |
|---|---|---|
| | output: | (None, 75, 75, 16) |

ACT                              PS1        PS4

| conv2d_2: Conv2D | input: | (None, 75, 75, 16) |
|---|---|---|
| | output: | (None, 75, 75, 32) |

| conv2d_3: Conv2D | input: | (None, 75, 75, 16) |
|---|---|---|
| | output: | (None, 75, 75, 32) | ⌐CONV3D

CONV3D

| batch_normalization_2:BatchNormalization | input: | (None, 75, 75, 32) |
|---|---|---|
| | output: | (None, 75, 75, 32) |

| batch_normalization_3: BatchNormalization | input: | (None, 75, 75, 32) |
|---|---|---|
| | output: | (None, 75, 75, 32) |

BN

| activation_2: Activation | input: | (None, 75, 75, 32) |
|---|---|---|
| | output: | (None, 75, 75, 32) |

| activation_3: Activation | input: | (None, 75, 75, 32) |
|---|---|---|
| | output: | (None, 75, 75, 32) | ⌐ACT

BN

ACT        PS2                                                      PS3

PSC

| concatenate_1: Concatenate | input: | [(None, 75, 75, 32), (None, 75, 75, 32)] |
|---|---|---|
| | output: | (None, 75, 75, 64) |

LA11

RFM1

PSS

| add_1: Add | input: | [(None, 75, 75, 64), (None, 75, 75, 64)] |
|---|---|---|
| | output: | (None, 75, 75, 64) |

EP 3 971 827 B1

FIG. 24

FIG. 25

EP 3 971 827 B1

**FIG. 26**

EP 3 971 827 B1

FIG. 27

EP 3 971 827 B1

# FIG. 28

EP 3 971 827 B1

**FIG.29**

V20

EP1 → [ S200 ]

EVFB2

[ S201 ]

HD

[ S202 ]

AS

S203 ◇ ? — N

TH

J

[ S204 ] ← EP2

ZVFB2    ID

EVFB2

**FIG. 30 (1/3)**

CNNA1

SG

| input_1: InputLayer | input: | (None, 1024, 1024, 1) |
|---|---|---|
| | output: | (None, 1024, 1024, 1) |

IWL

| lambda_1: Lambda | input: | (None, 1024, 1024, 1) |
|---|---|---|
| | output: | (None, 1024, 1024, 1) |

SKL

| conv2d_1: Conv2D | input: | (None, 1024, 1024, 1) |
|---|---|---|
| | output: | (None, 512, 512, 16) |

CONV2D, CS1

| conv2d_2: Conv2D | input: | (None, 512, 512, 16) |
|---|---|---|
| | output: | (None, 512, 512, 16) |

CONV2D

EP 3 971 827 B1

FIG. 30 (2/3)

```
1
2
3
```

| leaky_re_lu_1: LeakyReLU | input: | (None, 512, 512, 16) |
| | output: | (None, 512, 512, 16) |

LR

| average_pooling2d_1: AveragePooling2D | input: | (None, 512, 512, 16) |
| | output: | (None, 256, 256, 16) |

AP

BN

| batch_normalization_1: BatchNormalization | input: | (None, 256, 256, 16) |
| | output: | (None, 256, 256, 16) |

| leaky_re_lu_2: LeakyReLU | input: | (None, 256, 256, 16) |
| | output: | (None, 256, 256, 16) |

LR

EP 3 971 827 B1

FIG. 30 (3/3)

```
1
2
3
```

| conv2d_3: Conv2D | input: | (None, 256, 256, 16) |
|---|---|---|
| | output: | (None, 256, 256, 16) |

CONV2D

| batch_normalization_2: BatchNormalization | input: | (None, 256, 256, 16) |
|---|---|---|
| | output: | (None, 256, 256, 16) |

BN

| leaky_re_lu_3: LeakyReLU | input: | (None, 256, 256, 16) |
|---|---|---|
| | output: | (None, 256, 256, 16) |

LR

| conv2d_4: Conv2D | input: | (None, 256, 256, 16) |
|---|---|---|
| | output: | (None, 256, 256, 16) |

CONV2D

| module_out_0_0: Add | input: | [(None, 256, 256, 16), (None, 256, 256, 16)] |
|---|---|---|
| | output: | (None, 256, 256, 16) |

ADD

EP 3 971 827 B1

FIG. 31 (1/2)

FIG. 31 (1/2) 1/2    CNNA2

CONV2D

| conv2d_5: Conv2D | input: | (None, 256, 256, 16) |
|---|---|---|
| | output: | (None, 256, 256, 32) |

LR

| leaky_re_lu_4: LeakyReLU | input: | (None, 256, 256, 32) |
|---|---|---|
| | output: | (None, 256, 256, 32) |

CONV2D

| conv2d_14: Conv2D | input: | (None, 256, 256, 16) |
|---|---|---|
| | output: | (None, 256, 256, 16) |

AP

| average_pooling2d_2: AveragePooling2D | input: | (None, 256, 256, 32) |
|---|---|---|
| | output: | (None, 128, 128, 32) |

LR

| leaky_re_lu_13: LeakyReLU | input: | (None, 256, 256, 16) |
|---|---|---|
| | output: | (None, 256, 256, 16) |

BN

| batch_normalization_3: BatchNormalization | input: | (None, 128, 128, 32) |
|---|---|---|
| | output: | (None, 128, 128, 32) |

US

| up_sampling2d_1: UpSampling2D | input: | (None, 256, 256, 16) |
|---|---|---|
| | output: | (None, 1024, 1024, 16) |

LR

| leaky_re_lu_5: LeakyReLU | input: | (None, 128, 128, 32) |
|---|---|---|
| | output: | (None, 128, 128, 32) |

CONV2D

| conv2d_6: Conv2D | input: | (None, 128, 128, 32) |
|---|---|---|
| | output: | (None, 128, 128, 32) |

EP 3 971 827 B1

FIG. 31 (2/2)

BN

| batch_normalization_4: BatchNormalization | input: | (None, 128, 128, 32) |
|---|---|---|
| | output: | (None, 128, 128, 32) |

LR

| leaky_re_lu_6: LeakyReLU | input: | (None, 128, 128, 32) |
|---|---|---|
| | output: | (None, 128, 128, 32) |

CONV2D

| conv2d_7: Conv2D | input: | (None, 128, 128, 32) |
|---|---|---|
| | output: | (None, 128, 128, 32) |

ADD

| module_out_1_0: Add | input: | [(None, 128, 128, 32), (None, 128, 128, 32)] |
|---|---|---|
| | output: | (None, 128, 128, 32) |

CONV2D

| conv2d_8: Conv2D | input: | (None, 128, 128, 32) |
|---|---|---|
| | output: | (None, 128, 128, 48) |

EP 3 971 827 B1

**FIG. 32 (1/2)**

CNNA3

CONV2D — conv2d_8: Conv2D
| | input: | (None, 128, 128, 32) |
| --- | --- | --- |
| | output: | (None, 128, 128, 48) |

LR — leaky_re_lu_7: LeakyReLU
| | input: | (None, 128, 128, 48) |
| --- | --- | --- |
| | output: | (None, 128, 128, 48) |

AP — average_pooling2d_3: AveragePooling2D
| | input: | (None, 128, 128, 48) |
| --- | --- | --- |
| | output: | (None, 64, 64, 48) |

BN — batch_normalization_5: BatchNormalization
| | input: | (None, 64, 64, 48) |
| --- | --- | --- |
| | output: | (None, 64, 64, 48) |

LR — leaky_re_lu_8: LeakyReLU
| | input: | (None, 64, 64, 48) |
| --- | --- | --- |
| | output: | (None, 64, 64, 48) |

CONV2D — conv2d_9: Conv2D
| | input: | (None, 64, 64, 48) |
| --- | --- | --- |
| | output: | (None, 64, 64, 48) |

BN — batch_normalization_6: BatchNormalization
| | input: | (None, 64, 64, 48) |
| --- | --- | --- |
| | output: | (None, 64, 64, 48) |

CONV2D — conv2d_15: Conv2D
| | input: | (None, 128, 128, 32) |
| --- | --- | --- |
| | output: | (None, 128, 128, 16) |

LR — leaky_re_lu_14: LeakyReLU
| | input: | (None, 128, 128, 16) |
| --- | --- | --- |
| | output: | (None, 128, 128, 16) |

up_sampling2d_2: UpSampling2D
| | input: | (None, 128, 128, 16) |
| --- | --- | --- |
| | output: | (None, 1024, 1024, 16) |

US

EP 3 971 827 B1

# FIG. 32 (2/2)

EP 3 971 827 B1

FIG. 33 (1/2)

CNNA4

CONV2D

| conv2d_13: Conv2D | input: | (None, 32, 32, 96) |
|---|---|---|
| | output: | (None, 32, 32, 96) |

ADD

| module_out_3_0: Add | input: | [(None, 32, 32, 96), (None, 32, 32, 96)] |
|---|---|---|
| | output: | (None, 32, 32, 96) |

CONV2D

| conv2d_17: Conv2D | input: | (None, 32, 32, 96) |
|---|---|---|
| | output: | (None, 32, 32, 16) |

LR

| leaky_re_lu_16: LeakyReLU | input: | (None, 32, 32, 16) |
|---|---|---|
| | output: | (None, 32, 32, 16) |

US

| up_sampling2d_4: UpSampling2D | input: | (None, 32, 32, 16) |
|---|---|---|
| | output: | (None, 1024, 1024, 16) |

CONC

| concatenate_1: Concatenate | input: | [(None, 1024, 1024, 16), (None, 1024, 1024,16), (None, 1024, 1024, 16), (None, 1024, 1024, 16)] |
|---|---|---|
| | output: | (None, 1024, 1024, 64) |

EP 3 971 827 B1

FIG. 33 (2/2)

| 1 |
|---|
| 2 |

CONV2D— | conv2d_18: Conv2D | input: | (None, 1024, 1024, 64) |
| | output: | (None, 1024, 1024, 16) |

CONV2D— | conv2d_19: Conv2D | input: | (None, 1024, 1024, 16) |
| | output: | (None, 1024, 1024, 8) |

CONV2D— | conv2d_20: Conv2D | input: | (None, 1024, 1024, 8) |
| | output: | (None, 1024, 1024, 8) |

CONV2D— | conv2d_21: Conv2D | input: | (None, 1024, 1024, 8) |
| | output: | (None, 1024, 1024, 8) |

CONV2D— | conv2d_22: Conv2D | input: | (None, 1024, 1024, 8) |
| | output: | (None, 1024, 1024, 3) |

ACT— | output_0: Activation | input: | (None, 1024, 1024, 3) |
| | output: | (None, 1024, 1024, 3) |

MA1  MA2  MA3

EP 3 971 827 B1

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Nicht-Patentliteratur**

- **STEFAN GERLACH et al.** Automated Evaluation of Crithidia luciliae Based Indirect Immunofluorescence Tests: A Novel Application of the EUROPattern-Suite Technology. *JOURNAL OF IMMUNOLOGY RESEARCH,* 01. Januar 2015, vol. 2015, 1-8

- **SATOSHI ; SUZUKI.** Topological structural analysis of digitized binary images by border following. *Computer Vision, Graphics, and Image Processing,* 1985, vol. 30 (1), 32-46